(19) Europäisches Patentamt

European Patent Office

Office européen des brevets

(11) **EP 1 024 200 A2**

(12) **EUROPEAN PATENT APPLICATION**

(43) Date of publication:
02.08.2000 Bulletin 2000/31

(51) Int. Cl.$^7$: **C12Q 1/68**

(21) Application number: 00250023.9

(22) Date of filing: 26.01.2000

(84) Designated Contracting States:
**AT BE CH CY DE DK ES FI FR GB GR IE IT LI LU MC NL PT SE**
Designated Extension States:
**AL LT LV MK RO SI**

(30) Priority: 27.01.1999 US 238402

(71) Applicant:
**Affymetrix, Inc. (a California Corporation)
Santa Clara, CA 95051 (US)**

(72) Inventors:
• **Patil, Nila
Woodside, CA 94062 (US)**
• **Shah, Nila
Cupertino, CA 95014 (US)**
• **Warrigton, Janet A.
Los Altos, CA 94024 (US)**

(74) Representative:
**UEXKÜLL & STOLBERG
Patentanwälte
Beselerstrasse 4
22607 Hamburg (DE)**

(54) **Genetic compositions and methods**

(57)     This invention provides nucleic acid segments derived from the human genome, including polymorphic sites. Allele-specific primers and probes hybridizing to these sites and to regions flanking these sites are also provided. This invention further provides methods of analyzing a nucleic acid from an individual or a group of individuals. The nucleic acids, primers, and probes are useful tools for applications including forensics, paternity testing, medicine, e.g., the coorrelation of polymorphisms with phenotypic traits, and genetic analysis, e.g., genetic mapping of such phenotypic traits.

Fig. 1B

Fig. 1A

SEQUENCE LISTING

```
<110> Affymetrix Inc.
<120> Genetic Compositions and Methods
<130> 52944
<150> US 09/238,402
<151> 1999-01-27
<160> 632


<210> 1
<211> 31
<212> DNA
<213> unknown
<308> D42043
<400> 1
ACCAGCGAGG GGAGTRTATC CACCAAGCAG A

<210> 2
<211> 31
<212> DNA
<213> unknown
<308> D42043
<400> 2
GGGAAACTGT GTTTCRGGAC AGCAGCAGGA G

<210> 3
<211> 31
<212> DNA
<213> unknown
<308> D42043
<400> 3
GAGCATTCGG GTCAAWTCCA TGGACACCCT G

<210> 4
<211> 31
<212> DNA
<213> unknown
<308> D45248
<400> 4
GAGGGTGAAT GCCGTSAAGA CCAAAGTGGA A

<210> 5
<211> 31
<212> DNA
<213> unknown
<308> M69066
<400> 5
ATGGTGCCTT CAAGASCTTC ACCAAACATA C
```

```
<210> 6
<211> 31
<212> DNA
<213> unknown
<308> M69066
<400> 6
CTCCACAGCC ATCCCRGCCT TGGCATCTAG A

<210> 7
<211> 31
<212> DNA
<213> unknown
<308> M69066
<400> 7
GATTTTGGCC TCATTMCTTT ACCACCCCCA C

<210> 8
<211> 31
<212> DNA
<213> unknown
<308> U56637
<400> 8
AGCAAGTTTG TTCCARGTGA CCCATTGAGC T

<210> 9
<211> 31
<212> DNA
<213> unknown
<308> X12671
<400> 9
AGGGCGAAGG TAGGCWGGCA GATACGTTCG T

<210> 10
<211> 31
<212> DNA
<213> unknown
<308> X12671
<400> 10
CAGGTTCTAT TTGGAWTTTA TATACAACCT G

<210> 11
<211> 31
<212> DNA
<213> unknown
<308> AB006782
<400> 11
GAGATGGCCT TCAGCRGTTC CCAGGCTCCC T
```

<210> 12
<211> 31
<212> DNA
<213> unknown
<308> AB006782
<400> 12
AGCAGGCCTG ATGGCWTCCC ACTGGCCTCC A

<210> 13
<211> 31
<212> DNA
<213> unknown
<308> AB006782
<400> 13
CACTGGCCTC CACCAMCTGA CCAGAGTGTT C

<210> 14
<211> 31
<212> DNA
<213> unknown
<308> AB006782
<400> 14
TGACCAGAGT GTTCTYTTCA GAGGACTGGC T

<210> 15
<211> 31
<212> DNA
<213> unknown
<308> AB006782
<400> 15
GAGTGTTCTC TTCAGRGGAC TGGCTCCTTT C

<210> 16
<211> 31
<212> DNA
<213> unknown
<308> HT3708
<400> 16
GGAAACCTCA TTGTTSTGTA CAAAGTACTA G

<210> 17
<211> 31
<212> DNA
<213> unknown
<308> HT3708
<400> 17
TGAAACTCAT CTCATYACCC TTTTCTGGGT T

<210> 18
<211> 31
<212> DNA
<213> unknown
<308> J02621
<400> 18
AGAAACTAAA GAAGAYTTAC CTGCGGAAAA C

<210> 19
<211> 31
<212> DNA
<213> unknown
<308> J02621
<400> 19
AGAAGACTTA CCTGCRGAAA ACGGGGAAAC G

<210> 20
<211> 31
<212> DNA
<213> unknown
<308> J02621
<400> 20
GAGGAATATT TTTATSAACT ATTTTGTAAT G

<210> 21
<211> 31
<212> DNA
<213> unknown
<308> J02621
<400> 21
TTTTTATCAA CTATTYTGTA ATGCAAGTTT T

<210> 22
<211> 31
<212> DNA
<213> unknown
<308> J02621
<400> 22
TTGTTTATTT TTTGGYACAA CCAGAAAATA G

<210> 23
<211> 31
<212> DNA
<213> unknown
<308> J02621
<400> 23
TTTGGTACAA CCAGARAATA GTGTGGGATA T

```
<210> 24
<211> 31
<212> DNA
<213> unknown
<308> J02621
<400> 24
TACAACCAGA AAATASTGTG GGATATTGAA T

<210> 25
<211> 31
<212> DNA
<213> unknown
<308> J02621
<400> 25
GTGGGATATT GAATTRTGGG AGGCTCTGAC T

<210> 26
<211> 31
<212> DNA
<213> unknown
<308> J02621
<400> 26
GAATTATGGG AGGCTYTGAC TGTCTCGGGT G

<210> 27
<211> 31
<212> DNA
<213> unknown
<308> J02621
<400> 27
AATACAAAGC ATATTMAATG GCAATATGGA G

<210> 28
<211> 31
<212> DNA
<213> unknown
<308> J02621
<400> 28
TAAATTACTT CTATTMCCAT GTTGTTTTTT A

<210> 29
<211> 31
<212> DNA
<213> unknown
<308> J02621
<400> 29
TTACTTCTAT TACCAYGTTG TTTTTTAGTA G
```

```
<210> 30
<211> 31
<212> DNA
<213> unknown
<308> J02621
<400> 30
TTACCATGTT GTTTTKTAGT AGAATTGTTT C

<210> 31
<211> 31
<212> DNA
<213> unknown
<308> J02621
<400> 31
GAATTGTGTG CACTCKGTAA CATCTTTGGT T

<210> 32
<211> 31
<212> DNA
<213> unknown
<308> J02621
<400> 32
TGTAGTGTAC GTGCTRTTGA GTTGTGAACT G

<210> 33
<211> 31
<212> DNA
<213> unknown
<308> K01160
<400> 33
GATGCTGGGG GCCCTYGCCC TGACCACCGT G

<210> 34
<211> 31
<212> DNA
<213> unknown
<308> K01160
<400> 34
CCCTGACCAC CGTGAYGAGC CCCTGTGGAG G

<210> 35
<211> 31
<212> DNA
<213> unknown
<308> K01160
<400> 35
CACCGTGATG AGCCCYTGTG GAGGTGAAGA C
```

```
<210> 36
<211> 31
<212> DNA
<213> unknown
<308> K01160
<400> 36
CATTGTGGCT GACCAYGTCG CCTCTTATGG T


<210> 37
<211> 31
<212> DNA
<213> unknown
<308> K01160
<400> 37
TGTGGCTGAC CACGTYGCCT CTTATGGTGT A


<210> 38
<211> 31
<212> DNA
<213> unknown
<308> K01160
<400> 38
CCACGTCGCC TCTTAYGGTG TAAACTTGTA C


<210> 39
<211> 31
<212> DNA
<213> unknown
<308> K01160
<400> 39
CTTGTACCAG TCTTAYGGTC CCTCTGGCCA G


<210> 40
<211> 31
<212> DNA
<213> unknown
<308> K01160
<400> 40
CCTCTGGCCA GTACASCCAT GAATTTGATG G


<210> 41
<211> 31
<212> DNA
<213> unknown
<308> K01160
<400> 41
GACAAACATC GCTGTSCTAA AACATAACTT G
```

<210> 42
<211> 31
<212> DNA
<213> unknown
<308> K01160
<400> 42
CGCTGTCCTA AAACAYAACT TGAACAGTCT G

<210> 43
<211> 31
<212> DNA
<213> unknown
<308> K01160
<400> 43
ACGCTCCAAC TCTACYGCTG CTACCAATGA G

<210> 44
<211> 31
<212> DNA
<213> unknown
<308> K01160
<400> 44
CAAGTCTCCC GTGACRCTGG GTCAGCCCAA C

<210> 45
<211> 31
<212> DNA
<213> unknown
<308> K01160
<400> 45
TGGGTCAGCC CAACAYCCTC ATCTGTCTTG T

<210> 46
<211> 31
<212> DNA
<213> unknown
<308> K01160
<400> 46
TGTGGTCAAC ATCACMTGGC TGAGCAATGG G

<210> 47
<211> 31
<212> DNA
<213> unknown
<308> K01160
<400> 47
CTTCTGCTGA GGAGAKTTAT GACTGCAAGG T

```
<210> 48
<211> 31
<212> DNA
<213> unknown
<308> K01160
<400> 48
GGTGGAGCAC TGGGGMCTGG ACAAGCCTCT T


<210> 49
<211> 31
<212> DNA
<213> unknown
<308> K01160
<400> 49
CACTGGGGCC TGGACRAGCC TCTTCTGAAA C


<210> 50
<211> 31
<212> DNA
<213> unknown
<308> K01160
<400> 50
GCCTGAGATT CCAGCMCCTA TGTCAGAGCT C


<210> 51
<211> 31
<212> DNA
<213> unknown
<308> K01160
<400> 51
AGAGACTGTG GTCTGYGCCC TGGGATTGTC T


<210> 52
<211> 31
<212> DNA
<213> unknown
<308> K01160
<400> 52
GGTCTGCGCC CTGGGRTTGT CTGTGGGCCT C


<210> 53
<211> 31
<212> DNA
<213> unknown
<308> K01160
<400> 53
CTGTCTTCAT CATCCRAGGC CTGCGTTCAG T
```

```
<210> 54
<211> 31
<212> DNA
<213> unknown
<308> K01160
<400> 54
CAGACACCAA GGGCCMTTGT GAATCCCATC C


<210> 55
<211> 31
<212> DNA
<213> unknown
<308> K01160
<400> 55
AATCCCATCC TGGAAKGGAA GGTGCATCGC C


<210> 56
<211> 31
<212> DNA
<213> unknown
<308> K01160
<400> 56
AGAGTGGACT TGCTAMATGA CCTAGCATTA T


<210> 57
<211> 31
<212> DNA
<213> unknown
<308> K01160
<400> 57
TATCTGCTCA GAGCTYACAA ATGCCTTTGA A


<210> 58
<211> 31
<212> DNA
<213> unknown
<308> K01160
<400> 58
TGATTTTTTT CTTCTYAAGT GTTACCTACT A


<210> 59
<211> 31
<212> DNA
<213> unknown
<308> K01160
<400> 59
GTTACCTACT AAGAGWTGCC TGGAGTAAGC C
```

```
<210> 60
<211> 31
<212> DNA
<213> unknown
<308> K01160
<400> 60
CTAAGAGTTG CCTGGRGTAA GCCACCCAGC T


<210> 61
<211> 31
<212> DNA
<213> unknown
<308> K01160
<400> 61
TGCCTGGAGT AAGCCRCCCA GCTACCTAAT T


<210> 62
<211> 31
<212> DNA
<213> unknown
<308> K01160
<400> 62
AACAAATTCC CTTTAWGAGA TATATGTCAA A


<210> 63
<211> 31
<212> DNA
<213> unknown
<308> K01160
<400> 63
TTCCCTTTAT GAGATMTATG TCAAATTTTT C


<210> 64
<211> 31
<212> DNA
<213> unknown
<308> K01160
<400> 64
TGAAACCGTG GCTAASAATT GGGAGACTCT C


<210> 65
<211> 31
<212> DNA
<213> unknown
<308> K01160
<400> 65
CGTGGCTAAG AATTGKGAGA CTCTCTTGTT T
```

```
<210> 66
<211> 31
<212> DNA
<213> unknown
<308> K01160
<400> 66
TTTACCAGAT CATTTKTCAT GTCCAGTAAC A

<210> 67
<211> 31
<212> DNA
<213> unknown
<308> L12168
<400> 67
CATTACAGTA GATAAYTGTA AGAAACTTGG C

<210> 68
<211> 31
<212> DNA
<213> unknown
<308> L12168
<400> 68
AGATAATCAA CAGTARGGAT GTCAAAGTTC A

<210> 69
<211> 31
<212> DNA
<213> unknown
<308> L12168
<400> 69
TTCCCTGGAT TGTGARATAG TCAGTGCCAA A

<210> 70
<211> 31
<212> DNA
<213> unknown
<308> L12168
<400> 70
GCTTCTCTGC TCTGARAAGC ACAGCTACCT G

<210> 71
<211> 31
<212> DNA
<213> unknown
<308> L12168
<400> 71
GAGCATGAAG GTAGCRGAAG CTGGTGTGTT T
```

```
<210> 72
<211> 31
<212> DNA
<213> unknown
<308> M15661
<400> 72
TGTATGCCCA GGGAARGAGG CGCTATGATC G

<210> 73
<211> 31
<212> DNA
<213> unknown
<308> M29064
<400> 73
TAATGCAGAA GTAAGRAAGG CTTTGTCTAG A

<210> 74
<211> 31
<212> DNA
<213> unknown
<308> M37766
<400> 74
GCTCTGGAAT TGCTAYTGCT GCCTCTGTCA C

<210> 75
<211> 31
<212> DNA
<213> unknown
<308> M37766
<400> 75
AGCATTCAAG GTCACYTGGT ACATATGACC G

<210> 76
<211> 31
<212> DNA
<213> unknown
<308> M37766
<400> 76
TCTAAGGTCC AGAAASAGGA CAACAGCACC T

<210> 77
<211> 31
<212> DNA
<213> unknown
<308> M37766
<400> 77
CCCTTATGCC ACATAWTTAC TCCAGGTGTT A
```

```
<210> 78
<211> 31
<212> DNA
<213> unknown
<308> M37766
<400> 78
AAACTTCAAG GCCAGMAGAT CTTGCCTGTT G

<210> 79
<211> 31
<212> DNA
<213> unknown
<308> U05340
<400> 79
TGCGCCAGAG GGTTAYCAGA ACAGACTGAA A

<210> 80
<211> 31
<212> DNA
<213> unknown
<308> U05340
<400> 80
CCAGAGGGTT ATCAGRACAG ACTGAAAGTA C

<210> 81
<211> 31
<212> DNA
<213> unknown
<308> U05340
<400> 81
CTCCTGGCTC CAGCCSGAAG ACCTGCCGTT A

<210> 82
<211> 31
<212> DNA
<213> unknown
<308> U05340
<400> 82
TGTCCAGTGG TTCACRTTCT GGCCACATCC A

<210> 83
<211> 31
<212> DNA
<213> unknown
<308> U05340
<400> 83
GGTAGCAGAA CACCAYGTGG CCACACTGAG T
```

<210> 84
<211> 31
<212> DNA
<213> unknown
<308> U05340
<400> 84
GTGGGCTGCG CTGGGYCCCA GATGGACGAC A

<210> 85
<211> 31
<212> DNA
<213> unknown
<308> U05340
<400> 85
TTTGGCCAGT GGTGGYAATG ATAACTTGGT C

<210> 86
<211> 31
<212> DNA
<213> unknown
<308> U05340
<400> 86
TGATAACTTG GTCAAWGTGT GGCCTAGTGC T

<210> 87
<211> 31
<212> DNA
<213> unknown
<308> U05340
<400> 87
GGCAGTCCAA TGTCCYGGCA ACAGGAGGGG G

<210> 88
<211> 31
<212> DNA
<213> unknown
<308> U05340
<400> 88
AGTGATCGAC ACATTYGCAT CTGGAATGTG T

<210> 89
<211> 31
<212> DNA
<213> unknown
<308> U05340
<400> 89
CATTCGCATC TGGAAYGTGT GCTCTGGGGC C

```
<210> 90
<211> 31
<212> DNA
<213> unknown
<308> U05340
<400> 90
GTGCCGTGGA TGCCCWTTCC CAGGTGTGCT C


<210> 91
<211> 31
<212> DNA
<213> unknown
<308> U05340
<400> 91
CCTCTGGTCT CCCCAYTACA AGGAGCTCAT C


<210> 92
<211> 31
<212> DNA
<213> unknown
<308> U05340
<400> 92
CATCCCGGGT CCTGASTCTG ACCATGAGCC C


<210> 93
<211> 31
<212> DNA
<213> unknown
<308> U05340
<400> 93
CAGCAGATGA GACCCYGAGG CTATGGCGCT G


<210> 94
<211> 31
<212> DNA
<213> unknown
<308> U05340
<400> 94
GCAGCCAAAA GCAGCYTCAT CCACCAAGGC A


<210> 95
<211> 31
<212> DNA
<213> unknown
<308> U43522
<400> 95
GACTGCAATG TGCCGRTCTT AGCTGCTGCC T
```

```
<210> 96
<211> 31
<212> DNA
<213> unknown
<308> U43522
<400> 96
GTCCGAGCCC CTGAGYCGAG TAAAGTTGGG C

<210> 97
<211> 31
<212> DNA
<213> unknown
<308> U43522
<400> 97
AGTAAAGTTG GGCACRTTAC GCCGGCCTGA A


<210> 98
<211> 31
<212> DNA
<213> unknown
<308> U43522
<400> 98
CTTCAATCCT GGGAARAACT TCAAACTGGT C

<210> 99
<211> 31
<212> DNA
<213> unknown
<308> U43522
<400> 99
ACTGGCTGCA CCCACMGATG ACAGTGGGTG A

<210> 100
<211> 31
<212> DNA
<213> unknown
<308> U43522
<400> 100
GCACCCACAG ATGACRGTGG GTGAGGTGCA G

<210> 101
<211> 31
<212> DNA
<213> unknown
<308> U43522
<400> 101
AACCCTGCGA AGGCCYGGAG GTCCACAGTA T
```

```
<210> 102
<211> 31
<212> DNA
<213> unknown
<308> U43522
<400> 102
TGTCTACACA AATCAYAAAG GGGAGAAAAT C

<210> 103
<211> 31
<212> DNA
<213> unknown
<308> U43522
<400> 103
TTTATATGAA TGATAMGTCC CCATTGACGC C

<210> 104
<211> 31
<212> DNA
<213> unknown
<308> U43522
<400> 104
TAACCTGGAC CGGACYGATG ACCTGGTGTA C


<210> 105
<211> 31
<212> DNA
<213> unknown
<308> U43522
<400> 105
CAAGATGCGG CTGGCRCAGC AGAACGCCGT G

<210> 106
<211> 31
<212> DNA
<213> unknown
<308> U43522
<400> 106
CGCCGTGACC TCCCTRAGTG AGGAGTGCAA G

<210> 107
<211> 31
<212> DNA
<213> unknown
<308> U43522
<400> 107
GATGCTGACG GCTTCRCACA CCCTGGCTGT G
```

```
<210> 108
<211> 31
<212> DNA
<213> unknown
<308> U43522
<400> 108
GTGGGTCTTC CAGGGRGAAG GCCAAGGGGA G


<210> 109
<211> 31
<212> DNA
<213> unknown
<308> U43522
<400> 109
CCCCTAGCTT TATATRTGGA CATGGCAGGC C


<210> 110
<211> 31
<212> DNA
<213> unknown
<308> U46692
<400> 110
CAGAGCGTGC ACTTGYGATC CTAAAATAAG C


<210> 111
<211> 31
<212> DNA
<213> unknown
<308> U66711
<400> 111
CAAGTCCCAT GGACAKGCTG ACAGGGTCCC C


<210> 112
<211> 31
<212> DNA
<213> unknown
<308> U66711
<400> 112
CTGTGTACGT GGGTGKGCAG TGCACGTGAG A


<210> 113
<211> 31
<212> DNA
<213> unknown
<308> X05276
<400> 113
AACTTGCCCA GGCCARAGAA GAGAACGTGG G
```

```
<210> 114
<211> 31
<212> DNA
<213> unknown
<308> X05276
<400> 114
TTCGCTTTGC TGGAARTGTC AAGCAAATTA T

<210> 115
<211> 31
<212> DNA
<213> unknown
<308> X05276
<400> 115
CCCCACACTT TGAAGMATAC AGACCCCAGT A

<210> 116
<211> 31
<212> DNA
<213> unknown
<308> X05276
<400> 116
TGCAGGACAA ACATCYTCTC AAGCAGTCAA C

<210> 117
<211> 31
<212> DNA
<213> unknown
<308> X05276
<400> 117
TCTCAAGCAG TCAACSTAGA ATGCTTGGGA A

<210> 118
<211> 31
<212> DNA
<213> unknown
<308> X05276
<400> 118
TTCGAGACCA GCCCARCCAA CATGGCGAAA C

<210> 119
<211> 31
<212> DNA
<213> unknown
<308> X60221
<400> 119
AAGAATGCAG CCTTCYTAGG TCCAGGGGTA T
```

<210> 120
<211> 31
<212> DNA
<213> unknown
<308> X60221
<400> 120
TTTCTTTATC CTAAARCTGG TGTAACAGGA C

<210> 121
<211> 31
<212> DNA
<213> unknown
<308> X60221
<400> 121
AACAGGACCC TATGTRCTCG GAACTGGGCT T

<210> 122
<211> 31
<212> DNA
<213> unknown
<308> X60221
<400> 122
CCTTTTTGAT GTGCARAGGA ATAACATTGC T

<210> 123
<211> 31
<212> DNA
<213> unknown
<308> X60221
<400> 123
GGACTATCAT ATATCYGTGC AGAACATGAT G

<210> 124
<211> 31
<212> DNA
<213> unknown
<308> Y00097
<400> 124
ACTCTGCTGA AGCTGKCTGG GGGAGATGAT G

<210> 125
<211> 31
<212> DNA
<213> unknown
<308> Y00097
<400> 125
AATAGCAGAC ACACCYAGTG GAGACAAAAC T

```
<210> 126
<211> 31
<212> DNA
<213> unknown
<308> Y00097
<400> 126
ATTCATTGAG AAATAYGACA AGTCTCTCCA C


<210> 127
<211> 31
<212> DNA
<213> unknown
<308> Y00097
<400> 127
AATATGACAA GTCTCYCCAC CAAGCCATTG A


<210> 128
<211> 31
<212> DNA
<213> unknown
<308> Z48950
<400> 128
AAGCGCTCCC TCTACYGGCG GGGTGAAGAA G


<210> 129
<211> 31
<212> DNA
<213> unknown
<308> Z48950
<400> 129
CTCTAGCTGA TAATGSAAAC ACTAACTGGG G


<210> 130
<211> 31
<212> DNA
<213> unknown
<308> Z48950
<400> 130
CTCTGCTGAG GAAACRGTAC CGAAGTTCTT T


<210> 131
<211> 31
<212> DNA
<213> unknown
<308> Z49107
<400> 131
CTGGGAGGGC TGTACYCATC CAAGTCCATC C
```

<210> 132
<211> 31
<212> DNA
<213> unknown
<308> D11086
<400> 132
CCTCACTCTG CATTAWTGGT ACAAGAACTC G


<210> 133
<211> 31
<212> DNA
<213> unknown
<308> D11086
<400> 133
GCTAGAACTG AACTGSAACA ACAGATTCTT G


<210> 134
<211> 31
<212> DNA
<213> unknown
<308> D11086
<400> 134
GATTGATTAT CAGCCWTCTC TGTGTGTATT T


<210> 135
<211> 31
<212> DNA
<213> unknown
<308> D11086
<400> 135
TGTGCCCCCA TGTAASCACC CCTTCATTTG G


<210> 136
<211> 31
<212> DNA
<213> unknown
<308> D38076
<400> 136
CCGCCAAGGA CACTCRTGAG GACCATGATA C


<210> 137
<211> 31
<212> DNA
<213> unknown
<308> D38076
<400> 137
AGAATACAGA CGAGTMCAAC CATGACCCTC A

```
<210> 138
<211> 31
<212> DNA
<213> unknown
<308> D38076
<400> 138
AACTTTTTAA AATGCRGGCA AAACTGTTCC G

<210> 139
<211> 31
<212> DNA
<213> unknown
<308> D38076
<400> 139
CGGGCAAAAC TGTTCYGATT TGCCTCTGAG A

<210> 140
<211> 31
<212> DNA
<213> unknown
<308> D38076
<400> 140
GTTCCGATTT GCCTCWGAGA ACGATCTCCC A

<210> 141
<211> 31
<212> DNA
<213> unknown
<308> D38076
<400> 141
TCCGCCTCCT CATGCRGAGG GACAAGACCC T

<210> 142
<211> 31
<212> DNA
<213> unknown
<308> D38076
<400> 142
GGACAAGACC CTGAAKATCT GTGCCAACCA C

<210> 143
<211> 31
<212> DNA
<213> unknown
<308> D38076
<400> 143
GGAGCTGAAG CCCAAMGCAG GTAGCGACCG T
```

```
<210> 144
<211> 31
<212> DNA
<213> unknown
<308> D38076
<400> 144
GCCCAACGCA GGTAGYGACC GTGCCTGGGT C

<210> 145
<211> 31
<212> DNA
<213> unknown
<308> D38076
<400> 145
GGTCTGGAAC ACCCAYGCTG ACTTCGCCGA C

<210> 146
<211> 31
<212> DNA
<213> unknown
<308> D38076
<400> 146
GCTGACTTCG CCGACSAGTG CCCCAAGCCA G

<210> 147
<211> 31
<212> DNA
<213> unknown
<308> D38076
<400> 147
GAGCTGCTGG CCATCYGCTT CCTGAATGCT G

<210> 148
<211> 31
<212> DNA
<213> unknown
<308> D38076
<400> 148
GTTTGAAGAA TGCAGRAAAG AGATCGAAGA G

<210> 149
<211> 31
<212> DNA
<213> unknown
<308> D38076
<400> 149
AAAAATGATC ATGCCRAAAA AGTGGCGGAA A
```

```
<210> 150
<211> 31
<212> DNA
<213> unknown
<308> D38076
<400> 150
CCTCTTTTTC GGTTTRTTTT TATTCTTTCA T

<210> 151
<211> 31
<212> DNA
<213> unknown
<308> D87017
<400> 151
GAAGCTACAG CTGCCRGGTC ACGCATGAAG G

<210> 152
<211> 31
<212> DNA
<213> unknown
<308> L06132
<400> 152
TACAGAGAAA TGGAAYACCG ACAATACACT A

<210> 153
<211> 31
<212> DNA
<213> unknown
<308> L06132
<400> 153
CGAGGGCTGG CTGGCYGGCT ACCAGATGAA T

<210> 154
<211> 31
<212> DNA
<213> unknown
<308> L06132
<400> 154
CTATTTTGCA GCATASCTAC CTTCAGAATT T

<210> 155
<211> 31
<212> DNA
<213> unknown
<308> L06132
<400> 155
GGTACAGAAG AAACCYATTT CCAAAAAAGG T
```

<210> 156
<211> 31
<212> DNA
<213> unknown
<308> L06132
<400> 156
TCTAGAAATG GCTGCRAGTG GAAGCGGATA A

<210> 157
<211> 31
<212> DNA
<213> unknown
<308> L06132
<400> 157
AATGGCTGCA AGTGGWAGCG GATAATATGT A

<210> 158
<211> 31
<212> DNA
<213> unknown
<308> L06132
<400> 158
AATCGAACCT TGGTTYCCTA ACCCTAATTG A

<210> 159
<211> 31
<212> DNA
<213> unknown
<308> L06132
<400> 159
ACCTTGGTTC CCTAAYCCTA ATTGATGAGA G

<210> 160
<211> 31
<212> DNA
<213> unknown
<308> L06132
<400> 160
TGGTTCCCTA ACCCTRATTG ATGAGAGGCT C

<210> 161
<211> 31
<212> DNA
<213> unknown
<308> L06132
<400> 161
GGTTCCCTAA CCCTAMTTGA TGAGAGGCTC G

```
<210> 162
<211> 31
<212> DNA
<213> unknown
<308> L06132
<400> 162
GTTCCCTAAC CCTAAYTGAT GAGAGGCTCG C

<210> 163
<211> 31
<212> DNA
<213> unknown
<308> L06132
<400> 163
TTTTTAGACA GATCTYCATG ACCTGTTCCC A

<210> 164
<211> 31
<212> DNA
<213> unknown
<308> L06132
<400> 164
CAGCTATAAG CTTGGRAGTG TCTGTGTGAT T

<210> 165
<211> 31
<212> DNA
<213> unknown
<308> L06132
<400> 165
AATCACATGG TGACARCACT CAGAATCTAA A

<210> 166
<211> 31
<212> DNA
<213> unknown
<308> L06132
<400> 166
ACTCAATTTG TTTTTYAGCA GTTTAATGGG T

<210> 167
<211> 31
<212> DNA
<213> unknown
<308> M19311
<400> 167
ACTTCGCCAT GTGATKACAA ACCTTGGAGA G
```

<210> 168
<211> 31
<212> DNA
<213> unknown
<308> M19311
<400> 168
GCATGTGGCT TACTCYGGCT ATATCTAAGC C

<210> 169
<211> 31
<212> DNA
<213> unknown
<308> M19311
<400> 169
CACATCTAAA CTTAGRTGGA GTTGGTCAAA T

<210> 170
<211> 31
<212> DNA
<213> unknown
<308> M22760
<400> 170
TTCGAATCCT AGAGGYTGTT AAGGACAAAG C

<210> 171
<211> 31
<212> DNA
<213> unknown
<308> M22760
<400> 171
TGTCATCCAG GAACTYAGAC CAACTTTAAA T

<210> 172
<211> 31
<212> DNA
<213> unknown
<308> M58028
<400> 172
TACCTTTAGC ATCTGYGACA CCTCCAACTT C

<210> 173
<211> 31
<212> DNA
<213> unknown
<308> M58028
<400> 173
TTGGTGGCCT CACTGSCAGA ACCTGACTTT G

```
<210> 174
<211> 31
<212> DNA
<213> unknown
<308> M58028
<400> 174
TTCTCGCCCT GCCCASCTGC ACATTGGCTT C


<210> 175
<211> 31
<212> DNA
<213> unknown
<308> M58028
<400> 175
ACAAGTGCCT CCAGCRCCAG AACCGTTATG A


<210> 176
<211> 31
<212> DNA
<213> unknown
<308> M58028
<400> 176
CTGACTGCGT GACCTSGGCC TGCCACCACT G


<210> 177
<211> 31
<212> DNA
<213> unknown
<308> M58028
<400> 177
AAGCTCAGGA GCGCCRTTCT GGTCTGGGCC C


<210> 178
<211> 31
<212> DNA
<213> unknown
<308> M58028
<400> 178
CCGAGCTGCT GTGGCYACAT TCCTGCAGTC T


<210> 179
<211> 31
<212> DNA
<213> unknown
<308> M58028
<400> 179
ACCAGGAGCT GCAGASCGCC AATGCCTCTG T
```

```
<210> 180
<211> 31
<212> DNA
<213> unknown
<308> M60784
<400> 180
CCCTTTCTAT GACAARCCTA TGCGTATCCA G

<210> 181
<211> 31
<212> DNA
<213> unknown
<308> U23852
<400> 181
TTGACTACCT GCGCASTGTG CTGGAGGACT T


<210> 182
<211> 31
<212> DNA
<213> unknown
<308> U23852
<400> 182
CTGCACATGT CTTGTRCATG TGTAGCCTGT G

<210> 183
<211> 31
<212> DNA
<213> unknown
<308> U23852
<400> 183
ATGTATGTCT TGGACRCTGT ACAAGGTACC C

<210> 184
<211> 31
<212> DNA
<213> unknown
<308> U30825
<400> 184
GACCGACGTG CGCGARAAGG ACTTGGAGGA C

<210> 185
<211> 31
<212> DNA
<213> unknown
<308> U30825
<400> 185
TGGAGGACCT GTTCTWCAAG TACGGCCGCA T
```

```
<210> 186
<211> 31
<212> DNA
<213> unknown
<308> U30825
<400> 186
ACCTGAAGGA TCACAYGCGA GAAGCTGGGG A

<210> 187
<211> 31
<212> DNA
<213> unknown
<308> U60975
<400> 187
GCCACAGTAA CAACCRCACC AATTTATACA T

<210> 188
<211> 31
<212> DNA
<213> unknown
<308> U60975
<400> 188
TGGCTCAGCG CCTCASTCAG CTCCTCAACC T


<210> 189
<211> 31
<212> DNA
<213> unknown
<308> U60975
<400> 189
TCTAGCAGTG CTGGARCCAG GTGGCGAGAG G

<210> 190
<211> 31
<212> DNA
<213> unknown
<308> U60975
<400> 190
CATGGGGAGA CCACGRCGGA ATCATCACGG C

<210> 191
<211> 31
<212> DNA
<213> unknown
<308> U60975
<400> 191
TTCTGGCCTG GAGACWGTAG AAGCTTTGGC T
```

<210> 192
<211> 31
<212> DNA
<213> unknown
<308> U60975
<400> 192
AATACCTGTG TCAAASAAGA GAACACCTGT C

<210> 193
<211> 31
<212> DNA
<213> unknown
<308> U60975
<400> 193
CTGCAGGGAC TGGTCKGATG AAGCCAACTG T

<210> 194
<211> 31
<212> DNA
<213> unknown
<308> U60975
<400> 194
GTGTGACGGG GATACRGACT GCCAGGATGG T

<210> 195
<211> 31
<212> DNA
<213> unknown
<308> U60975
<400> 195
ACTGCCAGGA TGGTTSCGAT GAGGATCCAG T

<210> 196
<211> 31
<212> DNA
<213> unknown
<308> U60975
<400> 196
TCAACTGTGA GAAGARGTGC AATGGATTCC G

<210> 197
<211> 31
<212> DNA
<213> unknown
<308> U60975
<400> 197
ATTCCGCTGC CCAAAYGGCA CTTGCATCCC A

<210> 198
<211> 31
<212> DNA
<213> unknown
<308> U60975
<400> 198
TGATGAAGCC AACTGMGAAA ACCCCACAGA A

<210> 199
<211> 31
<212> DNA
<213> unknown
<308> U60975
<400> 199
ACCGAGATTG TGCAGRTGGC TCTGACGAGG A

<210> 200
<211> 31
<212> DNA
<213> unknown
<308> U60975
<400> 200
AAAAATGCCC TCTGCWTCTT GTGTATATAA T

<210> 201
<211> 31
<212> DNA
<213> unknown
<308> U60975
<400> 201
CAATCTGACA GCTCAWACAT CCTATGAGAT T

<210> 202
<211> 31
<212> DNA
<213> unknown
<308> U60975
<400> 202
CCTCTATCGC AACCCRAAGA GCTTGACTAC T

<210> 203
<211> 31
<212> DNA
<213> unknown
<308> U60975
<400> 203
GACTTGTTGT ATGCARTTGC AGTCAAAGAT C

```
<210> 204
<211> 31
<212> DNA
<213> unknown
<308> U60975
<400> 204
CACCGGAGGC TGCAGRGCAG CTTCACCGCC T

<210> 205
<211> 31
<212> DNA
<213> unknown
<308> X07743
<400> 205
AGCATGGAAC CAAAGYGGAT CAGAGAGGGC T

<210> 206
<211> 31
<212> DNA
<213> unknown
<308> X07743
<400> 206
GGTTCGGGAT ATCAAKAAGG CCATTAAATG C

<210> 207
<211> 31
<212> DNA
<213> unknown
<308> X07743
<400> 207
CATGATTGCT TCATCRCTGC TCAATGAGGG G

<210> 208
<211> 31
<212> DNA
<213> unknown
<308> X07743
<400> 208
CTGAAAACCC TTTCCKGGAC AACCCTGATG C

<210> 209
<211> 31
<212> DNA
<213> unknown
<308> X07743
<400> 209
GCACAGAGTG GATCARAGCC ATCCAGATGG C
```

```
<210> 210
<211> 31
<212> DNA
<213> unknown
<308> X07743
<400> 210
ACTGGGAAGT AAAGASACTC CTGCATTCCT C

<210> 211
<211> 31
<212> DNA
<213> unknown
<308> X07743
<400> 211
CCTGAGGGAA GCCCAYGGAC AAGCTCAGTC C

<210> 212
<211> 31
<212> DNA
<213> unknown
<308> X07743
<400> 212
TTCCTGAGGT CCCCCYAGCT TAAAAAAAAA A

<210> 213
<211> 31
<212> DNA
<213> unknown
<308> X07743
<400> 213
AAAAAAATCT GCCCCRTGAT TCTAACACTC G

<210> 214
<211> 31
<212> DNA
<213> unknown
<308> X07743
<400> 214
ATCTTCTACT TGCTCYTGGC CTTGAGATCG T

<210> 215
<211> 31
<212> DNA
<213> unknown
<308> X07743
<400> 215
CTGGTGAATG TGTCTYGCGG CAGCTGCAGC A
```

```
<210> 216
<211> 31
<212> DNA
<213> unknown
<308> X07743
<400> 216
GGTGAATGTG TCTTGYGGCA GCTGCAGCAA G


<210> 217
<211> 31
<212> DNA
<213> unknown
<308> X07743
<400> 217
ACAGCACCCA TTGAARCAGA TATGTGTGTG A


<210> 218
<211> 31
<212> DNA
<213> unknown
<308> X07743
<400> 218
CCCATTGAAA CAGATRTGTG TGTGAAAGTA T


<210> 219
<211> 31
<212> DNA
<213> unknown
<308> X51521
<400> 219
CCTGAAGATG TGGCTSAGGA GCTCATCCAG G


<210> 220
<211> 31
<212> DNA
<213> unknown
<308> X51521
<400> 220
CCCCCCTGAG ACTGCYGTGC TCTTGGGGTC C


<210> 221
<211> 31
<212> DNA
<213> unknown
<308> X51521
<400> 221
GCAGGCCTGA TTCTCRCGAT TATTCTCGAA T
```

<210> 222
<211> 31
<212> DNA
<213> unknown
<308> X51521
<400> 222
GGAGGACACT TGGATWTTTT TTTAGTTCTT T

<210> 223
<211> 31
<212> DNA
<213> unknown
<308> X51521
<400> 223
ATTAATGATC AGCTAYATAC TATTTATATA C

<210> 224
<211> 31
<212> DNA
<213> unknown
<308> X51521
<400> 224
CTATGCACAC TTTTAWTTTT GTCAGATTCA C

<210> 225
<211> 31
<212> DNA
<213> unknown
<308> X51521
<400> 225
GGTGGATTCC ATGCCRCAGA CATGAAATAA A

<210> 226
<211> 31
<212> DNA
<213> unknown
<308> X52882
<400> 226
ATTAAATACA CAGACRTAAG AGGCCAGCCA C

<210> 227
<211> 31
<212> DNA
<213> unknown
<308> X52882
<400> 227
GGTGGGATCC CAGGGYATGC CCAAGAGAAT C

<210> 228
<211> 31
<212> DNA
<213> unknown
<308> X52882
<400> 228
ATGATTTCAT GTGTGRTGAG ATGGAGCGCT C

<210> 229
<211> 31
<212> DNA
<213> unknown
<308> X52882
<400> 229
TTCTTGTTAT TCCCARTACA CTAGCAGTTA A

<210> 230
<211> 31
<212> DNA
<213> unknown
<308> X52882
<400> 230
GAATTGATGA TCTTAYTAAA TTACATCCAG A

<210> 231
<211> 31
<212> DNA
<213> unknown
<308> AC002115
<400> 231
CAAGAACTAC AAGACYGCCC CTTTTGACAG C

<210> 232
<211> 31
<212> DNA
<213> unknown
<308> AC002115
<400> 232
TAATAAAAAC TCATTKGAAA AGTGAGACTA T

<210> 233
<211> 31
<212> DNA
<213> unknown
<308> D13118
<400> 233
CACAGCAGCC AAGTTKATTG GTGCTGGGGC A

```
<210> 234
<211> 31
<212> DNA
<213> unknown
<308> D13118
<400> 234
CATTGGCTAT GCCAGRAACC CGTCTCTCAA G

<210> 235
<211> 31
<212> DNA
<213> unknown
<308> D13118
<400> 235
TTTGATGGTC GCCTTYCTCA TCCTCTTCGC C

<210> 236
<211> 31
<212> DNA
<213> unknown
<308> D13118
<400> 236
TACTGCAACT CCACAYCATT CTTGGTGCTG G

<210> 237
<211> 31
<212> DNA
<213> unknown
<308> D13118
<400> 237
AACTCCACAC CATTCYTGGT GCTGGGGTGT G

<210> 238
<211> 31
<212> DNA
<213> unknown
<308> D13118
<400> 238
TGGTGCTGGG GTGTGYTAAG CTTTACCATT A

<210> 239
<211> 31
<212> DNA
<213> unknown
<308> D26068
<400> 239
ACGGCTAGTC AGAGAMAAAG ACACAGATAA A
```

```
<210> 240
<211> 31
<212> DNA
<213> unknown
<308> D26068
<400> 240
TAAATTTAAA GGATTYTGCT ATGTAGAATT C

<210> 241
<211> 31
<212> DNA
<213> unknown
<308> D26068
<400> 241
AAGTCCCAGT CTGTCSTATG GGGAGCAGTT T

<210> 242
<211> 31
<212> DNA
<213> unknown
<308> D26068
<400> 242
AGTTTGGGGG CGGCCRGCAG CAGGAGCCTG G

<210> 243
<211> 31
<212> DNA
<213> unknown
<308> D31890
<400> 243
CTGCTGCTGC CACCARCCAC ACCACTGATA A

<210> 244
<211> 31
<212> DNA
<213> unknown
<308> D31890
<400> 244
GGGATCACCT GACTGMCATC ACCTTAAAGG T

<210> 245
<211> 31
<212> DNA
<213> unknown
<308> D31890
<400> 245
ACCAAGAAGG GTGAGSTGAG CATCATTCCG T
```

```
<210> 246
<211> 31
<212> DNA
<213> unknown
<308> D31890
<400> 246
CTTTGTGAGG CAGAAMTTTA TCATCCGCTC T

<210> 247
<211> 31
<212> DNA
<213> unknown
<308> D31890
<400> 247
GGTTGGTGGC ATCGASCGGG TTTATGAAAT T

<210> 248
<211> 31
<212> DNA
<213> unknown
<308> D31890
<400> 248
GCACAACAGT TGGCASTTCT GTCTAGAAAA T

<210> 249
<211> 31
<212> DNA
<213> unknown
<308> HT3238
<400> 249
CAGAACGCTT CAGTTSTGCT CTGCAAGGAT A

<210> 250
<211> 31
<212> DNA
<213> unknown
<308> HT3238
<400> 250
CTCTGCAAGG ATATAWAATA ACTGATTGGT G

<210> 251
<211> 31
<212> DNA
<213> unknown
<308> HT3238
<400> 251
GCATTCTGCT TCAGASCAAG AATGCTGGGG C
```

```
<210> 252
<211> 31
<212> DNA
<213> unknown
<308> HT3238
<400> 252
ACCAGCCAGC TCCCGYTCGA ATCTGATGCT G

<210> 253
<211> 31
<212> DNA
<213> unknown
<308> HT3238
<400> 253
TGCTGTGGAA TGCTTWAATT ACCAACACTA T

<210> 254
<211> 31
<212> DNA
<213> unknown
<308> HT3238
<400> 254
GGAATTATTG GGGTCRAAGG TGCTAAAATC A

<210> 255
<211> 31
<212> DNA
<213> unknown
<308> HT3238
<400> 255
GGGTTGTAGA GTGCAWAAAG ATCATCCTTG A

<210> 256
<211> 31
<212> DNA
<213> unknown
<308> HT3238
<400> 256
AGAGCTCGGA ATCTTYCTCT TCCTCCACCA C

<210> 257
<211> 31
<212> DNA
<213> unknown
<308> HT3238
<400> 257
GTGATCTTGG TGGACYTATT ATTACTACAC A
```

```
<210> 258
<211> 31
<212> DNA
<213> unknown
<308> HT3238
<400> 258
TTCTGGAAAG TTTTTYTAAG ACTAGTGAAG A


<210> 259
<211> 31
<212> DNA
<213> unknown
<308> HT3238
<400> 259
CCCATGTAAT TATTGYGAAC ACCTTGCTTT G


<210> 260
<211> 31
<212> DNA
<213> unknown
<308> HT3238
<400> 260
ATAGGCAAAA TAACTYGGCA AACTTAGTTC T


<210> 261
<211> 31
<212> DNA
<213> unknown
<308> HT3238
<400> 261
ATGGTTTGGA AGTCTRTTGC TGGGAAGAAA T


<210> 262
<211> 31
<212> DNA
<213> unknown
<308> M20471
<400> 262
AGTGGATCGA TTGCASTCAG AGCCTGAAAG T


<210> 263
<211> 31
<212> DNA
<213> unknown
<308> M20471
<400> 263
GTCTCCCGCA TGCGCYCAGT CCTCATCTCC C
```

```
<210> 264
<211> 31
<212> DNA
<213> unknown
<308> M20471
<400> 264
TCTTTTGGAC CAAACYTTTT TGTCTTTAGA G

<210> 265
<211> 31
<212> DNA
<213> unknown
<308> M22538
<400> 265
ATTTGCATAA GACAGYTATG CAAAATGGAG C


<210> 266
<211> 31
<212> DNA
<213> unknown
<308> M22538
<400> 266
CTTTTTATAC AATGTWTAAT CGAAAGCCAG T

<210> 267
<211> 31
<212> DNA
<213> unknown
<308> M22538
<400> 267
GTGTACAAGC AGGCCKTTAA TTTATATTGA A

<210> 268
<211> 31
<212> DNA
<213> unknown
<308> U10439
<400> 268
GTGCTAATTG ACATGRAAAG GCAGGGGGAT G

<210> 269
<211> 31
<212> DNA
<213> unknown
<308> U10439
<400> 269
CAATCCCTGA GACCARAAGA AACGCAGAGT T
```

<210> 270
<211> 31
<212> DNA
<213> unknown
<308> U10439
<400> 270
AAGCCAAGGA CAGTGRAAAA TCAGAAGAAT C

<210> 271
<211> 31
<212> DNA
<213> unknown
<308> U10439
<400> 271
AGGGAATCGC TGTGTRAAAG GAGATTCTCT C

<210> 272
<211> 31
<212> DNA
<213> unknown
<308> U10439
<400> 272
CACCAAGGTG GAGAAYGGAG AAGGCACAAT C

<210> 273
<211> 31
<212> DNA
<213> unknown
<308> U10439
<400> 273
AACGTGCTGG GCCTGMAAGG GGCACTGTTG A

<210> 274
<211> 31
<212> DNA
<213> unknown
<308> U10439
<400> 274
TGGCTGATGG CTATGRCCTG GAGATCCTGG A

<210> 275
<211> 31
<212> DNA
<213> unknown
<308> U10439
<400> 275
AAAAAGAACA TTTTTYTTCT ATTTAAGAAG C

<210> 276
<211> 31
<212> DNA
<213> unknown
<308> U10439
<400> 276
TTCTTTCCTT GTGATYTGAA TGTCTCCTTT T

<210> 277
<211> 31
<212> DNA
<213> unknown
<308> U10439
<400> 277
CCCTCAGAGG GCAAARAGGT GAACATAAAG G

<210> 278
<211> 31
<212> DNA
<213> unknown
<308> U10439
<400> 278
GCCTGCAGAT AATGCYCAGC CATCCTCCCA T

<210> 279
<211> 31
<212> DNA
<213> unknown
<308> U10439
<400> 279
TCTCTGCTCA GATAAYCATG ACTTAGCAAG A

<210> 280
<211> 31
<212> DNA
<213> unknown
<308> U10439
<400> 280
CGTCACTGTT CCACCYGGTG TAATATCTCT C

<210> 281
<211> 31
<212> DNA
<213> unknown
<308> U10439
<400> 281
GCAGACCTGA TCTTTMTAGG GTTGACATAG A

<210> 282
<211> 31
<212> DNA
<213> unknown
<308> U10439
<400> 282
CTGTCAGTGC TTGAARCTGT TTCCTTTACT C

<210> 283
<211> 31
<212> DNA
<213> unknown
<308> U10439
<400> 283
TTGGTGCCGT GGTGAWGGCT GCAGTCCAGT T

<210> 284
<211> 31
<212> DNA
<213> unknown
<308> U10439
<400> 284
CTTTGCTTTT ATTCTRTGCT CTCTGTACTT T

<210> 285
<211> 31
<212> DNA
<213> unknown
<308> U10439
<400> 285
TTGTCAGTGC TTGAARCTGT TTCCTTTACT C

<210> 286
<211> 31
<212> DNA
<213> unknown
<308> U10439
<400> 286
CTGCAGTCCA GTTTTRTGAT TCTGCTTTTA T

<210> 287
<211> 31
<212> DNA
<213> unknown
<308> U57342
<400> 287
CACGTTCCGG GCCGCSACCC TGACCCTGCA G

<210> 288
<211> 31
<212> DNA
<213> unknown
<308> U57342
<400> 288
AGGGCCCCGG GCCCTYAGCC TCTCTTGTAC A

<210> 289
<211> 31
<212> DNA
<213> unknown
<308> U57342
<400> 289
TTTCCTCTCG ATTCCYATCC CCAATTTAAT A

<210> 290
<211> 31
<212> DNA
<213> unknown
<308> U57342
<400> 290
TACCATTGAT GACTTYGCCT CTGCATCTAC T

<210> 291
<211> 31
<212> DNA
<213> unknown
<308> U57342
<400> 291
GCCCCCTCCA TCCCTYAGCT ACCCTGGATC T

<210> 292
<211> 31
<212> DNA
<213> unknown
<308> U57342
<400> 292
GGAGCAGACT GGTTCYTAAC TCTCTCCCTC C

<210> 293
<211> 31
<212> DNA
<213> unknown
<308> U62962
<400> 293
AATTGGGATG CAGCCRTGGA AGACCTTACA C

```
<210> 294
<211> 31
<212> DNA
<213> unknown
<308> U62962
<400> 294
GGGCAACTCA AGATTYTGGC TTCTACTGAA G

<210> 295
<211> 31
<212> DNA
<213> unknown
<308> X13238
<400> 295
TTTCTACAGA AACTAYGATG TCATGAAAGA T

<210> 296
<211> 31
<212> DNA
<213> unknown
<308> X14046
<400> 296
CATCTGGATC CTCATYGACA AGACCAGCTT C

<210> 297
<211> 31
<212> DNA
<213> unknown
<308> X14046
<400> 297
ACAGATCACC CTGGGMATCC TCATCTCCAC T

<210> 298
<211> 31
<212> DNA
<213> unknown
<308> X14046
<400> 298
TGGCACTACC CGCAGSACTG GTTCCAAGTC C

<210> 299
<211> 31
<212> DNA
<213> unknown
<308> X14046
<400> 299
CCCGCAGGAC TGGTTYCAAG TCCTCATCCT G
```

```
<210> 300
<211> 31
<212> DNA
<213> unknown
<308> X14046
<400> 300
TCACGTGCGC TGGGCWCTTC CCTGCTGCCT G


<210> 301
<211> 31
<212> DNA
<213> unknown
<308> X57346
<400> 301
AGGAAGGAAG AGGTCMTCTC GCTCGGAGCT T


<210> 302
<211> 31
<212> DNA
<213> unknown
<308> X57346
<400> 302
TGCAGCCCTC CCACGSCAGA GTCTCCAGAG A


<210> 303
<211> 31
<212> DNA
<213> unknown
<308> X57346
<400> 303
TGAGCAGGCT GAGCGMTATG ATGATATGGC T


<210> 304
<211> 31
<212> DNA
<213> unknown
<308> X57346
<400> 304
GCATGAACTC TCCAASGAAG AGAGAAATCT G


<210> 305
<211> 31
<212> DNA
<213> unknown
<308> X57346
<400> 305
TCGTGCTTTG TGATCYGTCC AGTGTCACTC T
```

<210> 306
<211> 31
<212> DNA
<213> unknown
<308> X74008
<400> 306
CTCCAAGGGG TATGAYCACA AAGCAAGCAA A

<210> 307
<211> 31
<212> DNA
<213> unknown
<308> X74008
<400> 307
AGGTGGTATG GTTGTRTACA CATGCTGCCT T

<210> 308
<211> 31
<212> DNA
<213> unknown
<308> Z29505
<400> 308
ACAGCTCCAT GACCAWCAGT ACCGCGGCCA G

<210> 309
<211> 31
<212> DNA
<213> unknown
<308> Z29505
<400> 309
ATCAAAGAGA TCCGCSAGAG TACGGGGGCG C

<210> 310
<211> 31
<212> DNA
<213> unknown
<308> Z37166
<400> 310
TGGGCAGGTG TCTGTRCTGG TGATGTGTCA C

<210> 311
<211> 31
<212> DNA
<213> unknown
<308> Z37166
<400> 311
TTTCAGATCA GCAAGRAATA TGAGCGCTTC T

```
<210> 312
<211> 31
<212> DNA
<213> unknown
<308> AC002045
<400> 312
TCTCCCTCTG CACAASTTAC CTTTGTGTGT C

<210> 313
<211> 31
<212> DNA
<213> unknown
<308> AC002045
<400> 313
GGTGGAAACC AAAGTWCGAG CTAAAATCTG T

<210> 314
<211> 31
<212> DNA
<213> unknown
<308> AC002045
<400> 314
GTGGAAACCA AAGTTMGAGC TAAAATCTGT A

<210> 315
<211> 31
<212> DNA
<213> unknown
<308> D26599
<400> 315
CACGTGAGAG GGCAGWGGAA CTCCTTAGGA A

<210> 316
<211> 31
<212> DNA
<213> unknown
<308> D30655
<400> 316
GTTCTCTATC ATTTARTAAT ATACTTGTGG A

<210> 317
<211> 31
<212> DNA
<213> unknown
<308> L08096
<400> 317
CCATTGGTCG CGGGCYTGGT GATCTGCCTC G
```

<210> 318
<211> 31
<212> DNA
<213> unknown
<308> L08096
<400> 318
AGCTGAATCA CACAGSACCT CAGCAGGACC C

<210> 319
<211> 31
<212> DNA
<213> unknown
<308> L08096
<400> 319
CCTGCATGGA CCAGARCTGG ACAAGGGGCA G

<210> 320
<211> 31
<212> DNA
<213> unknown
<08> L08096

<400> 320
CTGCATGGAC CAGAGMTGGA CAAGGGGCAG C

<210> 321
<211> 31
<212> DNA
<213> unknown
<308> L08096
<400> 321
TGCATGGACC AGAGCWGGAC AAGGGGCAGC T

<210> 322
<211> 31
<212> DNA
<213> unknown
<308> L08096
<400> 322
TCTACATGGT ACACAKCCAG GTGACGCTGG C

<210> 323
<211> 31
<212> DNA
<213> unknown
<308> L08096
<400> 323
CACATCCAGG TGACGYTGGC CATCTGCTCC T

```
<210> 324
<211> 31
<212> DNA
<213> unknown
<308> L08096
<400> 324
GACGCTGGCC ATCTGYTCCT CCACGACGGC C

<210> 325
<211> 31
<212> DNA
<213> unknown
<308> L08096
<400> 325
CCCCACCACC CTGGCYGTGG GAATCTGCTC T

<210> 326
<211> 31
<212> DNA
<213> unknown
<308> L08096
<400> 326
CCGTAGCATC AGCCTSCTGC GTCTCAGCTT C

<210> 327
<211> 31
<212> DNA
<213> unknown
<308> L08096
<400> 327
TAGCATCAGC CTGCTSCGTC TCAGCTTCCA C

<210> 328
<211> 31
<212> DNA
<213> unknown
<308> L11066
<400> 328
GAATGCCGGC CAAGCRACAG GCTGTCACCA A

<210> 329
<211> 31
<212> DNA
<213> unknown
<308> L11066
<400> 329
TCATTGGCCG GCGATRTGAT GATCCTGAAG T
```

```
<210> 330
<211> 31
<212> DNA
<213> unknown
<308> L11066
<400> 330
GCATTTGTGT TGATGRAGAT GAAAGAGACT G

<210> 331
<211> 31
<212> DNA
<213> unknown
<308> L11066
<400> 331
TCAATGACTC GCAGASACAG GCCACTAAAG A

<210> 332
<211> 31
<212> DNA
<213> unknown
<308> L11066
<400> 332
TATCCTGGAA ATTCARAAAG GAGTATTTGA G

<210> 333
<211> 31
<212> DNA
<213> unknown
<308> L11066
<400> 333
TGCTGAAAAG GCTAARTGTG AACTCTCCTC A

<210> 334
<211> 31
<212> DNA
<213> unknown
<308> L11066
<400> 334
TTGTCACTGA TCTAAYCAGA AGGACTATCG C

<210> 335
<211> 31
<212> DNA
<213> unknown
<308> L11066
<400> 335
TGTGTTGGCC GGCGAKGTCA CGGATGTGCT G
```

```
<210> 336
<211> 31
<212> DNA
<213> unknown
<308> L11066
<400> 336
ACTTATTAAT AGGAAYACCA CTATTCCAAC C

<210> 337
<211> 31
<212> DNA
<213> unknown
<308> L11066
<400> 337
GGTATTCTCT ACTGCYGCTG ATGGTCAAAC G

<210> 338
<211> 31
<212> DNA
<213> unknown
<308> L11066
<400> 338
CTTCAGCAGG CATCAYTGAA GCTGTTCGAA A

<210> 339
<211> 31
<212> DNA
<213> unknown
<308> L11066
<400> 339
TATAAAGATT TAACTRTTTT ATGCTGAAGT G

<210> 340
<211> 31
<212> DNA
<213> unknown
<308> L11066
<400> 340
GCTACCTTCT GCCCTSTGTC TGGCACCTAC A

<210> 341
<211> 31
<212> DNA
<213> unknown
<308> U05259
<400> 341
GAAACGATGG CAGAASGAGA AGCTCGGGTT G
```

<210> 342
<211> 31
<212> DNA
<213> unknown
<308> U05259
<400> 342
ACCTGGACGA CTGCTYCATG TATGAGGACA T


<210> 343
<211> 31
<212> DNA
<213> unknown
<308> U05259
<400> 343
CCTCAACATA GGAGAWGTCC AGCTGGAGAA G


<210> 344
<211> 31
<212> DNA
<213> unknown
<308> U44839
<400> 344
GGGCCCAGCT CTGGARTCAC GAACAGGTGC C


<210> 345
<211> 31
<212> DNA
<213> unknown
<308> U44839
<400> 345
GAAGCATGAC TGCGTYGGGT ACGTGATGAA G


<210> 346
<211> 31
<212> DNA
<213> unknown
<308> U44839
<400> 346
TGATGACAAC AGCGTMTCCC CTGTCAATGA G


<210> 347
<211> 31
<212> DNA
<213> unknown
<308> U44839
<400> 347
ACGCCAGGAC GTGGCRCGAC GCCTGCTGTC C

<210> 348
<211> 31
<212> DNA
<213> unknown
<308> U46025
<400> 348
AATAATGCAA AATACKGACC CTCACTCCCA A

<210> 349
<211> 31
<212> DNA
<213> unknown
<308> U46025
<400> 349
AGGTCTGCCG CATCTWCCTG CTGCGCATCC T

<210> 350
<211> 31
<212> DNA
<213> unknown
<308> U46025
<400> 350
TGAGGGCTCC TCAAASTCTG AGCAAGACCA G

<210> 351
<211> 31
<212> DNA
<213> unknown
<308> U46025
<400> 351
AGGGCGAGGA CTCGGYTGTG TTGATGGAGA G

<210> 352
<211> 31
<212> DNA
<213> unknown
<308> U46025
<400> 352
GAGGACTCGG CTGTGWTGAT GGAGAGACTG T

<210> 353
<211> 31
<212> DNA
<213> unknown
<308> U46025
<400> 353
CCTCTGCCAC ATCTAYCACC ATGCTCTGCA C

```
<210> 354
<211> 31
<212> DNA
<213> unknown
<308> U46025
<400> 354
TATCATCAAT GAGAARATGA ATGGGAAAGT G

<210> 355
<211> 31
<212> DNA
<213> unknown
<308> U46025
<400> 355
ACTGCCCAGC AGAACMTGGC TCTGCAGCTG G

<210> 356
<211> 31
<212> DNA
<213> unknown
<308> U70439
<400> 356
GCTAGCAAAC CCTTCYGACG GCCCTCGCTG C

<210> 357
<211> 31
<212> DNA
<213> unknown
<308> U70439
<400> 357
CTGGAGCTGA GGAACYGGAC CCCGGCAGCT G

<210> 358
<211> 31
<212> DNA
<213> unknown
<308> U70439
<400> 358
GAACTTAGAG TTCCTYAGTT TAATAAATGT A

<210> 359
<211> 31
<212> DNA
<213> unknown
<308> U70439
<400> 359
CTCAGTTTCA AATCTYCCCA AGCTGCCTAA A
```

```
<210> 360
<211> 31
<212> DNA
<213> unknown
<308> U70439
<400> 360
CATGTTAGCT GAAAARCTTC CAAATCTCAC A

<210> 361
<211> 31
<212> DNA
<213> unknown
<308> U70439
<400> 361
TTCCAAATCT CACACRTCTA AACTTAAGTG G

<210> 362
<211> 31
<212> DNA
<213> unknown
<308> U70439
<400> 362
GGAACCTTTG AAAAARTTAG AATGTCTGAA A

<210> 363
<211> 31
<212> DNA
<213> unknown
<308> U70439
<400> 363
CCCCAGCTTA CCTACWTGGA TGGCTATGAC C

<210> 364
<211> 31
<212> DNA
<213> unknown
<308> U70439
<400> 364
TGGATGGCTA TGACCRAGAG GACCAGGAAG C

<210> 365
<211> 31
<212> DNA
<213> unknown
<308> X07203
<400> 365
GGGTCTTCTG ATGATYCCAG CAGGGATCTA T
```

<210> 366
<211> 31
<212> DNA
<213> unknown
<308> X07203
<400> 366
TCATTTTCTC CATCARCAAC CAGGGAGACT G

<210> 367
<211> 31
<212> DNA
<213> unknown
<308> X15822
<400> 367
GCAGGAGTGA CTTCARTCAT CCCAGCAATC G

<210> 368
<211> 31
<212> DNA
<213> unknown
<308> X16663
<400> 368
GTACGGAGTT GAGAGRGACA GGGCAGACAA G

<210> 369
<211> 31
<212> DNA
<213> unknown
<308> X16663
<400> 369
AGAAGTGGAG AAGCAYACAT CTCAGAAAGA T

<210> 370
<211> 31
<212> DNA
<213> unknown
<308> X16663
<400> 370
GACGCCCATA GAAGCYGCTT CTAGTGGTGC C

<210> 371
<211> 31
<212> DNA
<213> unknown
<308> X16663
<400> 371
GCCGCTTCTA GTGGTRCCCG TGGGCTGAAG G

<210> 372
<211> 31
<212> DNA
<213> unknown
<308> X16663
<400> 372
GAAGAGCCAG TGTACRAAGC AGAGCCTGAG C

<210> 373
<211> 31
<212> DNA
<213> unknown
<308> X16663
<400> 373
GAGATGGACA GGCATRAGCA GGAGGATGAA C

<210> 374
<211> 31
<212> DNA
<213> unknown
<308> X16663
<400> 374
GATCATCAGG CTGCCYGGCT GGGGCTGGGG C

<210> 375
<211> 31
<212> DNA
<213> unknown
<308> X16663
<400> 375
ATCTCAGCTG TGGCTSTATA TGATTACCAA G

<210> 376
<211> 31
<212> DNA
<213> unknown
<308> X16663
<400> 376
CTTCCCTGCA AATTAYGTCA AGCTTCTGGA G

<210> 377
<211> 31
<212> DNA
<213> unknown
<308> X16663
<400> 377
ACTGTGATTT CCCATKTCCA AAGTGGCTCT G

```
<210> 378
<211> 31
<212> DNA
<213> unknown
<308> X16663
<400> 378
CCCTCCCTAT TCCTGMTGCA AATGTCTAAC C

<210> 379
<211> 31
<212> DNA
<213> unknown
<308> X16663
<400> 379
GTCTAACCAG ATGAGKTTCT GGACAGACTT C

<210> 380
<211> 31
<212> DNA
<213> unknown
<308> X16663
<400> 380
CTCCTTCCCA ATGAAYACCT CTCTGCCACC C

<210> 381
<211> 31
<212> DNA
<213> unknown
<308> X56468
<400> 381
GAAGACTGAG CTGATSCAGA AGGCCAAGCT G

<210> 382
<211> 31
<212> DNA
<213> unknown
<308> X56468
<400> 382
AGACTGAGCT GATCCWGAAG GCCAAGCTGG C

<210> 383
<211> 31
<212> DNA
<213> unknown
<308> X56468
<400> 383
GGCCGAGCGC TACGAYGACA TGGCCACCTG C
```

```
<210> 384
<211> 31
<212> DNA
<213> unknown
<308> X56468
<400> 384
GGTCATCTCT AGCATYGAGC AGAAGACCGA C


<210> 385
<211> 31
<212> DNA
<213> unknown
<308> X56468
<400> 385
CATCGAGCAG AAGACYGACA CCTCCGACAA G


<210> 386
<211> 31
<212> DNA
<213> unknown
<308> X56468
<400> 386
GGCTTTTGAT GAGGCSATTG CTGAACTTGA T


<210> 387
<211> 31
<212> DNA
<213> unknown
<308> X56468
<400> 387
CTGAATGAAG ACTCAKACAA AGACAGCACC C


<210> 388
<211> 31
<212> DNA
<213> unknown
<308> X56468
<400> 388
CACCCTCATC ATGCARTTGC TTAGAGACAA C


<210> 389
<211> 31
<212> DNA
<213> unknown
<308> X56468
<400> 389
GGCTGAAAAC TAAATMCATA CAGGGTGTCA T
```

<210> 390
<211> 31
<212> DNA
<213> unknown
<308> X56468
<400> 390
TCCATACAGG GTGTCMTCCT TCTTTCCTTC A

<210> 391
<211> 31
<212> DNA
<213> unknown
<308> X56468
<400> 391
TTTAAGTAAT CTGAAMCAGT TCTGCAAGTG A

<210> 392
<211> 31
<212> DNA
<213> unknown
<308> X56468
<400> 392
AAATTAAAGT ACACARGTCC AAGTCTAAAA C

<210> 393
<211> 31
<212> DNA
<213> unknown
<308> X57351
<400> 393
AGGACCTCCA CACCAYTAAC AAGATGAGCC T

<210> 394
<211> 31
<212> DNA
<213> unknown
<308> X57351
<400> 394
TGCATTTGAC AAATGSCAGG AAGAGGAAAC T

<210> 395
<211> 31
<212> DNA
<213> unknown
<308> X57351
<400> 395
TCTCCTGTCA ACAGCRGCCA GCCTCCCAAC T

```
<210> 396
<211> 31
<212> DNA
<213> unknown
<308> X57351
<400> 396
CTTCATAGCA TTCGCSTACT CCGTGAAGTC T

<210> 397
<211> 31
<212> DNA
<213> unknown
<308> X57351
<400> 397
CTTCATGACC ATTCTSCTCG TCATCATCCC A

<210> 398
<211> 31
<212> DNA
<213> unknown
<308> X57351
<400> 398
ATGACCATTC TGCTCRTCAT CATCCCAGTG T

<210> 399
<211> 31
<212> DNA
<213> unknown
<308> X59892
<400> 399
AGTGAGCCCG CATCTYTGCT GGAGCTGTTC A

<210> 400
<211> 31
<212> DNA
<213> unknown
<308> X59892
<400> 400
AGCTACAAAG CTGCCKCGGG GGAGGATTAC A

<210> 401
<211> 31
<212> DNA
<213> unknown
<308> X59892
<400> 401
CCTAAACCAG CCCTGYTGCA CTCCACCTTC T
```

```
<210> 402
<211> 31
<212> DNA
<213> unknown
<308> X59892
<400> 402
AATTGATAAG TAAAAKCTTC GTTATACATT T


<210> 403
<211> 31
<212> DNA
<213> unknown
<308> X59892
<400> 403
AGACTTGGGG GTGAARCTTT CCAGTTTACT G


<210> 404
<211> 31
<212> DNA
<213> unknown
<308> X59932
<400> 404
TCCGGTACAG AATGTWTTGC CAAGTACAAC T


<210> 405
<211> 31
<212> DNA
<213> unknown
<308> X59932
<400> 405
CTTCCACGGC ACTGCYGAGC AGGACCTGCC C


<210> 406
<211> 31
<212> DNA
<213> unknown
<308> X59932
<400> 406
GGTGCAGCTC CTGGGYGTGA TCGTGGAGGA G


<210> 407
<211> 31
<212> DNA
<213> unknown
<308> X59932
<400> 407
GACTACCTGC GGTCTMGGGG TCGGTCAGTG C
```

<210> 408
<211> 31
<212> DNA
<213> unknown
<308> X59932
<400> 408
TGCTGGCACC TGGACRCCGC CATGCGGCCC T

<210> 409
<211> 31
<212> DNA
<213> unknown
<308> X59932
<400> 409
TGCCCCTGCT CACTGRGCCC GAGCCTGAAC T

<210> 410
<211> 31
<212> DNA
<213> unknown
<308> X59932
<400> 410
GAGGAAGGAG GCCACRGAGC GGGAGGCAGC G

<210> 411
<211> 31
<212> DNA
<213> unknown
<308> X59932
<400> 411
ATTCCTTTCC TTTTTKGAGA TTTTTTTTCC G

<210> 412
<211> 31
<212> DNA
<213> unknown
<308> X59932
<400> 412
CTTGTGAGAT GGAATYGTAA TAAACCACGC C

<210> 413
<211> 31
<212> DNA
<213> unknown
<308> Z47055
<400> 413
TGGAGTACAA TGCCAYTGGA GGCAAGTATA A

<210> 414
<211> 31
<212> DNA
<213> unknown
<308> STSG-13126
<400> 414
TCCGGCCCTA TCGTCYTTCC TGTGGGCCAT T

<210> 415
<211> 31
<212> DNA
<213> unknown
<308> STSG-13353
<400> 415
GAAATAGGAC CAATCYGAGG CTTTCGTGAG A

<210> 416
<211> 31
<212> DNA
<213> unknown
<308> STSG-13369
<400> 416
GTGCTTTGGT ATATASAGGT AACAATGTAC T

<210> 417
<211> 31
<212> DNA
<213> unknown
<308> STSG-13448
<400> 417
ATGACAGATT CAAGAYGATG TCGCCTCCTG T

<210> 418
<211> 31
<212> DNA
<213> unknown
<308> D00761
<400> 418
GCCATGTATT CGGCTSCTGG CAGAGACTTG G

<210> 419
<211> 31
<212> DNA
<213> unknown
<308> D00761
<400> 419
TCAAGGCTGG AGGCTSAGCA AGTGCCATGC T

```
<210> 420
<211> 31
<212> DNA
<213> unknown
<308> D28137
<400> 420
GGCCCAGAAG GGCTTYCAGG ATGTGGAGGC C

<210> 421
<211> 31
<212> DNA
<213> unknown
<308> D43950
<400> 421
TTGCCAAGCT GATGGYGGAA CTGTCCAAGT C

<210> 422
<211> 31
<212> DNA
<213> unknown
<308> D43950
<400> 422
CTCGTGTTGC TATTGWACAC CTGGACAAGA T

<210> 423
<211> 31
<212> DNA
<213> unknown
<308> D43950
<400> 423
TCTTGTACAG GAGATSTCAT TTGGGACAAC T

<210> 424
<211> 31
<212> DNA
<213> unknown
<308> D43950
<400> 424
TAATCGTGGG GGTACYATCT CAACTGCTTT T

<210> 425
<211> 31
<212> DNA
<213> unknown
<308> HT3200
<400> 425
ACGCCATCCG CGAGGYCCTG GAGCTGGACT C
```

```
<210> 426
<211> 31
<212> DNA
<213> unknown
<308> J03077
<400> 426
TGCGGAGTCA GACGGYGCTA TGTACGCCCT C


<210> 427
<211> 31
<212> DNA
<213> unknown
<308> J03077
<400> 427
ATAAATATGG ATGGCRAGCT CCTAGGCCTC T


<210> 428
<211> 31
<212> DNA
<213> unknown
<308> L08666
<400> 428
AAAGTTACTG GGACCYTGGA GACCAAATAC A


<210> 429
<211> 31
<212> DNA
<213> unknown
<308> L08666
<400> 429
AGGCTTTTTT CCCCCMAGAA GATGATCAAA A


<210> 430
<211> 31
<212> DNA
<213> unknown
<308> L08666
<400> 430
TGCAGTCACC TATACRTTAT TTAAATGTAT T


<210> 431
<211> 31
<212> DNA
<213> unknown
<308> L22009
<400> 431
AACTCATTAT GATCCRCCAC GAAAGCTTAT G
```

```
<210> 432
<211> 31
<212> DNA
<213> unknown
<308> L22009
<400> 432
TGATTTATGC TTACTYTAAG TGGAAATCAG G


<210> 433
<211> 31
<212> DNA
<213> unknown
<308> L22009
<400> 433
CTCAAGTTAG TTATAKGATG TACTTAAGCA G



<210> 434
<211> 31
<212> DNA
<213> unknown
<308> L22009
<400> 434
TTAGTTATAG GATGTWCTTA AGCAGTAAGC G


<210> 435
<211> 31
<212> DNA
<213> unknown
<308> L22009
<400> 435
CTTTTTTTTT TTTTAMGTTA ATGGGAAAAA T


<210> 436
<211> 31
<212> DNA
<213> unknown
<308> L22009
<400> 436
TTGGTGTTTG TATGARATTC TGAGGCCTTG A


<210> 437
<211> 31
<212> DNA
<213> unknown
<308> L28010
<400> 437
TTCTCATGCA AAATTYTCTT CTAGCATGTG A
```

```
<210> 438
<211> 31
<212> DNA
<213> unknown
<308> L38951
<400> 438
TGCCAAGGAT TGTTAYCCTG CTGTCCAGAA A

<210> 439
<211> 31
<212> DNA
<213> unknown
<308> L38951
<400> 439
TTCTGTCTTT TGGCCRGTGC CGAGTGGAAT G

<210> 440
<211> 31
<212> DNA
<213> unknown
<308> L38951
<400> 440
GTAAAAGGCA GCCTGRCCTT TGCTACTGAG G


<210> 441
<211> 31
<212> DNA
<213> unknown
<308> L38951
<400> 441
TGTACCTTTG CCACAYGGTA CTGTATGCTT G

<210> 442
<211> 31
<212> DNA
<213> unknown
<308> L38951
<400> 442
CATGGTACTG TATGCYTGCC AGCTAGAAGG A

<210> 443
<211> 31
<212> DNA
<213> unknown
<308> L38951
<400> 443
TACTGTATGC TTGCCRGCTA GAAGGAGGGT C
```

```
<210> 444
<211> 31
<212> DNA
<213> unknown
<308> L38951
<400> 444
TATCCACATT CTCAASTTCA AGTCATTGCT G

<210> 445
<211> 31
<212> DNA
<213> unknown
<308> L38951
<400> 445
AAGTTCTGTC TACTGRTGCA GCACAAGAGA T

<210> 446
<211> 31
<212> DNA
<213> unknown
<308> L38951
<400> 446
CAATTGTTAC AAATARCGCA GACTTCAAAA A

<210> 447
<211> 31
<212> DNA
<213> unknown
<308> L38951
<400> 447
AAACAAACCA AAATTKAAAT GATCAGAATT G

<210> 448
<211> 31
<212> DNA
<213> unknown
<308> L38951
<400> 448
CCAGAAAATT GTGCCRAAGA GTTTAGAAAA A

<210> 449
<211> 31
<212> DNA
<213> unknown
<308> L38951
<400> 449
AAGAGTTTAG AAAAAYAAAT ATACAATAAA A
```

```
<210> 450
<211> 31
<212> DNA
<213> unknown
<308> M22490
<400> 450
CCAACACCGT GAGGASCTTC CACCACGAAG A

<210> 451
<211> 31
<212> DNA
<213> unknown
<308> M22632
<400> 451
GGGAGTTGGT GCCTAYCGGG ATGATAATGG A

<210> 452
<211> 31
<212> DNA
<213> unknown
<308> M22632
<400> 452
CTACCGGGAT GATAAYGGAA AGCCTTACGT T

<210> 453
<211> 31
<212> DNA
<213> unknown
<308> M22632
<400> 453
TGGAAAGCCT TACGTKCTGC CTAGCGTCCG C

<210> 454
<211> 31
<212> DNA
<213> unknown
<308> M22632
<400> 454
CGGGAGTGGA CCCGCRTCCG GAACAGTGGA A

<210> 455
<211> 31
<212> DNA
<213> unknown
<308> M22632
<400> 455
GGGCATTAAT GTTTGYCTCT GCCAATCATA T
```

<210> 456
<211> 31
<212> DNA
<213> unknown
<308> M22632
<400> 456
TGCAAGAAGT GAAAGKCATG GCTGACCGCA T

<210> 457
<211> 31
<212> DNA
<213> unknown
<308> M22632
<400> 457
TCAGCCACAG TGTGTRACAC TCAGCATTTG A

<210> 458
<211> 31
<212> DNA
<213> unknown
<308> M22632
<400> 458
CCTTATCAAC AGGCCRCACT GCAGAAATGA T

<210> 459
<211> 31
<212> DNA
<213> unknown
<308> M22632
<400> 459
TTTTTCTGCT GATGCYGTAC CCTCACCCTT T

<210> 460
<211> 31
<212> DNA
<213> unknown
<308> M22632
<400> 460
ATGTGGTGTG CAGCCWCTCA TCTCACACTG T

<210> 461
<211> 31
<212> DNA
<213> unknown
<308> M22632
<400> 461
CGGCGCAGGG TGGTASCAGG AAAGAGGATA T

EP 1 024 200 A2

```
<210> 462
<211> 31
<212> DNA
<213> unknown
<308> M57567
<400> 462
GCTGGGGCTA CAGCAYTTAC GCAGCCGCAC G

<210> 463
<211> 31
<212> DNA
<213> unknown
<308> M57567
<400> 463
TGATGCCCGG AAGCTSCTGC GTGCATCCCC G

<210> 464
<211> 31
<212> DNA
<213> unknown
<308> M63483
<400> 464
GAATCTGAAG ATGAGYTACT TGTAGATGAA G

<210> 465
<211> 31
<212> DNA
<213> unknown
<308> M63483
<400> 465
CCAACAAAGA TACAASTGAA AACGCAGATG G

<210> 466
<211> 31
<212> DNA
<213> unknown
<308> M63483
<400> 466
AGATACAAGT GAAAAYGCAG ATGGTCAAAG T

<210> 467
<211> 31
<212> DNA
<213> unknown
<308> M87503
<400> 467
TGATACAGCT AAGACYATGT TCCGGATTCC C
```

109

```
<210> 468
<211> 31
<212> DNA
<213> unknown
<308> M95627
<400> 468
AGCGAGGTCA CCTTTRCATT GCACTCAGCA T


<210> 469
<211> 31
<212> DNA
<213> unknown
<308> M95627
<400> 469
CTGCTTACTG ACTACYGGGG CCACACGGCT G


<210> 470
<211> 31
<212> DNA
<213> unknown
<308> M95627
<400> 470
GCCCTCCCAC CCTTGYCCAG ACCTGGTGGG C


<210> 471
<211> 31
<212> DNA
<213> unknown
<308> U12595
<400> 471
CCAGGCGAAA CCCAGYCTGG AGCTTGCAGG C


<210> 472
<211> 31
<212> DNA
<213> unknown
<308> U12595
<400> 472
CTGCCAGAAA TGGAGRTTCA CTTGCAGACC A


<210> 473
<211> 31
<212> DNA
<213> unknown
<308> U12595
<400> 473
AGAACCGGGA CAAAARTCAT CATCCACCTG A
```

```
<210> 474
<211> 31
<212> DNA
<213> unknown
<308> U12595
<400> 474
GACCCCAAGG ATGTCSGTGA GTGGCAACAT G


<210> 475
<211> 31
<212> DNA
<213> unknown
<308> U12595
<400> 475
CCGCAGCATC TTCTAYGTGC CCGACATGAA A



<210> 476
<211> 31
<212> DNA
<213> unknown
<308> U12595
<400> 476
GGTGGACAGT GAGGASATTC CCCTGAACCT C


<210> 477
<211> 31
<212> DNA
<213> unknown
<308> U12595
<400> 477
TGCTGGACTT GTTGAYGACC CTAGGGCCAT G


<210> 478
<211> 31
<212> DNA
<213> unknown
<308> U12595
<400> 478
GGGCCATGGT GGGCCRCTTG AATGAGCTGC T


<210> 479
<211> 31
<212> DNA
<213> unknown
<308> U15085
<400> 479
CTAGGGGCTG GGTATYCCCA CATCACTCAT T
```

<210> 480
<211> 31
<212> DNA
<213> unknown
<308> U15085
<400> 480
GCCCATGTGG AAAGCRCCTG TCTGTTGGAT G

<210> 481
<211> 31
<212> DNA
<213> unknown
<308> U15085
<400> 481
TGGAAAGCAC CTGTCKGTTG GATGATGCTG G

<210> 482
<211> 31
<212> DNA
<213> unknown
<308> U15085
<400> 482
TCTCCTTCAA CAAGGWTCTG CTGACCTGCT G

<210> 483
<211> 31
<212> DNA
<213> unknown
<308> U15085
<400> 483
TTGGGGTGCT GAATARCTTG GCGAATGTCC T

<210> 484
<211> 31
<212> DNA
<213> unknown
<308> U15085
<400> 484
TGCCTCACAG CAGTGMGCAC AAGACTGCCC A

<210> 485
<211> 31
<212> DNA
<213> unknown
<308> U15085
<400> 485
GTGTGGTAGA GCACAYTGGG GCTCCTGAGC C

```
<210> 486
<211> 31
<212> DNA
<213> unknown
<308> U15085
<400> 486
TCTTCTCTCT TGGTGYGATC AGCTGGCGGA G

<210> 487
<211> 31
<212> DNA
<213> unknown
<308> U15085
<400> 487
ATTCAAACTC AATGAYGCTA CCATGCCTCT C

<210> 488
<211> 31
<212> DNA
<213> unknown
<308> U15085
<400> 488
TGTAAAACTT AGCAAYTTGG GGGACCTCAT T

<210> 489
<211> 31
<212> DNA
<213> unknown
<308> U15085
<400> 489
TCAGTGTGCC ATAGARGACA GCAACTGGTG A

<210> 490
<211> 31
<212> DNA
<213> unknown
<308> U28386
<400> 490
ACAGAAATGA GGCGTYGCAG AATAGAGGTC A

<210> 491
<211> 31
<212> DNA
<213> unknown
<308> U28386
<400> 491
ATGGAGGTGC CATCCSAGCA TTCATTTCTC T
```

```
<210> 492
<211> 31
<212> DNA
<213> unknown
<308> U28386
<400> 492
CAGTTCCTGA TATGTMATCT TTAGCATGTG G

<210> 493
<211> 31
<212> DNA
<213> unknown
<308> U28386
<400> 493
TGATGATCCA GAAGTRTTAG CAGATACCTG C

<210> 494
<211> 31
<212> DNA
<213> unknown
<308> U28386
<400> 494
CCCATTCCTT GTCAGYGTTC TCTCTAAGGC A

<210> 495
<211> 31
<212> DNA
<213> unknown
<308> U28386
<400> 495
GGCTGCTGAG AAACTRGGTG AAACTGAGAA A

<210> 496
<211> 31
<212> DNA
<213> unknown
<308> U28386
<400> 496
GAGGCTTAGA CAAAAWTGAA GCTCTACAAA A

<210> 497
<211> 31
<212> DNA
<213> unknown
<308> U30255
<400> 497
AACATGAATG ACCACSGCTT TGTGGTCTGT G
```

```
<210> 498
<211> 31
<212> DNA
<213> unknown
<308> U30255
<400> 498
TGTCTCCAAA GTTGAYGATT TCTTGGCCAA T

<210> 499
<211> 31
<212> DNA
<213> unknown
<308> U30255
<400> 499
TCTCAAGTTC CAAGAYACCG ATGGCAAACA C

<210> 500
<211> 31
<212> DNA
<213> unknown
<308> U30255
<400> 500
AAGTTCCAAG ACACCRATGG CAAACACCTG C

<210> 501
<211> 31
<212> DNA
<213> unknown
<308> U30255
<400> 501
CACCGTGTCA TCCTCRTCAT ACAATGCCTG A

<210> 502
<211> 31
<212> DNA
<213> unknown
<308> U45328
<400> 502
GGAGTGCGCC ATTCCRGGAA AGAAAGGGAC T

<210> 503
<211> 31
<212> DNA
<213> unknown
<308> U45328
<400> 503
CTTGTGGCAT CGTCARAAGG AAGGGATTGG T
```

<210> 504
<211> 31
<212> DNA
<213> unknown
<308> U45328
<400> 504
GCGGGTGTGC GGTCTSGATT CGCTGAATTG C

<210> 505
<211> 31
<212> DNA
<213> unknown
<308> U45328
<400> 505
TGTGCGGTCT CGATTSGCTG AATTGCCCGT T

<210> 506
<211> 31
<212> DNA
<213> unknown
<308> U51127
<400> 506
GGATGAAGCC GATCCRGCCA AGTGGAAGGC C

<210> 507
<211> 31
<212> DNA
<213> unknown
<308> U51127
<400> 507
ACAGCCCCGA TGAGCWCCTG GCTGGCTGCA G

<210> 508
<211> 31
<212> DNA
<213> unknown
<308> U51127
<400> 508
TTCCTGGAAG TGGATYTGGG CCAAGAAGGA G

<210> 509
<211> 31
<212> DNA
<213> unknown
<308> U88964
<400> 509
GAGGGTCCCC AAGGARCATG GCTGGGAGCC G

```
<210> 510
<211> 31
<212> DNA
<213> unknown
<308> U88964
<400> 510
GAGTGAGCGC CTCCTRCACA AGAGCATCCA G


<210> 511
<211> 31
<212> DNA
<213> unknown
<308> X15729
<400> 511
TTTCCCTGCA AATGTSATGG ATGTTATTGC A

<210> 512
<211> 31
<212> DNA
<213> unknown
<308> X15729
<400> 512
GACAGAATTT CACTGMACCC ACTGCTATTC A

<210> 513
<211> 31
<212> DNA
<213> unknown
<308> X15729
<400> 513
CTCAGGGATG GCCAGKTGCT CTAAGTGGAT T

<210> 514
<211> 31
<212> DNA
<213> unknown
<308> X15729
<400> 514
ATTTGCTTCC TGCCAYTGTC CACATCAATC A

<210> 515
<211> 31
<212> DNA
<213> unknown
<308> X15729
<400> 515
ATTGTGGATG TGTGTSATGA CGTAGAAAAG G
```

```
<210> 516
<211> 31
<212> DNA
<213> unknown
<308> X15729
<400> 516
GTCGAAGACA GAGGTKCAGG TCGTTCCAGG G

<210> 517
<211> 31
<212> DNA
<213> unknown
<308> X56253
<400> 517
GCAGTGAGAG AATCCWGGCA GACAGAAGAA A

<210> 518
<211> 31
<212> DNA
<213> unknown
<308> X75861
<400> 518
TGCTTTCATG GGCACRGCAA TGATCTTTAC C

<210> 519
<211> 31
<212> DNA
<213> unknown
<308> X75861
<400> 519
CCTTGTTGAT ACTCARCTCA TTATTGAAAA G

<210> 520
<211> 31
<212> DNA
<213> unknown
<308> X75861
<400> 520
CTCCCTTTTT TGTCARCCCC ATCTGTAGCC T

<210> 521
<211> 31
<212> DNA
<213> unknown
<308> X75861
<400> 521
CCCTTCCTTC CTCATYGTTG TTTGGTATGC G
```

```
<210> 522
<211> 31
<212> DNA
<213> unknown
<308> X75861
<400> 522
CCATTGGAAA GAAGTKCAGT GGTCCCATTG T

<210> 523
<211> 31
<212> DNA
<213> unknown
<308> D13900
<400> 523
GAGAGCCATG GCCGCYCTGC GTGTCCTGCT G

<210> 524
<211> 31
<212> DNA
<213> unknown
<308> D13900
<400> 524
TCCTGCTGTC CTGCGYCCGC GGCCCGCTGA G

<210> 525
<211> 31
<212> DNA
<213> unknown
<308> D13900
<400> 525
ACCAGGCCCT GAAGAYCTTC GAGGAGGACC C

<210> 526
<211> 31
<212> DNA
<213> unknown
<308> D13900
<400> 526
CTTCGAGGAG GACCCRGCCG TTGGGGGCAT T

<210> 527
<211> 31
<212> DNA
<213> unknown
<308> D13900
<400> 527
GGAGATGGTC CTCACYGGTG ACGCGATCTC A
```

```
<210> 528
<211> 31
<212> DNA
<213> unknown
<308> D13900
<400> 528
CCGTGGTGTG CAGTTYCTCT CCAATTGCTG C


<210> 529
<211> 31
<212> DNA
<213> unknown
<308> D13900
<400> 529
GGAAAGCGGG GCCTGYGGGT CCTTGTGTCC C


<210> 530
<211> 31
<212> DNA
<213> unknown
<308> D14812
<400> 530
TGTTTCAGTT TGTTTYCCGT TCTGTTTTAA A


<210> 531
<211> 31
<212> DNA
<213> unknown
<308> D14812
<400> 531
GTTCCTATCC CTTTGMCACT ACACAATTTT C


<210> 532
<211> 31
<212> DNA
<213> unknown
<308> D14812
<400> 532
AGGCATTCTA TCTTTSCTCA AATTGTTGAA G


<210> 533
<211> 31
<212> DNA
<213> unknown
<308> D28473
<400> 533
GCTGGCCCGC GGGGCWGTCC AGGCACGCGA G
```

<210> 534
<211> 31
<212> DNA
<213> unknown
<308> D28473
<400> 534
AAGATTTGGA TGGGAKTGCC ATGGCTTACC T

<210> 535
<211> 31
<212> DNA
<213> unknown
<308> D28473
<400> 535
GGACGATTAC TCATTKTAAT GGAAGCCAGA T

<210> 536
<211> 31
<212> DNA
<213> unknown
<308> D28473
<400> 536
AGAACTGTCA GGAGCRAAGA TCTCAGATCT C

<210> 537
<211> 31
<212> DNA
<213> unknown
<308> D28473
<400> 537
TCTACAATGA GAACAYGGTT AGAGAAAGCC C

<210> 538
<211> 31
<212> DNA
<213> unknown
<308> D28473
<400> 538
AGATAAAAAC AAGTAYGGCA TTCGGCTAAG G

<210> 539
<211> 31
<212> DNA
<213> unknown
<308> D28473
<400> 539
AGATGAAGGA ATGGCKCGGG AAGTCATCAA T

```
<210> 540
<211> 31
<212> DNA
<213> unknown
<308> D28473
<400> 540
AAGCAAAGTC TGAAGKAACA TATCTGAATA G

<210> 541
<211> 31
<212> DNA
<213> unknown
<308> D28473
<400> 541
TTGAAACCAT ATCCARTTTC TCCATCGGAT A

<210> 542
<211> 31
<212> DNA
<213> unknown
<308> D28473
<400> 542
CAGCTCCTGA ATGCARAGCC ACAAGAGTGT T

<210> 543
<211> 31
<212> DNA
<213> unknown
<308> D28473
<400> 543
GGTGTGTGTA AAATAYGCTG CTTGGATTGA A

<210> 544
<211> 31
<212> DNA
<213> unknown
<308> D28473
<400> 544
GTCAAGTAAC TTGAGWCCTC ACAGTAATCT T

<210> 545
<211> 31
<212> DNA
<213> unknown
<308> D32050
<400> 545
TTCAGAAGGG CACAGRTGCC CGACCATACA C
```

```
<210> 546
<211> 31
<212> DNA
<213> unknown
<308> D32050
<400> 546
GGTGCTGGCT GACCAYGCTC GGACCATCAC T

<210> 547
<211> 31
<212> DNA
<213> unknown
<308> D32050
<400> 547
TCCCGGAGAC ACTGCYTGGC TCCTCTATGA C

<210> 548
<211> 31
<212> DNA
<213> unknown
<308> D32050
<400> 548
CCAAACAAGG ATGTGSAGAG GGAGATCGCT G

<210> 549
<211> 31
<212> DNA
<213> unknown
<308> D32050
<400> 549
GTGCCTGTGT CAAGTYCCCC AGAATGCAGC C

<210> 550
<211> 31
<212> DNA
<213> unknown
<308> D32050
<400> 550
TCGCATCTAT AGATAMCGGC TCTCCAGACC T

<210> 551
<211> 31
<212> DNA
<213> unknown
<308> D78151
<400> 551
GGGAGGCCGG GACAARGCGC CGGTGCAGCC C
```

<210> 552
<211> 31
<212> DNA
<213> unknown
<308> D78151
<400> 552
CCACAATGCA GAGCAKGAGG CTTGCGACCT G


<210> 553
<211> 31
<212> DNA
<213> unknown
<308> D78151
<400> 553
TGAAGATGTC GAGGAKTATG AGGACCTGAC A


<210> 554
<211> 31
<212> DNA
<213> unknown
<308> D78151
<400> 554
CCTCCTCTTT TGTGARTGGC TTTGTGAATG C


<210> 555
<211> 31
<212> DNA
<213> unknown
<308> D78151
<400> 555
TCCGGAATGA GTGTGWCCCT GCTCTGGCAC T


<210> 556
<211> 31
<212> DNA
<213> unknown
<308> D78151
<400> 556
TGCTCTCAGA CTATGWTCTC CACAACAGCA A


<210> 557
<211> 31
<212> DNA
<213> unknown
<308> D78151
<400> 557
CAGTTTTGCC AACACRCTGG TGGATGTGTG T

```
<210> 558
<211> 31
<212> DNA
<213> unknown
<308> D78151
<400> 558
AGATATGGGG AGCCTRCACT CCGGAGGGCT G

<210> 559
<211> 31
<212> DNA
<213> unknown
<308> D78151
<400> 559
CGTCTGGCTG CAATGYTGCG CCAGTTAGCT C

<210> 560
<211> 31
<212> DNA
<213> unknown
<308> D78151
<400> 560
ATACAACCCC AGTGTYGTTG GCCCACGGGG A

<210> 561
<211> 31
<212> DNA
<213> unknown
<308> D78151
<400> 561
AGGGGCTCTG AACTGYAGCT GATGTTATCA G

<210> 562
<211> 31
<212> DNA
<213> unknown
<308> D78151
<400> 562
GATGTTATCA GCAGGMCATG CATCCTGCTG C

<210> 563
<211> 31
<212> DNA
<213> unknown
<308> J04611
<400> 563
TGGTTGATGC CTCCARGGCT ATGTTTGAAT C
```

```
<210> 564
<211> 31
<212> DNA
<213> unknown
<308> J04611
<400> 564
GTGATCTCTG TGGGCRTTTA TAATCTGGTC C

<210> 565
<211> 31
<212> DNA
<213> unknown
<308> J04611
<400> 565
CCTAGCGATA CCAAGMGGTC TCAGATCTAT G

<210> 566
<211> 31
<212> DNA
<213> unknown
<308> J04611
<400> 566
TACTGCTGAA GAAACRCCAT TACCTGAGGC C

<210> 567
<211> 31
<212> DNA
<213> unknown
<308> J04611
<400> 567
AAAAGACTGG GCTCCYTGGT GGATGAGTTT A

<210> 568
<211> 31
<212> DNA
<213> unknown
<308> J04611
<400> 568
TTTAAGGAGC TTGTTYACCC ACCAGATTAC A

<210> 569
<211> 31
<212> DNA
<213> unknown
<308> J04611
<400> 569
CGGGCTGAAG AGTGGKCTGA AGAAGCAGGA G
```

```
<210> 570
<211> 31
<212> DNA
<213> unknown
<308> L15189
<400> 570
GAGGCTCATG GGAAAYTGTA TTCTCCGAGT C

<210> 571
<211> 31
<212> DNA
<213> unknown
<308> L15189
<400> 571
TTGGAGCATT TGTGTYGATG AAGATGAAAG A

<210> 572
<211> 31
<212> DNA
<213> unknown
<308> L15189
<400> 572
AGGGGTTGAT TTGACYAAAG ACAACATGGC A

<210> 573
<211> 31
<212> DNA
<213> unknown
<308> L15189
<400> 573
TGCTGAAAAG GCTAARTGTG AACTCTCCTC A

<210> 574
<211> 31
<212> DNA
<213> unknown
<308> L15189
<400> 574
CCAGTCTTCT GGTGGRTTAA GCAAAGATGA T

<210> 575
<211> 31
<212> DNA
<213> unknown
<308> L15189
<400> 575
GAAGAGATTT CCAAARTGAG GGAGCTCCTG G
```

<210> 576
<211> 31
<212> DNA
<213> unknown
<308> L15189
<400> 576
CTTCAGCAGG CATCAYTGAA GCTGTTCGAA A

<210> 577
<211> 31
<212> DNA
<213> unknown
<308> M29877
<400> 577
GGATGAGGTC GCGGCSGGCG GGTCCCGCGC T

<210> 578
<211> 31
<212> DNA
<213> unknown
<308> M29877
<400> 578
CGACTTCGGA CCGCARTTCA CTGCGCGCTT C

<210> 579
<211> 31
<212> DNA
<213> unknown
<308> M29877
<400> 579
CAAGTATGTA GTTTTSACGA CAAAGCATCA C

<210> 580
<211> 31
<212> DNA
<213> unknown
<308> M29877
<400> 580
GCTTCACAAA CTGGCYGAGT CCTGTGTCTT G

<210> 581
<211> 31
<212> DNA
<213> unknown
<308> M29877
<400> 581
ATAAATTCAA GCCACRGAGC TTGCCAGATC A

```
<210> 582
<211> 31
<212> DNA
<213> unknown
<308> M97935
<400> 582
AAGCTTCTTG GTCCTMACGC CAGCCCCGAT G

<210> 583
<211> 31
<212> DNA
<213> unknown
<308> M97935
<400> 583
AGTCTATTAG CCACARAATT GGGAAAGGAG T

<210> 584
<211> 31
<212> DNA
<213> unknown
<308> M97935
<400> 584
TGTATTGCAT TAAATRTAAT ATCGACACAG T

<210> 585
<211> 31
<212> DNA
<213> unknown
<308> U09587
<400> 585
CCAGCTTGAT AACTAYGGAC AGCAAGAACT T

<210> 586
<211> 31
<212> DNA
<213> unknown
<308> U09587
<400> 586
AGAAATTGAG CACTTYGTAG ATCCCAGTGA G

<210> 587
<211> 31
<212> DNA
<213> unknown
<308> U09587
<400> 587
GAAATGGAGA TGCTGSTGAA TGAGAAAGGG G
```

```
<210> 588
<211> 31
<212> DNA
<213> unknown
<308> U09587
<400> 588
CTGCTGAATG AGAAARGGGA ATTCACAATT G

<210> 589
<211> 31
<212> DNA
<213> unknown
<308> U09587
<400> 589
TTTCCCTGCT GTAGTYGCTC CATTCAAATG T

<210> 590
<211> 31
<212> DNA
<213> unknown
<308> U09587
<400> 590
CATTCAAATG TTCCGYCCTC CCACTGAGCC A

<210> 591
<211> 31
<212> DNA
<213> unknown
<308> U09587
<400> 591
CAATGGCAAC ATCACRTGGG CTGATGTGGA G

<210> 592
<211> 31
<212> DNA
<213> unknown
<308> U41371
<400> 592
GAGCTTCAGG CCAAGKTGGC AGAGATCGGA G

<210> 593
<211> 31
<212> DNA
<213> unknown
<308> U41371
<400> 593
AGGATCTTTG AGGCTKTTAA GCTCACTGAT G
```

```
<210> 594
<211> 31
<212> DNA
<213> unknown
<308> U54778
<400> 594
TGAGACTGAG CTAAARTTAA TCTGTTGTGA C


<210> 595
<211> 31
<212> DNA
<213> unknown
<308> U54778
<400> 595
TTTTTCAGCA TTACTRTGTC GTCTCCGTGC C


<210> 596
<211> 31
<212> DNA
<213> unknown
<308> U54778
<400> 596
AAGCCATTAG ACTTAYAGGT GATGCAAGCA T


<210> 597
<211> 31
<212> DNA
<213> unknown
<308> U54778
<400> 597
ACACTATAGA GGCATRAGTG GTATCAGTTT T


<210> 598
<211> 31
<212> DNA
<213> unknown
<308> U54778
<400> 598
TAATTGGGTG TTAGCYTGAG ATGGTTAAAG G


<210> 599
<211> 31
<212> DNA
<213> unknown
<308> U54778
<400> 599
ACAGATACAG AATCTKTATT GTTCTTACTG A
```

```
<210> 600
<211> 31
<212> DNA
<213> unknown
<308> U57650
<400> 600
GAGCCTCTCC GAGACRTTGT TCCAGCGACT G

<210> 601
<211> 31
<212> DNA
<213> unknown
<308> U57650
<400> 601
GACAAGCCTG TTGTCRTCCA TTGAAGACAA G


<210> 602
<211> 31
<212> DNA
<213> unknown
<308> U57650
<400> 602
GAAAATCCTG CAGCTSATTA AGTCACAGAA A

<210> 603
<211> 31
<212> DNA
<213> unknown
<308> U57650
<400> 603
TCCCAGGACA CAGGARTCAA GGCCCAGTGA C

<210> 604
<211> 31
<212> DNA
<213> unknown
<308> U57650
<400> 604
AACACCGAGC TCCCGYATCA CGGCAAGCAC C

<210> 605
<211> 31
<212> DNA
<213> unknown
<308> U57650
<400> 605
CCATGCAGTG AAGCCYTCAG TGAGCTGCCA C
```

```
<210> 606
<211> 31
<212> DNA
<213> unknown
<308> U57650
<400> 606
TCTGGGTCCC CAGCTYGCTC TTGGTACTTG G

<210> 607
<211> 31
<212> DNA
<213> unknown
<308> U57650
<400> 607
GGGACCCCAG TGCCTYGTTG AGGGCGCCAT T

<210> 608
<211> 31
<212> DNA
<213> unknown
<308> U57650
<400> 608
GATCGAACAC TCATGRTGTG CCAAGTGCTG T

<210> 609
<211> 31
<212> DNA
<213> unknown
<308> U57650
<400> 609
GCTTTAGCTA AAGTCYCGCG GGTTCCGGCA T

<210> 610
<211> 31
<212> DNA
<213> unknown
<308> U80040
<400> 610
GAACCGGCCG CTGACMCTCT CGGAGAAGAT T

<210> 611
<211> 31
<212> DNA
<213> unknown
<308> U80040
<400> 611
GGGATCCCCT GGGAGYTGAA GTGCCCCAAG G
```

```
<210> 612
<211> 31
<212> DNA
<213> unknown
<308> U80040
<400> 612
AATCTGCAAC ATGGGYGCAG AAATTGGGGC C

<210> 613
<211> 31
<212> DNA
<213> unknown
<308> U80040
<400> 613
CCAACAACCT GCTCAYTGGT GCCATCAACA T

<210> 614
<211> 31
<212> DNA
<213> unknown
<308> U80040
<400> 614
GATTCTAGAG AACCTKTTGT TCTTGCAAGG A

<210> 615
<211> 31
<212> DNA
<213> unknown
<308> U83843
<400> 615
ATTCTGAAAC TTCTTSATGT TGTCCATCCT G

<210> 616
<211> 31
<212> DNA
<213> unknown
<308> U83843
<400> 616
TGAGGATTAT CAGGCRATTG TTGATGCTGA G

<210> 617
<211> 31
<212> DNA
<213> unknown
<308> U83843
<400> 617
GGCAATTGTT GATGCYGAGT GGAACATTCT C
```

```
<210> 618
<211> 31
<212> DNA
<213> unknown
<308> U83843
<400> 618
GGATCTGAAG AGGACRATGA TGGCCTGTGG A

<210> 619
<211> 31
<212> DNA
<213> unknown
<308> U83843
<400> 619
GGAGACAGAG CGGTCYCTGC ATGATGCCAT C

<210> 620
<211> 31
<212> DNA
<213> unknown
<308> U91932
<400> 620
AGTGCCCACC CGGTCSGCCC GGCACAGCCA T

<210> 621
<211> 31
<212> DNA
<213> unknown
<308> X54942
<400> 621
TGAGAAATGT ACAAAYCTTT CATCCATACC T

<210> 622
<211> 31
<212> DNA
<213> unknown
<308> X54942
<400> 622
AGAAATGTAC AAATCYTTCA TCCATACCTG T

<210> 623
<211> 31
<212> DNA
<213> unknown
<308> X59417
<400> 623
TTTAAAGTAG TGCTTSTACC AACATGTCCC G
```

```
<210> 624
<211> 31
<212> DNA
<213> unknown
<308> X59417
<400> 624
AAGGCCCTCA GGTATRTAAG TGTGATCCTG C

<210> 625
<211> 31
<212> DNA
<213> unknown
<308> X59417
<400> 625
TGATTGGACA TTTGARCAGA CAGTGGAAAC T

<210> 626
<211> 31
<212> DNA
<213> unknown
<308> X59417
<400> 626
AGTTGAAAAT CCTAARTTCA GGATTCTTAC A

<210> 627
<211> 31
<212> DNA
<213> unknown
<308> X64364
<400> 627
AGCCTCCGGG GCTGCYGGCA CAGTCTTCAC T

<210> 628
<211> 31
<212> DNA
<213> unknown
<308> X64364
<400> 628
GTTCAAGGTG GACTCSGACG ACCAGTGGGG A

<210> 629
<211> 31
<212> DNA
<213> unknown
<308> X64364
<400> 629
TCCCAGAGTG AAGGCYGTGA AGTCGTCAGA A
```

```
<210> 630
<211> 31
<212> DNA
<213> unknown
<308> X64364
<400> 630
GACGCTCCCT GCTCCRCGTC TGCGCCGCCG C

<210> 631
<211> 31
<212> DNA
<213> unknown
<308> X64364
<400> 631
ACCGAGGGCG ACCCCRTCAC AGCCTCAAGT C

<210> 632
<211> 31
<212> DNA
<213> unknown
<308> X64364
<400> 632
GGACGGCCGG CTCTCYATAG CACCAGGGCT C
```

**Description**

CROSS-REFERENCES TO RELATED APPLICATIONS

The present application claims priority from USSN 09/238,402 filed January 27, 1999, the disclosure of which is incorporated by reference.

BACKGROUND OF THE INVENTION

The genomes of all organisms undergo spontaneous mutation in the course of their continuing evolution generating variant forms of progenitor sequences (See, e.g., Gusella, *Ann. Rev. Biochem.* 55, 831-854 (1986)). A variant form may be neutral or confer an evolutionary advantage or disadvantage relative to a progenitor sequence. In some instances, a variant form confers a lethal disadvantage. In other instances, a variant form confers an evolutionary advantage to the species and is eventually incorporated into the deoxyribonucleic acid (DNA) of many or most members of the species and effectively becomes the progenitor sequence. In many instances, both the progenitor and the variant form(s) survive and co-exist in a species population. The coexistence of multiple forms of a sequence gives rise to polymorphisms.

Several different types of polymorphism have been reported. A restriction fragment length polymorphism (RFLP) is a variation in a DNA sequence that alters the length of a restriction fragment (see, e.g., Botstein et al., *Am. J. Hum. Genet.* 32, 314-331 (1980). The RFLP may create or delete a restriction site, thus changing the length of the restriction fragment. RFLPs have been widely used in human and animal genetic analyses (See, e.g., WO 90/13668; WO 90/11369; Donis-Keller, *Cell* 51, 319-337 (1987); and, Lander et al., *Genetics* 121, 85-99 (1989)). Where a heritable trait can be linked to a particular RFLP, the presence of such RFLP in a human can be used to predict the likelihood that the individual will also exhibit the trait.

Other polymorphisms take the form of short tandem repeats (STRs), for example, tandem di-, tri- and tetra-nucleotide repeated motifs. These tandem repeats are also referred to as variable number tandem repeat (VNTR) polymorphisms. VNTRs have been used in identity and paternity analyses as well as in a large number of genetic mapping studies. (See, e.g., US 5,075,217; Armour et al., *FEBS Lett.* 307, 113-115 (1992); Horn et al., W0 91/14003; Jeffreys, EP 370,719).

Other polymorphisms take the form of single nucleotide variations between individuals of the same species. Such polymorphisms are far more frequent than RFLPs, STRs and VNTRs. Some single nucleotide polymorphisms (SNPs) occur in protein-coding sequences, in which case, one of the polymorphic forms may give rise to the expression of a defective, or other variant, protein and, potentially, a genetic disease. Examples of genes, in which polymorphisms within coding sequences give rise to genetic disease include â - globin (sickle cell anemia) and CFTR (cystic fibrosis). SNPs also occur in noncoding regions and these SNPs may result in defective protein expression (e.g., as a result of defective splicing). Further, some SNPs have no phenotypic effects.

SNPs can be used in the same manner as RFLPs and VNTRs but offer several advantages. SNPs occur with greater frequency and are spaced more uniformly throughout the genome than other polymorphisms. The greater frequency and uniformity of SNPs mean that there is a greater probability that such a SNP will be found in close proximity to a genetic locus of interest than would be the case for other polymorphisms. Also, the different forms of characterized SNPs are often easier to distinguish than other polymorphisms (e.g., by use of assays employing allele-specific hybridization probes or primers).

Despite the increased amount of nucleotide sequence data being generated in recent years, only a minute proportion of the total repository of polymorphisms in humans and other organisms has been identified. The paucity of polymorphisms hitherto identified is due to, in substantial part, the large amount of work required for their detection by conventional methods. A conventional approach to identifying polymorphisms, for example, sequences the same stretch of oligonucleotides in a population of individuals by dideoxy sequencing. In this type of approach, the amount of work increases in proportion to both the length of the sequence and the number of individuals in a population and, as those skilled in the art will understand, becomes impractical for large stretches of DNA or large numbers of subjects.

All documents, i.e., publications and patent applications, cited in this disclosure, including the foregoing, are incorporated by reference herein in their entireties for all purposes to the same extent as if each of the individual documents were specifically and individually indicated to be so incorporated by reference herein in its entirety.

SUMMARY OF THE INVENTION

This invention provides nucleic acid segments of between 10 and 100 contiguous bases from a fragment shown in TABLE 1, column 1 including a polymorphic site or an immediately adjacent base. Complements of these segments are also provided in this invention. The segments can be DNA or ribonucleic acid (RNA), and can be double- or single-stranded. Some segments are 10-20 or 10-50 bases length. Preferred segments include a diallelic polymorphic site. The base occupying the polymorphic site in the segments can be the reference (TABLE 1, column 3) or an alternative base (TABLE 1, column 5).

The invention further provides allele-specific oligonucleotides that hybridizes to a segment of a fragment shown in TABLE 1, column 8 or its complement. These oligonucleotides can be probes or primers. Also provided are isolated nucleic acids comprising a sequence shown in TABLE 1, column 8, or the complement thereto, in which the polymorphic site within the sequence is occupied by a base other than the reference base shown in TABLE 1, column 3.

The invention further provides methods of analyzing a nucleic acid from a subject, which may be, but is not limited to a human being. The novel methods determine which base is present at any one of the polymorphic sites shown in TABLE 1. Optionally, a set of bases occupying a set of the polymorphic sites shown in TABLE 1 is determined. These types of analyses can be performed on a plurality of subjects who are tested for the presence of a disease phenotype. The presence or absence of disease phenotype can then be correlated with a base or set of bases present at the polymorphic sites in the subjects tested.

DEFINITIONS

An oligonucleotide, as the case may be, can be DNA or RNA, and single-or double-stranded. Oligonucleotides can be composed of naturally occurring or synthetic bases, but in either case are generally prepared by synthetic means. Preferred oligonucleotides of the invention include segments of DNA, or their complements including any one of the polymorphic sites shown in TABLE 1. The segments are usually between 5 and 100 bases in length, and often between 5-10, 5-20, 10-20, 10-50, 20-50 or 20-100 bases in length. The polymorphic site can occur within any position of the segment. The segments can be from any of the allelic forms of DNA shown in TABLE 1.

Hybridization probes are oligonucleotides capable of binding in a base-specific manner to a complementary strand of nucleic acid. Such probes include peptide nucleic acids. (See, e.g., Nielsen et al., *Science* 254, 1497-1500 (1991)).

A primer is a single-stranded oligonucleotide capable of acting as a point of initiation for template-directed DNA synthesis under suitable conditions e.g., buffer and temperature, in the presence of four different nucleoside triphosphates and an agent for polymerization, such as, for example, DNA or RNA polymerase or reverse transcriptase. The length of the primer, in any given case, depends on, for example, the intended use of the primer, and generally ranges from 15 to 30 nucleotides. Short primer molecules generally require cooler temperatures to form sufficiently stable hybrid complexes with the template. A primer need not reflect the exact sequence of the template but must be sufficiently complementary to hybridize with such template. The primer site is the area of the template to which a primer hybridizes. The primer pair is a set of primers including a 5' upstream primer that hybridizes with the 5' end of the sequence to be amplified and a 3' downstream primer that hybridizes with the complement of the 3' end of the sequence to be amplified.

Linkage describes the tendency of genes, alleles, loci or genetic markers to be inherited together as a result of their location on the same chromosome, and can be measured by percent recombination between the two genes, alleles, loci or genetic markers.

Polymorphism refers to the occurrence of two or more genetically determined alternative sequences or alleles in a population. A polymorphic marker or site is the locus at which divergence occurs. Preferred markers have at least two alleles, each occurring at frequency of greater than about 1 percent (%), and more preferably greater than about 10% or 20% of a selected population. A polymorphic locus may be as small as one base pair. Polymorphic markers include RFLPs, variable number of tandem repeats VNTRs, hypervariable regions, minisatellites, dinucleotide repeats, trinucleotide repeats, tetranucleotide repeats, simple sequence repeats, and insertion elements such as, for example, Alu. The first identified allelic form is arbitrarily designated the reference form while subsequently identified allelic forms are designated as alternative or variant alleles. The allelic form occurring most frequently in a selected population is often referred to as the wildtype form. Diploid organisms may be homozygous or heterozygous for allelic forms. A diallelic polymorphism has two forms. A triallelic polymorphism has three forms.

A SNP occurs at a polymorphic site occupied by a single nucleotide, which is the site of variation between allelic sequences. This site of variation is usually both preceded by and followed by highly conserved sequences e.g., sequences that vary in less than 1/100 or 1/1000 members of the populations of the given allele.

A SNP usually arises due to the substitution of one nucleotide for another at the polymorphic site. These substitutions include both transitions (i.e. the replacement of one purine by another purine or one pyrimidine by another pyrimidine) and transversions (i.e. the replacement of a purine by a pyrimidine or vice versa). SNPs can also arise from either a deletion of a nucleotide or from an insertion of a nucleotide relative to a reference allele.

Hybridizations, e.g., allele-specific probe hybridizations, are generally performed under stringent conditions. For example, conditions where the salt concentration is no more than about 1 Molar (M) and a temperature of at least 25 degrees-Celcius (°C), e.g., 750 mM NaCl, 50 mM NaPhosphate, 5 mM EDTA, pH 7.4 (5X SSPE) and a temperature of from about 25 to about 30°C.

An isolated nucleic acid is an object species invention that is the predominant species present (i.e., on a molar basis it is more abundant than any other individual species in the composition). Preferably, an isolated nucleic acid comprises at least about 50, 80 or 90% (on a molar basis) of all macromolecular species present. Most preferably, the object species is purified to essential homogeneity (contaminant species cannot be detected in the composition by conventional detection methods).

DESCRIPTION OF THIS INVENTION

I. NOVEL POLYMORPHISMS OF THIS INVENTION

The novel polymorphisms of this invention are listed in TABLE 1. The second column of TABLE 1 lists the names assigned to the fragments in which the polymorphism occurs. Sequences of fragments beginning with "HT" are available from the TIGR database which is accessible at: http://www.tigr.org/tdb/hgi/searching/hgi_reports.html, and those starting with "STSG" are from the Whitehead database which is accessible at http://carbon.wi.mit.edu:8000/cgi-bin/contig/sts_info?database=release. Sequences can also be obtained from the GenBank database accession number which may be obtained by using the BLAST program available at http://www.ncbi.nlm.nih.gov/BLAST. The fragments are all human genomic fragments. Also included within the scope of this invention are all analogous fragments of other species to such human genomic fragments of TABLE 1.

The second column of TABLE 1 lists the position of a polymorphic site within the fragment. These positions are numbered consecutively with the first base of the fragment sequence assigned the number one. The third column of TABLE 1 lists the base occupying the polymorphic site in the sequence in the data base. This base is arbitrarily designated the reference, or prototypical form, but is not necessarily the most frequently occurring form. The fifth column of TABLE 1 lists other base(s) found at the polymorphic site. The eighth column of TABLE 1 lists about 15 bases of sequence on either side of the polymorphic site in the fragment. These sequences are designated successive SEQ ID Nos. starting at SEQ ID No. 1. The sequences can be either DNA or RNA. In the latter, the T's shown in TABLE 1 are replaced by U's. The base occupying the polymorphic site is indicated using IUPAC-IUB nomenclature.

EP 1 024 200 A2

| FragmentCode | Position | RefAllele | FreqP | AltAllele | FreqQ | Heterozygocity | SequenceTag | ReversePrimer |
|---|---|---|---|---|---|---|---|---|
| D42043 | 1470 | G | 0.92 | A | 0.08 | 0.15 | ACCAGCGAGGGGAGTRTATCCACCAAGCAGA | |
| D42043 | 1697 | A | 0.54 | G | 0.46 | 0.5 | GGGAAACTGTGTTTCRGGACAGCAGCAGGAG | |
| D42043 | 2303 | A | 0.81 | T | 0.19 | 0.31 | GAGCATTCGGGTCAAWTCCATGGACACCCTG | |
| D45248 | 523 | C | 0.95 | G | 0.05 | 0.09 | GAGGGTGAATGCCGTSAAGACCAAAGTGGAA | |
| M69066 | 2504 | C | 0.72 | G | 0.28 | 0.4 | ATGGTGCCTTCAAGASCTTCACCAAACATAC | |
| M69066 | 2874 | A | 0.78 | G | 0.22 | 0.35 | CTCCACAGCCATCCCRGCCTTGGCATCTAGA | |
| M69066 | 3321 | A | 0.78 | C | 0.22 | 0.35 | GATTTTGGCCTCATTMCTTTACCACCCCCAC | |
| U56637 | 2228 | G | 0.13 | A | 0.88 | 0.22 | AGCAAGTTTGTTCCARGTGACCCATTGAGCT | |
| X12671 | 18 | T | 0.39 | A | 0.61 | 0.48 | AGGGCGAAGGTAGGCWGGCAGATACGTTCGT | |
| X12671 | 1504 | A | 0.94 | T | 0.06 | 0.1 | CAGGTTCTATTTGGAWTTTATATACAACCTG | |
| AB006782 | 93 | G | 0.82 | A | 0.18 | 0.29 | GAGATGGCCTTCAGCRGTTCCCAGGCTCCCT | |
| AB006782 | 1622 | T | 0.86 | A | 0.14 | 0.24 | AGCAGGCCTGATGGCWTCCCACTGGCCTCCA | |
| AB006782 | 1641 | C | 0.74 | A | 0.26 | 0.39 | CACTGGCCTCCACCAMCTGACCAGAGTGTTC | |
| AB006782 | 1658 | C | 0.91 | T | 0.09 | 0.16 | TGACCAGAGTGTTCTYTTCAGAGGACTGGCT | |
| AB006782 | 1664 | A | 0.62 | G | 0.38 | 0.47 | GAGTGTTCTCTTCAGRGGACTGGCTCCTTTC | |
| HT3708 | 1303 | C | 0.94 | G | 0.06 | 0.1 | GGAAACCTCATTGTTSTGTACAAAGTACTAG | |
| HT3708 | 1645 | C | 0.53 | T | 0.47 | 0.5 | TGAAACTCATCTCATYACCCTTTTCTGGGTT | |
| J02621 | 368 | C | 0.71 | T | 0.29 | 0.41 | AGAAACTAAAGAAGAYTTACCTGCGGAAAAC | |
| J02621 | 377 | G | 0.5 | A | 0.5 | 0.5 | AGAAGACTTACCTGCRGAAAACGGGGAAACG | |
| J02621 | 522 | C | 0.79 | G | 0.21 | 0.34 | GAGGAATATTTTTATSAACTATTTTGTAATG | |
| J02621 | 530 | T | 0.86 | C | 0.14 | 0.24 | TTTTTATCAACTATTYTGTAATGCAAGTTTT | |
| J02621 | 659 | T | 0.38 | C | 0.63 | 0.47 | TTGTTTATTTTTTGGYACAACCAGAAAATAG | |
| J02621 | 669 | A | 0.91 | G | 0.09 | 0.16 | TTTGGTACAACCAGARAATAGTGTGGGATAT | |
| J02621 | 674 | G | 0.86 | C | 0.14 | 0.24 | TACAACCAGAAAATASTGTGGGATATTGAAT | |
| J02621 | 691 | A | 0.95 | G | 0.05 | 0.1 | GTGGGATATTGAATTRTGGGAGGCTCTGACT | |
| J02621 | 701 | C | 0.85 | T | 0.15 | 0.25 | GAATTATGGGAGGCTYTGACTGTCTCGGGTG | |
| J02621 | 777 | A | 0.91 | C | 0.09 | 0.16 | AATACAAAGCATATTMAATGGCAATATGGAG | |
| J02621 | 838 | A | 0.64 | C | 0.36 | 0.46 | TAAATTACTTCTATTMCCATGTTGTTTTTTA | |
| J02621 | 842 | T | 0.85 | C | 0.15 | 0.25 | TTACTTCTATTACCAYGTTGTTTTTTAGTAG | |
| J02621 | 851 | T | 0.91 | G | 0.09 | 0.16 | TTACCATGTTGTTTTKTAGTAGAATTGTTTC | |
| J02621 | 921 | T | 0.91 | G | 0.09 | 0.16 | GAATTGTGTGCACTCKGTAACATCTTTGGTT | |
| J02621 | 1014 | A | 0.42 | G | 0.58 | 0.49 | TGTAGTGTACGTGCTRTTGAGTTGTGAACTG | |
| K01160 | 45 | T | 0.56 | C | 0.44 | 0.49 | GATGCTGGGGGCCCTYGCCCTGACCACCGTG | |
| K01160 | 62 | T | 0.91 | C | 0.09 | 0.17 | CCCTGACCACCGTGAYGAGCCCCTGTGGAGG | |

| Sequence | Value | Value | Base | Value | Base | Position | Accession |
|---|---|---|---|---|---|---|---|
| CACCGTGATGAGCCCYTGTGTGGAGGTGAAGAC | 0.42 | 0.3 | T | 0.7 | C | 69 | K01160 |
| CATTGTGGCTGACCAYGTCGCCTCTTATGGT | 0.49 | 0.44 | T | 0.56 | C | 99 | K01160 |
| TGTGGCTGACCACGTYGCCTCTTAYGGTGTGTA | 0.49 | 0.43 | T | 0.57 | C | 102 | K01160 |
| CCACGTCGCCTCTTAYGGTGTAAACTTGTAC | 0.31 | 0.2 | C | 0.8 | T | 111 | K01160 |
| CTTGTACCAGTCTTAYGGTCCCTCTGGCCAG | 0.3 | 0.19 | T | 0.81 | C | 135 | K01160 |
| CCTCTGGCCAGTACASCCATGAATTTGATGG | 0.38 | 0.25 | G | 0.75 | C | 155 | K01160 |
| GACAAACATCGCTGTSCTAAAACATAACTTG | 0.49 | 0.58 | G | 0.42 | C | 279 | K01160 |
| CGCTGTCCTAAAACAYAACTTGAACAGTCTG | 0.22 | 0.13 | C | 0.88 | T | 288 | K01160 |
| ACGCTCCAACTCTACYGCTGCTACCAATGAG | 0.36 | 0.23 | T | 0.77 | C | 324 | K01160 |
| CAAGTCTCCCGTGACRCTGGGTCAGCCCAAC | 0.41 | 0.29 | G | 0.71 | A | 378 | K01160 |
| TGGGTCAGCCCAACAYCCTCATCTGTCTTGT | 0.49 | 0.56 | C | 0.44 | T | 395 | K01160 |
| TGTGGTCAACATCACMTGGCTGAGCAATGGG | 0.48 | 0.4 | C | 0.6 | A | 444 | K01160 |
| CTTCTGCTGAGGAGAKTTATGACTGCAAGGT | 0.43 | 0.31 | T | 0.69 | G | 563 | K01160 |
| GGTGGAGCACTGGGGMCTGGACAAGCCTCTT | 0.45 | 0.35 | A | 0.65 | C | 591 | K01160 |
| CACTGGGGCCTGGACRAGCCTCTCTGAAAC | 0.5 | 0.5 | G | 0.5 | A | 598 | K01160 |
| GCCTGAGATTCCAGCMCCTATGTCAGAGCTC | 0.22 | 0.13 | A | 0.88 | C | 636 | K01160 |
| AGAGACTGTGGTCTGYGCCCTGGGATTGTCT | 0.3 | 0.19 | T | 0.81 | C | 669 | K01160 |
| GGTCTGCGCCCTGGGRTTGTCTGTGTGGGCCTC | 0.5 | 0.54 | G | 0.46 | A | 678 | K01160 |
| CTGTCTTCATCATCCRAGGCCTGCGTTCAGT | 0.19 | 0.11 | A | 0.89 | G | 728 | K01160 |
| CAGACACCACCAAGGGCCMTTGTGAATCCCATCC | 0.3 | 0.19 | A | 0.81 | C | 768 | K01160 |
| AATCCCATCCTGGAAKGGAAGGTGCATCGCC | 0.48 | 0.4 | G | 0.6 | T | 789 | K01160 |
| AGAGTGGACTTGCTAMATGACCTAGCATTAT | 0.13 | 0.07 | A | 0.93 | C | 835 | K01160 |
| TATCTGCTCAGAGCTYACAAATGCCTTTGAA | 0.38 | 0.25 | T | 0.75 | C | 945 | K01160 |
| TGATTTTTTCTTCTYAAGTGTTACCTACTA | 0.43 | 0.69 | C | 0.31 | T | 993 | K01160 |
| GTTACCTACTAAGAGWTGCCTGGAGTAAGCC | 0.13 | 0.93 | A | 0.07 | T | 1013 | K01160 |
| CTAAGAGTTGCCTGGRGTAAGCCACCACCAGCT | 0.12 | 0.06 | G | 0.94 | A | 1021 | K01160 |
| TGCCTGGAGTAAGCCRCCCAGCTACCTAATT | 0.3 | 0.19 | G | 0.81 | A | 1029 | K01160 |
| AACAAATTCCCTTAWGAGATATATGTCAAA | 0.12 | 0.06 | A | 0.94 | T | 1095 | K01160 |
| TTCCCTTTATGAGATMTATGTCAAATTTTTC | 0.38 | 0.25 | C | 0.75 | A | 1101 | K01160 |
| TGAAACCGTGGCTAASAATTGGGAGACTCTC | 0.22 | 0.13 | C | 0.88 | G | 1153 | K01160 |
| CGTGGCTAAGAATTGKGAGACTCTCTGTTT | 0.22 | 0.13 | T | 0.88 | G | 1159 | K01160 |
| TTTACCAGATCATTTKTCATGTCCAGTAACA | 0.36 | 0.23 | T | 0.77 | G | 1208 | K01160 |
| CATTACAGTAGATAAYTGTAAGAAACTTGGC | 0.22 | 0.13 | T | 0.88 | C | 1160 | L12168 |
| AGATAATCAACAGTARGGATGTCAAAGTTCA | 0.22 | 0.13 | G | 0.88 | A | 1222 | L12168 |
| TTCCCTGGATTGTGARATAGTCAGTGCCAAA | 0.32 | 0.2 | G | 0.8 | A | 1322 | L12168 |

| L12168 | 1585 | G | 0.88 | A | 0.13 | 0.22 | GCTTCTCTGCTCTGARAAGCACAGCTACCTG |
|--------|------|---|------|---|------|------|----------------------------------|
| L12168 | 1877 | A | 0.95 | G | 0.05 | 0.1 | GAGCATGAAGGTAGCRGAAGCTGGTGTGTTT |
| M15661 | 142 | G | 0.92 | A | 0.08 | 0.15 | TGTATGCCCAGGGAARGAGGCGCTATGATCG |
| M29064 | 703 | A | 0.63 | G | 0.38 | 0.47 | TAATGCAGAAGTAAGRAAGGCTTTGTCTAGA |
| M37766 | 65 | C | 0.92 | T | 0.08 | 0.15 | GCTCTGGAATTGCTAYTGCTGCCTCTGTCAC |
| M37766 | 107 | T | 0.95 | C | 0.05 | 0.1 | AGCATTCAAGGTCACYTGGTACATATGACCG |
| M37766 | 323 | G | 0.87 | C | 0.13 | 0.23 | TCTAAGGTCCAGAAASAGGACAACAGCACCT |
| M37766 | 585 | A | 0.95 | T | 0.05 | 0.1 | CCCTTATGCCACATAWTTACTCCAGGTGTTA |
| M37766 | 790 | A | 0.83 | C | 0.17 | 0.28 | AAACTTCAAGGCCAGMAGATCTTGCCTGTTG |
| U05340 | 541 | T | 0.67 | C | 0.33 | 0.44 | TGCGCCAGAGGGTTAYCAGAACAGACTGAAA |
| U05340 | 545 | A | 0.88 | G | 0.13 | 0.22 | CCAGAGGGTTATCAGRACAGACTGAAAGTAC |
| U05340 | 594 | G | 0.95 | C | 0.05 | 0.1 | CTCCTGGCTCCAGCCSGAAGACCTGCCGTTA |
| U05340 | 966 | G | 0.88 | A | 0.13 | 0.22 | TGTCCAGTGGTTCACRTTCTGGCCACATCCA |
| U05340 | 1012 | T | 0.89 | C | 0.11 | 0.2 | GGTAGCAGAACACCAYGTGGCCACACTGAGT |
| U05340 | 1062 | C | 0.84 | T | 0.16 | 0.26 | GTGGGCTGCGCTGGGYCCCAGATGGACGACA |
| U05340 | 1093 | T | 0.88 | C | 0.13 | 0.22 | TTTGGCCAGTGGTGGYAATGATAACTTGGTC |
| U05340 | 1111 | T | 0.84 | A | 0.16 | 0.26 | TGATAACTTGGTCAAWGTGTGGCCTAGTGCT |
| U05340 | 1221 | T | 0.38 | C | 0.63 | 0.47 | GGCAGTCCAATGTCCYGGCAACAGGAGGGGG |
| U05340 | 1256 | C | 0.88 | T | 0.13 | 0.22 | AGTGATCGACACATTYGCATCTGGAATGTGT |
| U05340 | 1267 | T | 0.84 | C | 0.16 | 0.26 | CATTCGCATCTGGAAYGTGTGCTCTGGGGCC |
| U05340 | 1305 | A | 0.63 | T | 0.38 | 0.47 | GTGCCGTGGATGCCCWTTCCCAGGTGTGCTC |
| U05340 | 1339 | T | 0.84 | C | 0.16 | 0.26 | CCTCTGGTCTCCCCAYTACAAGGAGCTCATC |
| U05340 | 1452 | G | 0.87 | C | 0.13 | 0.23 | CATCCCGGGTCCTGASTCTGACCATGAGCCC |
| U05340 | 1509 | T | 0.81 | C | 0.19 | 0.3 | CAGCAGATGAGACCCYGAGGCTATGGCGCTG |
| U05340 | 1586 | C | 0.81 | T | 0.19 | 0.3 | GCAGCCAAAAGCAGCYTCATCCACCAAGGCA |
| U43522 | 221 | A | 0.63 | G | 0.38 | 0.47 | GACTGCAATGTGCCGRTCTTAGCTGCTGCCT |
| U43522 | 269 | T | 0.63 | C | 0.37 | 0.47 | GTCCGAGCCCCTGAGYCGAGTAAAGTTGGGC |
| U43522 | 287 | G | 0.74 | A | 0.26 | 0.38 | AGTAAAGTTGGGCACRTTACGCCGGCCTGAA |
| U43522 | 404 | A | 0.68 | G | 0.32 | 0.44 | CTTCAATCCTGGGAARAACTTCAAACTGGTC |
| U43522 | 565 | A | 0.94 | C | 0.06 | 0.1 | ACTGGCTGCACCCACMGATGACAGTGGGTGA |
| U43522 | 572 | A | 0.49 | G | 0.51 | 0.5 | GCACCCACAGATGACRGTGGGTGAGGTGCAG |
| U43522 | 1481 | C | 0.89 | T | 0.11 | 0.2 | AACCCTGCGAAGGCCYGGAGGTCCACAGTAT |
| U43522 | 1583 | T | 0.46 | C | 0.54 | 0.5 | TGTCTACACAAATCAYAAAGGGGAGAAAATC |
| U43522 | 2755 | A | 0.63 | C | 0.37 | 0.47 | TTTATATGAATGATAMGTCCCCATTGACGCC |
| U43522 | 2870 | C | 0.82 | T | 0.18 | 0.3 | TAACCTGGACCGGACYGATGACCTGGTGTAC |

| | | | | | | | |
|---|---|---|---|---|---|---|---|
| U43522 | 3122 | G | 0.82 | A | 0.18 | 0.29 | CAAGATGCGGCTGGCRCAGCAGAACGCCGTG |
| U43522 | 3146 | G | 0.81 | A | 0.19 | 0.31 | CGCCGTGACCTCCCTRAGTGAGGAGTGCAAG |
| U43522 | 3182 | A | 0.95 | G | 0.05 | 0.1 | GATGCTGACGGCTTCRCACACCCTGGCTGTG |
| U43522 | 3361 | A | 0.69 | G | 0.31 | 0.42 | GTGGGTCTTCCAGGGRGAAGGCCAAGGGGAG |
| U43522 | 3610 | A | 0.83 | G | 0.17 | 0.28 | CCCCTAGCTTTATATRTGGACATGGCAGGCC |
| U46692 | 466 | T | 0.94 | C | 0.06 | 0.1 | CAGAGCGTGCACTTGYGATCCTAAAATAAGC |
| U66711 | 820 | T | 0.9 | G | 0.1 | 0.18 | CAAGTCCCATGGACAKGCTGACAGGGTCCCC |
| U66711 | 884 | T | 0.95 | G | 0.05 | 0.1 | CTGTGTACGTGGGTGKGCAGTGCACGTGAGA |
| X05276 | 732 | A | 0.94 | G | 0.06 | 0.12 | AACTTGCCCAGGCCARAGAAGAGAACGTGGG |
| X05276 | 903 | A | 0.83 | G | 0.17 | 0.28 | TTCGCTTTGCTGGAARTGTCAAGCAAATTAT |
| X05276 | 1140 | A | 0.88 | C | 0.13 | 0.22 | CCCCACACTTTGAAGMATACAGACCCCAGTA |
| X05276 | 1386 | T | 0.92 | C | 0.08 | 0.15 | TGCAGGACAAACATCYTCTCAAGCAGTCAAC |
| X05276 | 1402 | G | 0.94 | C | 0.06 | 0.12 | TCTCAAGCAGTCAACSTAGAATGCTTGGGAA |
| X05276 | 1607 | G | 0.88 | A | 0.13 | 0.22 | TTCGAGACCAGCCCARCCAACATGGCGAAAC |
| X60221 | 98 | C | 0.89 | T | 0.11 | 0.2 | AAGAATGCAGCCTTCYTAGGTCCAGGGGTAT |
| X60221 | 242 | A | 0.94 | G | 0.06 | 0.1 | TTTCTTTATCCTAAARCTGGTGTAACAGGAC |
| X60221 | 265 | A | 0.78 | G | 0.22 | 0.35 | AACAGGACCCTATGTRCTCGGAACTGGGCTT |
| X60221 | 541 | A | 0.83 | G | 0.17 | 0.28 | CCTTTTTGATGTGCARAGGAATAACATTGCT |
| X60221 | 637 | T | 0.87 | C | 0.13 | 0.23 | GGACTATCATATATCYGTGCAGAACATGATG |
| Y00097 | 1060 | T | 0.8 | G | 0.2 | 0.32 | ACTCTGCTGAAGCTGKCTGGGGGAGATGATG |
| Y00097 | 1707 | T | 0.18 | C | 0.82 | 0.3 | AATAGCAGACACACCYAGTGGAGACAAAACT |
| Y00097 | 2034 | T | 0.94 | C | 0.06 | 0.1 | ATTCATTGAGAAATAYGACAAGTCTCTCCAC |
| Y00097 | 2045 | T | 0.94 | C | 0.06 | 0.1 | AATATGACAAGTCTCYCCACCAAGCCATTGA |
| Z48950 | 687 | C | 0.95 | T | 0.05 | 0.1 | AAGCGCTCCCTCTACYGGCGGGGTGAAGAAG |
| Z48950 | 1729 | C | 0.88 | G | 0.13 | 0.22 | CTCTAGCTGATAATGSAAACACTAACTGGGG |
| Z48950 | 1893 | A | 0.95 | G | 0.05 | 0.1 | CTCTGCTGAGGAAACRGTACCGAAGTTCTTT |
| Z49107 | 612 | C | 0.9 | T | 0.1 | 0.18 | CTGGGAGGGCTGTACYCATCCAAGTCCATCC |
| D11086 | 280 | T | 0.63 | A | 0.38 | 0.47 | CCTCACTCTGCATTAWTGGTACAAGAACTCG |
| D11086 | 535 | G | 0.94 | C | 0.06 | 0.12 | GCTAGAACTGAACTGSAACAACAGATTCTTG |
| D11086 | 840 | T | 0.75 | A | 0.25 | 0.38 | GATTGATTATCAGCCWTCTCTGTGTGTATTT |
| D11086 | 1260 | G | 0.94 | C | 0.06 | 0.12 | TGTGCCCCCATGTAASCACCCCTTCATTTGG |
| D38076 | 171 | A | 0.35 | G | 0.65 | 0.45 | CCGCCAAGGACACTCRTGAGGACCATGATAC |
| D38076 | 210 | C | 0.56 | A | 0.44 | 0.49 | AGAATACAGACGAGTMCAACCATGACCCTCA |
| D38076 | 303 | G | 0.8 | A | 0.2 | 0.32 | AACTTTTTAAAATGCRGGCAAAACTGTTCCG |
| D38076 | 317 | C | 0.73 | T | 0.27 | 0.4 | CGGGCAAAACTGTTCYGATTTGCCTCTGAGA |

| | | | | | | | |
|---|---|---|---|---|---|---|---|
| D38076 | 328 | T | 0.71 | A | 0.29 | 0.41 | GTTCCGATTTGCCTCWGAGAACGATCTCCCA |
| D38076 | 420 | G | 0.55 | A | 0.45 | 0.5 | TCCGCCTCCTCATGCRGAGGGACAAGACCCT |
| D38076 | 439 | G | 0.7 | T | 0.3 | 0.42 | GGACAAGACCCTGAAKATCTGTGCCAACCAC |
| D38076 | 487 | C | 0.83 | A | 0.17 | 0.28 | GGAGCTGAAGCCCAAMGCAGGTAGCGACCGT |
| D38076 | 496 | C | 0.89 | T | 0.11 | 0.2 | GCCCAACGCAGGTAGYGACCGTGCCTGGGTC |
| D38076 | 523 | C | 0.79 | T | 0.21 | 0.33 | GGTCTGGAACACCCAYGCTGACTTCGCCGAC |
| D38076 | 539 | G | 0.8 | C | 0.2 | 0.32 | GCTGACTTCGCCGACSAGTGCCCCAAGCCAG |
| D38076 | 569 | C | 0.77 | T | 0.23 | 0.35 | GAGCTGCTGGCCATCYGCTTCCTGAATGCTG |
| D38076 | 625 | G | 0.68 | A | 0.32 | 0.43 | GTTTGAAGAATGCAGRAAAGAGATCGAAGAG |
| D38076 | 677 | G | 0.71 | A | 0.29 | 0.42 | AAAAATGATCATGCCRAAAAGTGGCGGAAA |
| D38076 | 831 | G | 0.83 | A | 0.17 | 0.28 | CCTCTTTTTCGGTTTRTTTTTATTCTTTCAT |
| D87017 | 259 | G | 0.94 | A | 0.06 | 0.1 | GAAGCTACAGCTGCCRGGTCACGCATGAAGG |
| L06132 | 326 | T | 0.78 | C | 0.22 | 0.35 | TACAGAGAAATGGAAYACCGACAATACACTA |
| L06132 | 551 | C | 0.94 | T | 0.06 | 0.1 | CGAGGGCTGGCTGGCYGGCTACCAGATGAAT |
| L06132 | 996 | G | 0.94 | C | 0.06 | 0.1 | CTATTTTGCAGCATASCTACCTTCAGAATTT |
| L06132 | 1128 | T | 0.72 | C | 0.28 | 0.4 | GGTACAGAAGAAACCYATTTCCAAAAAAGGT |
| L06132 | 1222 | A | 0.78 | G | 0.22 | 0.35 | TCTAGAAATGGCTGCRAGTGGAAGCGGATAA |
| L06132 | 1228 | A | 0.95 | T | 0.05 | 0.1 | AATGGCTGCAAGTGGWAGCGGATAATATGTA |
| L06132 | 1311 | C | 0.78 | T | 0.22 | 0.35 | AATCGAACCTTGGTTYCCTAACCCTAATTGA |
| L06132 | 1317 | C | 0.95 | T | 0.05 | 0.1 | ACCTTGGTTCCCTAAYCCTAATTGATGAGAG |
| L06132 | 1321 | A | 0.72 | G | 0.28 | 0.4 | TGGTTCCCTAACCCTRATTGATGAGAGGCTC |
| L06132 | 1322 | A | 0.83 | C | 0.17 | 0.28 | GGTTCCCTAACCCTAMTTGATGAGAGGCTCG |
| L06132 | 1323 | T | 0.83 | C | 0.17 | 0.28 | GTTCCCTAACCCTAAYTGATGAGAGGCTCGC |
| L06132 | 1391 | T | 0.78 | C | 0.22 | 0.35 | TTTTTAGACAGATCTYCATGACCTGTTCCCA |
| L06132 | 1605 | A | 0.78 | G | 0.22 | 0.35 | CAGCTATAAGCTTGGRAGTGTCTGTGTGATT |
| L06132 | 1638 | A | 0.83 | G | 0.17 | 0.28 | AATCACATGGTGACARCACTCAGAATCTAAA |
| L06132 | 1693 | T | 0.83 | C | 0.17 | 0.28 | ACTCAATTTGTTTTTYAGCAGTTTAATGGGT |
| M19311 | 381 | G | 0.94 | T | 0.06 | 0.12 | ACTTCGCCATGTGATKACAAACCTTGGAGAG |
| M19311 | 726 | T | 0.88 | C | 0.13 | 0.22 | GCATGTGGCTTACTCYGGCTATATCTAAGCC |
| M19311 | 763 | A | 0.8 | G | 0.2 | 0.32 | CACATCTAAACTTAGRTGGAGTTGGTCAAAT |
| M22760 | 350 | T | 0.85 | C | 0.15 | 0.25 | TTCGAATCCTAGAGGYTGTTAAGGACAAAGC |
| M22760 | 408 | T | 0.81 | C | 0.19 | 0.31 | TGTCATCCAGGAACTYAGACCAACTTTAAAT |
| M58028 | 962 | T | 0.9 | C | 0.1 | 0.18 | TACCTTTAGCATCTGYGACACCTCCAACTTC |
| M58028 | 1059 | G | 0.83 | C | 0.17 | 0.28 | TTGGTGGCCTCACTGSCAGAACCTGACTTTG |
| M58028 | 1112 | G | 0.7 | C | 0.3 | 0.42 | TTCTCGCCCTGCCCASCTGCACATTGGCTTC |

| | | | | | | | |
|---|---|---|---|---|---|---|---|
| M58028 | 1468 | G | 0.83 | A | 0.17 | 0.29 | ACAAGTGCCTCCAGCRCCAGAACCGTTATGA |
| M58028 | 2254 | G | 0.94 | C | 0.06 | 0.12 | CTGACTGCGTGACCTSGGCCTGCCACCACTG |
| M58028 | 2348 | G | 0.85 | A | 0.15 | 0.26 | AAGCTCAGGAGCGCCRTTCTGGTCTGGGCCC |
| M58028 | 2492 | C | 0.89 | T | 0.11 | 0.2 | CCGAGCTGCTGTGGCYACATTCCTGCAGTCT |
| M58028 | 2575 | G | 0.94 | C | 0.06 | 0.12 | ACCAGGAGCTGCAGASCGCCAATGCCTCTGT |
| M60784 | 376 | A | 0.75 | G | 0.25 | 0.38 | CCCTTTCTATGACAARCCTATGCGTATCCAG |
| U23852 | 1626 | G | 0.94 | C | 0.06 | 0.12 | TTGACTACCTGCGCASTGTGCTGGAGGACTT |
| U23852 | 1874 | A | 0.64 | G | 0.36 | 0.46 | CTGCACATGTCTTGTRCATGTGTAGCCTGTG |
| U23852 | 1906 | A | 0.95 | G | 0.05 | 0.1 | ATGTATGTCTTGGACRCTGTACAAGGTACCC |
| U30825 | 132 | G | 0.89 | A | 0.11 | 0.19 | GACCGACGTGCGCGARAAGGACTTGGAGGAC |
| U30825 | 155 | A | 0.95 | T | 0.05 | 0.1 | TGGAGGACCTGTTCTWCAAGTACGGCCGCAT |
| U30825 | 443 | T | 0.94 | C | 0.06 | 0.1 | ACCTGAAGGATCACAYGCGAGAAGCTGGGGA |
| U60975 | 1302 | G | 0.93 | A | 0.07 | 0.13 | GCCACAGTAACAACCRCACCAATTTATACAT |
| U60975 | 1641 | G | 0.86 | C | 0.14 | 0.24 | TGGCTCAGCGCCTCASTCAGCTCCTCAACCT |
| U60975 | 1778 | G | 0.67 | A | 0.33 | 0.44 | TCTAGCAGTGCTGGARCCAGGTGGCGAGAGG |
| U60975 | 1833 | G | 0.94 | A | 0.06 | 0.1 | CATGGGGAGACCACGRCGGAATCATCACGGC |
| U60975 | 2695 | A | 0.95 | T | 0.05 | 0.1 | TTCTGGCCTGGAGACWGTAGAAGCTTTGGCT |
| U60975 | 3416 | G | 0.95 | C | 0.05 | 0.1 | AATACCTGTGTCAAASAAGAGAACACCTGTC |
| U60975 | 3757 | T | 0.67 | G | 0.33 | 0.44 | CTGCAGGGACTGGTCKGATGAAGCCAACTGT |
| U60975 | 3862 | G | 0.94 | A | 0.06 | 0.1 | GTGTGACGGGGATACRGACTGCCAGGATGGT |
| U60975 | 3879 | C | 0.93 | G | 0.07 | 0.13 | ACTGCCAGGATGGTTSCGATGAGGATCCAGT |
| U60975 | 3909 | A | 0.93 | G | 0.07 | 0.13 | TCAACTGTGAGAAGARGTGCAATGGATTCCG |
| U60975 | 3934 · | C | 0.52 | T | 0.48 | 0.5 | ATTCCGCTGCCCAAAYGGCACTTGCATCCCA |
| U60975 | 4273 | C | 0.93 | A | 0.07 | 0.13 | TGATGAAGCCAACTGMGAAAACCCCACAGAA |
| U60975 | 4533 | A | 0.93 | G | 0.07 | 0.13 | ACCGAGATTGTGCAGRTGGCTCTGACGAGGA |
| U60975 | 4948 | T | 0.54 | A | 0.46 | 0.5 | AAAAATGCCCTCTGCWTCTTGTGTATATAAT |
| U60975 | 5635 | T | 0.9 | A | 0.1 | 0.18 | CAATCTGACAGCTCAWACATCCTATGAGATT |
| U60975 | 5851 | G | 0.95 | A | 0.05 | 0.1 | CCTCTATCGCAACCCRAAGAGCTTGACTACT |
| U60975 | 6095 | A | 0.94 | G | 0.06 | 0.12 | GACTTGTTGTATGCARTTGCAGTCAAAGATC |
| U60975 | 6695 | A | 0.71 | G | 0.29 | 0.41 | CACCGGAGGCTGCAGRGCAGCTTCACCGCCT |
| X07743 | 72 | C | 0.95 | T | 0.05 | 0.1 | AGCATGGAACCAAAGYGGATCAGAGAGGGCT |
| X07743 | 350 | T | 0.42 | G | 0.58 | 0.49 | GGTTCGGGATATCAAKAAGGCCATTAAATGC |
| X07743 | 608 | G | 0.76 | A | 0.24 | 0.37 | CATGATTGCTTCATCRCTGCTCAATGAGGGG |
| X07743 | 688 | T | 0.95 | G | 0.05 | 0.1 | CTGAAAACCCTTTCCKGGACAACCCTGATGC |
| X07743 | 1078 | A | 0.9 | G | 0.1 | 0.19 | GCACAGAGTGGATCARAGCCATCCAGATGGC |

| | | | | | | | | |
|---|---|---|---|---|---|---|---|---|
| X07743 | 1116 | G | 0.68 | C | 0.32 | 0.43 | ACTGGGAAGTAAAGASACTCCTGCATTCCTC |
| X07743 | 1154 | T | 0.84 | C | 0.16 | 0.26 | CCTGAGGGAAGCCCAYGGACAAGCTCAGTCC |
| X07743 | 1437 | T | 0.7 | C | 0.3 | 0.42 | TTCCTGAGGTCCCCCYAGCTTAAAAAAAAAA |
| X07743 | 1464 | A | 0.7 | G | 0.3 | 0.42 | AAAAAAATCTGCCCCRTGATTCTAACACTCG |
| X07743 | 1661 | T | 0.95 | C | 0.05 | 0.1 | ATCTTCTACTTGCTCYTGGCCTTGAGATCGT |
| X07743 | 2077 | T | 0.67 | C | 0.33 | 0.44 | CTGGTGAATGTGTCTYGCGGCAGCTGCAGCA |
| X07743 | 2079 | C | 0.9 | T | 0.1 | 0.18 | GGTGAATGTGTCTTGYGGCAGCTGCAGCAAG |
| X07743 | 2422 | A | 0.35 | G | 0.65 | 0.46 | ACAGCACCCATTGAARCAGATATGTGTGTGA |
| X07743 | 2428 | A | 0.95 | G | 0.05 | 0.1 | CCCATTGAAACAGATRTGTGTGTGAAAGTAT |
| X51521 | 387 | G | 0.88 | C | 0.13 | 0.22 | CCTGAAGATGTGGCTSAGGAGCTCATCCAGG |
| X51521 | 482 | C | 0.95 | T | 0.05 | 0.1 | CCCCCCTGAGACTGCYGTGCTCTTGGGGTCC |
| X51521 | 2090 | G | 0.94 | A | 0.06 | 0.1 | GCAGGCCTGATTCTCRCGATTATTCTCGAAT |
| X51521 | 2467 | A | 0.39 | T | 0.61 | 0.48 | GGAGGACACTTGGATWTTTTTTAGTTCTTT |
| X51521 | 2544 | T | 0.94 | C | 0.06 | 0.1 | ATTAATGATCAGCTAYATACTATTTATATAC |
| X51521 | 2797 | A | 0.9 | T | 0.1 | 0.18 | CTATGCACACTTTTAWTTTTGTCAGATTCAC |
| X51521 | 3013 | G | 0.95 | A | 0.05 | 0.1 | GGTGGATTCCATGCCRCAGACATGAAATAAA |
| X52882 | 570 | A | 0.94 | G | 0.06 | 0.1 | ATTAAATACACAGACRTAAGAGGCCAGCCAC |
| X52882 | 692 | C | 0.78 | T | 0.22 | 0.35 | GGTGGGATCCCAGGGYATGCCCAAGAGAATC |
| X52882 | 1177 | A | 0.81 | G | 0.19 | 0.3 | ATGATTTCATGTGTGRTGAGATGGAGCGCTC |
| X52882 | 1369 | A | 0.95 | G | 0.05 | 0.1 | TTCTTGTTATTCCCARTACACTAGCAGTTAA |
| X52882 | 1612 | T | 0.88 | C | 0.12 | 0.21 | GAATTGATGATCTTAYTAAATTACATCCAGA |
| AC002115 | 41 | C | 0.75 | T | 0.25 | 0.38 | CAAGAACTACAAGACYGCCCCTTTTGACAGC |
| AC002115 | 386 | G | 0.88 | T | 0.13 | 0.22 | TAATAAAAACTCATTKGAAAAGTGAGACTAT |
| D13118 | 250 | T | 0.71 | G | 0.29 | 0.41 | CACAGCAGCCAAGTTKATTGGTGCTGGGGCA |
| D13118 | 340 | G | 0.83 | A | 0.17 | 0.28 | CATTGGCTATGCCAGRAACCCGTCTCTCAAG |
| D13118 | 433 | C | 0.9 | T | 0.1 | 0.18 | TTTGATGGTCGCCTTYCTCATCCTCTTCGCC |
| D13118 | 499 | C | 0.83 | T | 0.17 | 0.28 | TACTGCAACTCCACAYCATTCTTGGTGCTGG |
| D13118 | 505 | T | 0.92 | C | 0.08 | 0.15 | AACTCCACACCATTCYTGGTGCTGGGGTGTG |
| D13118 | 521 | T | 0.79 | C | 0.21 | 0.34 | TGGTGCTGGGGTGTGYTAAGCTTTACCATTA |
| D26068 | 232 | C | 0.83 | A | 0.17 | 0.28 | ACGGCTAGTCAGAGAMAAAGACACAGATAAA |
| D26068 | 259 | C | 0.86 | T | 0.14 | 0.24 | TAAATTTAAAGGATTYTGCTATGTAGAATTC |
| D26068 | 1977 | C | 0.33 | G | 0.67 | 0.44 | AAGTCCCAGTCTGTCSTATGGGGAGCAGTTT |
| D26068 | 2003 | G | 0.9 | A | 0.1 | 0.18 | AGTTTGGGGGCGGCCRGCAGCAGGAGCCTGG |
| D31890 | 183 | A | 0.92 | G | 0.08 | 0.15 | CTGCTGCTGCCACCARCCACACCACTGATAA |
| D31890 | 381 | A | 0.93 | C | 0.07 | 0.13 | GGGATCACCTGACTGMCATCACCTTAAAGGT |

| | | | | | | | |
|---|---|---|---|---|---|---|---|
| D31890 | 599 | C | 0.9 | G | 0.1 | 0.18 | ACCAAGAAGGGTGAGSTGAGCATCATTCCGT |
| D31890 | 742 | A | 0.92 | C | 0.08 | 0.15 | CTTTGTGAGGCAGAAMTTTATCATCCGCTCT |
| D31890 | 952 | C | 0.9 | G | 0.1 | 0.18 | GGTTGGTGGCATCGASCGGGTTTATGAAATT |
| D31890 | 1797 | G | 0.1 | C | 0.9 | 0.18 | GCACAACAGTTGGCASTTCTGTCTAGAAAAT |
| HT3238 | 53 | G | 0.17 | C | 0.83 | 0.28 | CAGAACGCTTCAGTTSTGCTCTGCAAGGATA |
| HT3238 | 71 | T | 0.74 | A | 0.26 | 0.39 | CTCTGCAAGGATATAWAATAACTGATTGGTG |
| HT3238 | 258 | G | 0.94 | C | 0.06 | 0.1 | GCATTCTGCTTCAGASCAAGAATGCTGGGGC |
| HT3238 | 479 | C | 0.78 | T | 0.22 | 0.35 | ACCAGCCAGCTCCCGYTCGAATCTGATGCTG |
| HT3238 | 505 | A | 0.75 | T | 0.25 | 0.38 | TGCTGTGGAATGCTTWAATTACCAACACTAT |
| HT3238 | 593 | A | 0.94 | G | 0.06 | 0.1 | GGAATTATTGGGGTCRAAGGTGCTAAAATCA |
| HT3238 | 723 | T | 0.78 | A | 0.22 | 0.35 | GGGTTGTAGAGTGCAWAAAGATCATCCTTGA |
| HT3238 | 1028 | C | 0.78 | T | 0.22 | 0.35 | AGAGCTCGGAATCTTYCTCTTCCTCCACCAC |
| HT3238 | 1263 | C | 0.94 | T | 0.06 | 0.1 | GTGATCTTGGTGGACYTATTATTACTACACA |
| HT3238 | 1495 | C | 0.81 | T | 0.19 | 0.3 | TTCTGGAAAGTTTTTYTAAGACTAGTGAAGA |
| HT3238 | 1775 | T | 0.89 | C | 0.11 | 0.2 | CCCATGTAATTATTGYGAACACCTTGCTTTG |
| HT3238 | 2088 | T | 0.85 | C | 0.15 | 0.25 | ATAGGCAAAATAACTYGGCAAACTTAGTTCT |
| HT3238 | 2131 | A | 0.94 | G | 0.06 | 0.1 | ATGGTTTGGAAGTCTRTTGCTGGGAAGAAAT |
| M20471 | 385 | G | 0.9 | C | 0.1 | 0.18 | AGTGGATCGATTGCASTCAGAGCCTGAAAGT |
| M20471 | 779 | T | 0.88 | C | 0.13 | 0.22 | GTCTCCCGCATGCGCYCAGTCCTCATCTCCC |
| M20471 | 924 | C | 0.88 | T | 0.13 | 0.22 | TCTTTTGGACCAAACYTTTTGTCTTTAGAG |
| M22538 | 103 | C | 0.8 | T | 0.2 | 0.32 | ATTTGCATAAGACAGYTATGCAAAATGGAGC |
| M22538 | 382 | A | 0.93 | T | 0.07 | 0.13 | CTTTTTATACAATGTWTAATCGAAAGCCAGT |
| M22538 | 763 | T | 0.92 | G | 0.08 | 0.15 | GTGTACAAGCAGGCCKTTAATTTATATTGAA |
| U10439 | 1165 | G | 0.78 | A | 0.22 | 0.35 | GTGCTAATTGACATGRAAAGGCAGGGGGATG |
| U10439 | 1304 | A | 0.43 | G | 0.57 | 0.49 | CAATCCCTGAGACCARAAGAAACGCAGAGTT |
| U10439 | 1889 | G | 0.78 | A | 0.22 | 0.35 | AAGCCAAGGACAGTGRAAAATCAGAAGAATC |
| U10439 | 2835 | G | 0.74 | A | 0.26 | 0.38 | AGGGAATCGCTGTGTRAAGGAGATTCTCTC |
| U10439 | 3171 | C | 0.95 | T | 0.05 | 0.1 | CACCAAGGTGGAGAAYGGAGAAGGCACAATC |
| U10439 | 3298 | C | 0.72 | A | 0.28 | 0.4 | AACGTGCTGGGCCTGMAAGGGGCACTGTTGA |
| U10439 | 3557 | A | 0.95 | G | 0.05 | 0.1 | TGGCTGATGGCTATGRCCTGGAGATCCTGGA |
| U10439 | 3640 | C | 0.67 | T | 0.33 | 0.44 | AAAAAGAACATTTTTYTTCTATTTAAGAAGC |
| U10439 | 4320 | C | 0.95 | T | 0.05 | 0.1 | TTCTTTCCTTGTGATYTGAATGTCTCCTTTT |
| U10439 | 4353 | G | 0.93 | A | 0.07 | 0.13 | CCCTCAGAGGGCAAARAGGTGAACATAAAGG |
| U10439 | 4454 | C | 0.53 | T | 0.47 | 0.5 | GCCTGCAGATAATGCYCAGCCATCCTCCCAT |
| U10439 | 4792 | T | 0.79 | C | 0.21 | 0.33 | TCTCTGCTCAGATAAYCATGACTTAGCAAGA |

| Sequence | Score 1 | Score 2 | Base | Score 3 | Base | Position | Accession |
|---|---|---|---|---|---|---|---|
| CGTCACTGTTCCACCYGGTGTAATATCTCTC | 0.47 | 0.61 | T | 0.39 | C | 4851 | U10439 |
| GCAGACCTGATCTTTMTAGGGTGACATAGA | 0.41 | 0.29 | C | 0.71 | A | 5077 | U10439 |
| CTGTCAGTGCTTGAARCTGTTCCTTTACTC | 0.5 | 0.48 | G | 0.52 | A | 5274 | U10439 |
| TTGGTGCCGTGGTGAWGGCTGCAGTCCAGTT | 0.4 | 0.27 | A | 0.73 | T | 5384 | U10439 |
| CTTTGCTTTATTCTRTGCTCTCTGTACTTT | 0.44 | 0.68 | G | 0.32 | A | 5625 | U10439 |
| TTGTCAGTGCTTGAARCTGTTCCTTTACTC | 0.48 | 0.39 | A | 0.61 | G | 6359 | U10439 |
| CTGCAGTCCAGTTTTRTGATTCTGCTTTTAT | 0.49 | 0.56 | C | 0.44 | A | 6490 | U10439 |
| CACGTTCCGGGCCGCSACCCTGACCCTGCAG | 0.26 | 0.15 | T | 0.85 | G | 81 | U57342 |
| AGGGCCCCGGGCCCTYAGCCTCTCTTGTACA | 0.1 | 0.05 | T | 0.95 | C | 940 | U57342 |
| TTTCCTCTCGATTCCYATCCCAATTTAATA | 0.18 | 0.1 | T | 0.9 | C | 1007 | U57342 |
| TACCATTGATGACTTYGCCTCTGCATCTACT | 0.1 | 0.05 | T | 0.95 | C | 1140 | U57342 |
| GCCCCCTCCATCCCTYAGCTACCCTGGATCT | 0.1 | 0.05 | T | 0.95 | C | 1283 | U57342 |
| GGAGCAGACTGGTTCYTAACTCTCTCCCTCC | 0.2 | 0.11 | G | 0.89 | A | 1375 | U57342 |
| AATTGGGATGCAGCCRRTGGAAGACCTTACAC | 0.15 | 0.08 | T | 0.92 | C | 577 | U62962 |
| GGGCAACTCAAGATTYTGGCTTCTACTGAAG | 0.28 | 0.17 | T | 0.83 | C | 1346 | U62962 |
| TTTCTACAGAAACTAYGATGTCATGAAAGAT | 0.43 | 0.32 | T | 0.68 | C | 167 | X13238 |
| CATCTGGATCCTCATYGACAAGACCAGCTTC | 0.18 | 0.1 | C | 0.9 | A | 176 | X14046 |
| ACAGATCACCCTGGGMATCCTCATCTCCACT | 0.2 | 0.11 | C | 0.89 | G | 377 | X14046 |
| TGGCACTACCCGCAGSACTGGTTCCAAGTCC | 0.44 | 0.33 | T | 0.67 | C | 540 | X14046 |
| CCCGCAGGACTGGTTYCAAGTCCTCATCCTG | 0.4 | 0.28 | T | 0.72 | A | 548 | X14046 |
| TCACGTGCGCTGGGCWCTTCCCTGCTGCCTG | 0.5 | 0.53 | T | 0.47 | A | 958 | X14046 |
| AGGAAGGAAGAGGTCMTCTGCTCGGGAGCTT | 0.32 | 0.2 | C | 0.8 | G | 220 | X57346 |
| TGCAGCCCTCCACGSCAGAGTCTCCAGAGA | 0.32 | 0.2 | C | 0.8 | C | 274 | X57346 |
| TGAGCAGGCTGAGCGMTATGATGATATGGCT | 0.42 | 0.7 | A | 0.3 | C | 431 | X57346 |
| GCATGAACTCTCCAASGAAGAGAGAAATCTG | 0.18 | 0.1 | G | 0.9 | C | 491 | X57346 |
| TCGTGCTTTGTGATCYGTCCAGTGTCACTCT | 0.32 | 0.2 | C | 0.8 | T | 1134 | X57346 |
| CTCCAAGGGTATGAYCACAAAGCAAGCAAA | 0.42 | 0.7 | G | 0.3 | T | 1103 | X74008 |
| AGGTGGTATGGTTGTRTACACATGCTGCCTT | 0.22 | 0.13 | T | 0.88 | A | 1845 | X74008 |
| ACAGCTCCATGACCAWCAGTACCGCGGCCAG | 0.18 | 0.1 | C | 0.9 | A | 350 | Z29505 |
| ATCAAAGAGATCCGCSAGAGTACGGGGGCGC | 0.42 | 0.3 | G | 0.7 | G | 457 | Z29505 |
| TGGGCAGGTGTCTGTRCTGGGTGATGTGTCAC | 0.33 | 0.79 | A | 0.21 | A | 515 | Z37166 |
| TTTCAGATCAGCAAGRAATATGAGCGCTTCT | 0.32 | 0.2 | C | 0.8 | G | 561 | Z37166 |
| TCTCCCTCTGCACAASTTACCTTTGTGTGTC | 0.2 | 0.11 | A | 0.89 | G | 181 | AC002045 |
| GGTGGAAACCAAAGTWGGAGCTAAAATCTGT | 0.1 | 0.06 | A | 0.94 | T | 350 | AC002045 |
| GTGGAAACCAAAGTTMGAGCTAAAATCTGTA | 0.49 | 0.44 | A | 0.56 | C | 351 | AC002045 |

| | | | | | | | |
|---|---|---|---|---|---|---|---|
| D26599 | 498 | T | 0.5 | A | 0.5 | 0.5 | CACGTGAGAGGGCAGWGGAACTCCTTAGGAA |
| D30655 | 1547 | A | 0.83 | G | 0.17 | 0.28 | GTTCTCTATCATTTARTAATATACTTGTGGA |
| L08096 | 237 | T | 0.73 | C | 0.27 | 0.4 | CCATTGGTCGCGGGCYTGGTGATCTGCCTCG |
| L08096 | 346 | G | 0.86 | C | 0.14 | 0.24 | AGCTGAATCACACAGSACCTCAGCAGGACCC |
| L08096 | 419 | G | 0.75 | A | 0.25 | 0.38 | CCTGCATGGACCAGARCTGGACAAGGGGCAG |
| L08096 | 420 | C | 0.92 | A | 0.08 | 0.15 | CTGCATGGACCAGAGMTGGACAAGGGGCAGC |
| L08096 | 421 | T | 0.38 | A | 0.62 | 0.47 | TGCATGGACCAGAGCWGGACAAGGGGCAGCT |
| L08096 | 472 | T | 0.75 | G | 0.25 | 0.38 | TCTACATGGTACACAKCCAGGTGACGCTGGC |
| L08096 | 483 | C | 0.84 | T | 0.16 | 0.27 | CACATCCAGGTGACGYTGGCCATCTGCTCCT |
| L08096 | 494 | C | 0.65 | T | 0.35 | 0.46 | GACGCTGGCCATCTGYTCCTCCACGACGGCC |
| L08096 | 536 | C | 0.88 | T | 0.13 | 0.22 | CCCCACCACCCTGGCYGTGGGAATCTGCTCT |
| L08096 | 575 | G | 0.88 | C | 0.13 | 0.22 | CCGTAGCATCAGCCTSCTGCGTCTCAGCTTC |
| L08096 | 578 | G | 0.89 | C | 0.11 | 0.2 | TAGCATCAGCCTGCTSCGTCTCAGCTTCCAC |
| L11066 | 409 | G | 0.89 | A | 0.11 | 0.2 | GAATGCCGGCCAAGCRACAGGCTGTCACCAA |
| L11066 | 472 | A | 0.84 | G | 0.16 | 0.27 | TCATTGGCCGGCGATRTGATGATCCTGAAGT |
| L11066 | 606 | A | 0.83 | G | 0.17 | 0.28 | GCATTGTGTTGATGRAGATGAAAGAGACTG |
| L11066 | 694 | G | 0.89 | C | 0.11 | 0.2 | TCAATGACTCGCAGASACAGGCCACTAAAGA |
| L11066 | 863 | G | 0.9 | A | 0.1 | 0.18 | TATCCTGGAAATTCARAAGGAGTATTTGAG |
| L11066 | 1037 | A | 0.57 | G | 0.43 | 0.49 | TGCTGAAAAGGCTAARTGTGAACTCTCCTCA |
| L11066 | 1165 | T | 0.94 | C | 0.06 | 0.1 | TTGTCACTGATCTAAYCAGAAGGACTATCGC |
| L11066 | 1382 | T | 0.95 | G | 0.05 | 0.1 | TGTGTTGGCCGGCGAKGTCACGGATGTGCTG |
| L11066 | 1472 | T | 0.85 | C | 0.15 | 0.26 | ACTTATTAATAGGAAYACCACTATTCCAACC |
| L11066 | 1514 | C | 0.83 | T | 0.17 | 0.28 | GGTATTCTCTACTGCYGCTGATGGTCAAACG |
| L11066 | 2022 | T | 0.62 | C | 0.38 | 0.47 | CTTCAGCAGGCATCAYTGAAGCTGTTCGAAA |
| L11066 | 2412 | A | 0.89 | G | 0.11 | 0.2 | TATAAAGATTTAACTRTTTTATGCTGAAGTG |
| L11066 | 2746 | G | 0.94 | C | 0.06 | 0.12 | GCTACCTTCTGCCCTSTGTCTGGCACCTACA |
| U05259 | 548 | C | 0.85 | G | 0.15 | 0.26 | GAAACGATGGCAGAASGAGAAGCTCGGGTTG |
| U05259 | 625 | C | 0.95 | T | 0.05 | 0.1 | ACCTGGACGACTGCTYCATGTATGAGGACAT |
| U05259 | 695 | T | 0.85 | A | 0.15 | 0.26 | CCTCAACATAGGAGAWGTCCAGCTGGAGAAG |
| U44839 | 1927 | G | 0.94 | A | 0.06 | 0.1 | GGGCCCAGCTCTGGARTCACGAACAGGTGCC |
| U44839 | 2169 | C | 0.89 | T | 0.11 | 0.2 | GAAGCATGACTGCGTYGGGTACGTGATGAAG |
| U44839 | 2586 | C | 0.94 | A | 0.06 | 0.1 | TGATGACAACAGCGTMTCCCCTGTCAATGAG |
| U44839 | 2658 | G | 0.83 | A | 0.17 | 0.28 | ACGCCAGGACGTGGCRCGACGCCTGCTGTCC |
| U46025 | 1385 | T | 0.89 | G | 0.11 | 0.19 | AATAATGCAAAATACKGACCCTCACTCCCAA |
| U46025 | 1507 | A | 0.95 | T | 0.05 | 0.1 | AGGTCTGCCGCATCTWCCTGCTGCGCATCCT |

| Sequence | | | | | | Position | Accession |
|---|---|---|---|---|---|---|---|
| TGAGGGCTCCTCAAASTCTGAGCAAGACCAG | 0.1 | 0.05 | C | 0.95 | G | 1592 | U46025 |
| AGGGCGAGGACTCGGYTGTGTTGATGGAGAG | 0.1 | 0.05 | T | 0.95 | C | 1633 | U46025 |
| GAGGACTCGGCTGTGWTGATGGAGAGACTGT | 0.1 | 0.05 | A | 0.95 | T | 1638 | U46025 |
| CCTCTGCCACATCTAYCACCATGCTCTGCAC | 0.1 | 0.05 | T | 0.95 | C | 1721 | U46025 |
| TATCATCAATGAGAAARATGAATGGGAAAGTG | 0.1 | 0.06 | A | 0.94 | G | 2279 | U46025 |
| ACTGCCCAGCAGAACMTGGCTCTGCAGCTGG | 0.32 | 0.2 | A | 0.8 | C | 2562 | U46025 |
| GCTAGCAAACCCTTCYGACGGCCCTCGCTGC | 0.5 | 0.5 | T | 0.5 | C | 120 | U70439 |
| CTGGAGCTGAGGAACYGGACCCCGGCAGCTG | 0.43 | 0.31 | T | 0.69 | C | 249 | U70439 |
| GAACTTAGAGTTCCTYAGTTAATAAATGTA | 0.29 | 0.18 | T | 0.82 | C | 350 | U70439 |
| CTCAGTTCAAATCTYCCCAAGCTGCCTAAA | 0.47 | 0.38 | T | 0.63 | C | 389 | U70439 |
| CATGTTAGCTGAAAARCTTCCAAATCTCACA | 0.22 | 0.13 | G | 0.88 | A | 467 | U70439 |
| TTCCAAATCTCACACRTCTAAACTTAAGTGG | 0.29 | 0.18 | G | 0.82 | A | 484 | U70439 |
| GGAACCTTTGAAAAARTTAGAATGTCTGAAA | 0.35 | 0.22 | A | 0.78 | G | 542 | U70439 |
| CCCCAGCTTACCTACWTGGATGGCTATGACC | 0.26 | 0.15 | A | 0.85 | T | 642 | U70439 |
| TGGATGGCTATGACCRAGAGGACCAGGAAGC | 0.29 | 0.17 | A | 0.83 | G | 658 | U70439 |
| GGGTCTTCTGATGATYCCAGCAGGGATCTAT | 0.19 | 0.11 | T | 0.89 | C | 305 | X07203 |
| TCATTTTCTCCATCARCAACCAGGAGAGACTG | 0.43 | 0.32 | A | 0.68 | G | 1314 | X07203 |
| GCAGGAGTGGACTTCARTCATCCCAGCAATCG | 0.19 | 0.11 | A | 0.89 | G | 270 | X15822 |
| GTACGGAGTTGAGAGRGACAGGGCAGACAAG | 0.4 | 0.28 | A | 0.72 | G | 425 | X16663 |
| AGAAGTGGAGAAGCAYACATCTCAGAAAGAT | 0.39 | 0.74 | C | 0.26 | T | 482 | X16663 |
| GACGCCCATAGAAGCYGCTTCTAGTGGTGCC | 0.35 | 0.22 | T | 0.78 | C | 731 | X16663 |
| GCCGCTTCTAGTGGTRCCCGTGGGCTGAAGG | 0.42 | 0.29 | A | 0.71 | G | 744 | X16663 |
| GAAGAGCCAGTGTACRAAGCAGAGCCTGAGC | 0.29 | 0.18 | A | 0.82 | G | 1122 | X16663 |
| GAGATGGACAGGCATRAGCAGGAGGATGAAC | 0.12 | 0.06 | A | 0.94 | G | 1203 | X16663 |
| GATCATCAGCGTGCCYGGCTGGGGCTGGGGC | 0.1 | 0.06 | T | 0.94 | C | 1297 | X16663 |
| ATCTCAGCTGTGGCTSTATATGATTACCAAG | 0.12 | 0.06 | G | 0.94 | C | 1347 | X16663 |
| CTTCCCTGCAAATTAYGTCAAGCTTCTGGAG | 0.1 | 0.06 | C | 0.94 | T | 1484 | X16663 |
| ACTGTGATTTCCCATKTCCAAAGTGGCTCTG | 0.1 | 0.06 | T | 0.94 | G | 1540 | X16663 |
| CCCTCCTATTCCTGMTCAAATGTCTAACC | 0.39 | 0.74 | C | 0.26 | A | 1578 | X16663 |
| GTCTAACCAGATGAGKTTCTGGACAGACTTC | 0.39 | 0.74 | G | 0.26 | T | 1601 | X16663 |
| CTCCTTCCCAATGAAYACCTCTCTGCCACCC | 0.1 | 0.06 | T | 0.94 | C | 1747 | X16663 |
| GAAGACTGAGCTGATSCAGAAGGCCAAGCTG | 0.12 | 0.06 | G | 0.94 | A | 145 | X56468 |
| AGACTGAGCTGATCCWGAAGGCCAAGCTGGC | 0.43 | 0.31 | T | 0.69 | C | 147 | X56468 |
| GGCCGAGCGGCTACGAYGACATGGCCACCTGC | 0.33 | 0.21 | T | 0.79 | C | 184 | X56468 |
| GGTCATCTCTAGCATYGAGCAGAAGACCGAC | 0.22 | 0.13 | T | 0.88 | C | 319 | X56468 |

| X56468 | 331 | C | 0.63 | T | 0.38 | 0.47 | CATCGAGCAGAAGACYGACACCTCCGACAAG | |
|---|---|---|---|---|---|---|---|---|
| X56468 | 721 | C | 0.94 | G | 0.06 | 0.1 | GGCTTTTGATGAGGCSATTGCTGAACTTGAT | |
| X56468 | 755 | T | 0.89 | G | 0.11 | 0.2 | CTGAATGAAGACTCAKACAAAGACAGCACCC | |
| X56468 | 781 | G | 0.71 | A | 0.29 | 0.41 | CACCCTCATCATGCARTTGCTTAGAGACAAC | |
| X56468 | 865 | C | 0.58 | A | 0.42 | 0.49 | GGCTGAAAACTAAATMCATACAGGGTGTCAT | |
| X56468 | 879 | A | 0.73 | C | 0.27 | 0.39 | TCCATACAGGGTGTCMTCCTTCTTTCCTTCA | |
| X56468 | 1165 | C | 0.72 | A | 0.28 | 0.4 | TTTAAGTAATCTGAAMCAGTTCTGCAAGTGA | |
| X56468 | 1676 | A | 0.83 | G | 0.17 | 0.28 | AAATTAAAGTACACARGTCCAAGTCTAAAAC | |
| X57351 | 34 | T | 0.3 | C | 0.7 | 0.42 | AGGACCTCCACACCAYTAACAAGATGAGCCT | |
| X57351 | 186 | C | 0.9 | G | 0.1 | 0.18 | TGCATTTGACAAATGSCAGGAAGAGGAAACT | |
| X57351 | 318 | G | 0.88 | A | 0.13 | 0.22 | TCTCCTGTCAACAGCRGCCAGCCTCCCAACT | |
| X57351 | 512 | C | 0.79 | G | 0.21 | 0.33 | CTTCATAGCATTCGCSTACTCCGTGAAGTCT | |
| X57351 | 635 | G | 0.63 | C | 0.38 | 0.47 | CTTCATGACCATTCTSCTCGTCATCATCCCA | |
| X57351 | 639 | G | 0.67 | A | 0.33 | 0.44 | ATGACCATTCTGCTCRTCATCATCCCAGTGT | |
| X59892 | 135 | C | 0.94 | T | 0.06 | 0.1 | AGTGAGCCCGCATCTYTGCTGGAGCTGTTCA | |
| X59892 | 270 | G | 0.89 | T | 0.11 | 0.2 | AGCTACAAAGCTGCCKCGGGGGAGGATTACA | |
| X59892 | 1113 | T | 0.72 | C | 0.28 | 0.41 | CCTAAACCAGCCCTGYTGCACTCCACCTTCT | |
| X59892 | 2051 | G | 0.94 | T | 0.06 | 0.1 | AATTGATAAGTAAAAKCTTCGTTATACATTT | |
| X59892 | 2159 | A | 0.93 | G | 0.07 | 0.13 | AGACTTGGGGGTGAARCTTTCCAGTTTACTG | |
| X59932 | 175 | A | 0.56 | T | 0.44 | 0.49 | TCCGGTACAGAATGTWTTGCCAAGTACAACT | |
| X59932 | 204 | C | 0.94 | T | 0.06 | 0.12 | CTTCCACGGCACTGCYGAGCAGGACCTGCCC | |
| X59932 | 891 | C | 0.88 | T | 0.12 | 0.21 | GGTGCAGCTCCTGGGYGTGATCGTGGAGGAG | |
| X59932 | 973 | A | 0.91 | C | 0.09 | 0.16 | GACTACCTGCGGTCTMGGGGTCGGTCAGTGC | |
| X59932 | 1411 | G | 0.94 | A | 0.06 | 0.1 | TGCTGGCACCTGGACRCCGCCATGCGGCCCT | |
| X59932 | 1563 | G | 0.5 | A | 0.5 | 0.5 | TGCCCCTGCTCACTGRGCCCGAGCCTGAACT | |
| X59932 | 1704 | G | 0.94 | A | 0.06 | 0.12 | GAGGAAGGAGGCCACRGAGCGGGAGGCAGCG | |
| X59932 | 1788 | T | 0.67 | G | 0.33 | 0.44 | ATTCCTTTCCTTTTTKGAGATTTTTTTTCCG | |
| X59932 | 2114 | T | 0.41 | C | 0.59 | 0.48 | CTTGTGAGATGGAATYGTAATAAACCACGCC | |
| Z47055 | 146 | T | 0.88 | C | 0.12 | 0.21 | TGGAGTACAATGCCAYTGGAGGCAAGTATAA | |
| STSG-13126 | 33 | T | 0.88 | C | 0.13 | 0.22 | TCCGGCCCTATCGTCYTTCCTGTGGGCCATT | GACAATGGTGG |
| STSG-13353 | 52 | T | 0.86 | C | 0.14 | 0.24 | GAAATAGGACCAATCYGAGGCTTTCGTGAGA | AACCGCAAGTT |
| STSG-13369 | 63 | G | 0.93 | C | 0.07 | 0.13 | GTGCTTTGGTATATASAGGTAACAATGTACT | ATTCATTTTCTC |
| STSG-13448 | 40 | T | 0.69 | C | 0.31 | 0.43 | ATGACAGATTCAAGAYGATGTCGCCTCCTGT | TGTGTTCTCCG |
| D00761 | 42 | C | 0.6 | G | 0.4 | 0.48 | GCCATGTATTCGGCTSCTGGCAGAGACTTGG | |
| D00761 | 514 | C | 0.94 | G | 0.06 | 0.1 | TCAAGGCTGGAGGCTSAGCAAGTGCCATGCT | |

| | | | | | | | |
|---|---|---|---|---|---|---|---|
| D28137 | 251 | T | 0.95 | C | 0.05 | 0.1 | GGCCCAGAAGGGCTTYCAGGATGTGGAGGCC |
| D43950 | 291 | T | 0.78 | C | 0.22 | 0.35 | TTGCCAAGCTGATGGYGGAACTGTCCAAGTC |
| D43950 | 453 | A | 0.95 | T | 0.05 | 0.09 | CTCGTGTTGCTATTGWACACCTGGACAAGAT |
| D43950 | 1102 | C | 0.95 | G | 0.05 | 0.09 | TCTTGTACAGGAGATSTCATTTGGGACAACT |
| D43950 | 1823 | C | 0.16 | T | 0.84 | 0.27 | TAATCGTGGGGGTACYATCTCAACTGCTTTT |
| HT3200 | 432 | C | 0.51 | T | 0.49 | 0.5 | ACGCCATCCGCGAGGYCCTGGAGCTGGACTC |
| J03077 | 34 | T | 0.22 | C | 0.78 | 0.35 | TGCGGAGTCAGACGGYGCTATGTACGCCCTC |
| J03077 | 2509 | G | 0.53 | A | 0.48 | 0.5 | ATAAATATGGATGGCRAGCTCCTAGGCCTCT |
| L08666 | 496 | T | 0.83 | C | 0.17 | 0.28 | AAAGTTACTGGGACCYTGGAGACCAAATACA |
| L08666 | 1265 | C | 0.12 | A | 0.88 | 0.21 | AGGCTTTTTTCCCCCMAGAAGATGATCAAAA |
| L08666 | 1393 | A | 0.94 | G | 0.06 | 0.1 | TGCAGTCACCTATACRTTATTTAAATGTATT |
| L22009 | 662 | A | 0.95 | G | 0.05 | 0.09 | AACTCATTATGATCCRCCACGAAAGCTTATG |
| L22009 | 1653 | C | 0.95 | T | 0.05 | 0.09 | TGATTTATGCTTACTYTAAGTGGAAATCAGG |
| L22009 | 1790 | G | 0.94 | T | 0.06 | 0.1 | CTCAAGTTAGTTATAKGATGTACTTAAGCAG |
| L22009 | 1796 | A | 0.83 | T | 0.17 | 0.28 | TTAGTTATAGGATGTWCTTAAGCAGTAAGCG |
| L22009 | 2033 | A | 0.95 | C | 0.05 | 0.09 | CTTTTTTTTTTTTTAMGTTAATGGGAAAAAT |
| L22009 | 2098 | A | 0.95 | G | 0.05 | 0.09 | TTGGTGTTGTATGARATTCTGAGGCCTTGA |
| L28010 | 1437 | T | 0.92 | C | 0.08 | 0.15 | TTCTCATGCAAAATTYTCTTCTAGCATGTGA |
| L38951 | 1968 | C | 0.13 | T | 0.88 | 0.22 | TGCCAAGGATTGTTAYCCTGCTGTCCAGAAA |
| L38951 | 3310 | A | 0.85 | G | 0.15 | 0.26 | TTCTGTCTTTTGGCCRGTGCCGAGTGGAATG |
| L38951 | 3390 | G | 0.78 | A | 0.22 | 0.35 | GTAAAAGGCAGCCTGRCCTTTGCTACTGAGG |
| L38951 | 3457 | T | 0.85 | C | 0.15 | 0.26 | TGTACCTTTGCCACAYGGTACTGTATGCTTG |
| L38951 | 3470 | T | 0.88 | C | 0.13 | 0.22 | CATGGTACTGTATGCYTGCCAGCTAGAAGGA |
| L38951 | 3475 | A | 0.83 | G | 0.17 | 0.29 | TACTGTATGCTTGCCRGCTAGAAGGAGGGTC |
| L38951 | 3550 | C | 0.83 | G | 0.17 | 0.28 | TATCCACATTCTCAASTTCAAGTCATTGCTG |
| L38951 | 3635 | A | 0.88 | G | 0.13 | 0.22 | AAGTTCTGTCTACTGRTGCAGCACAAGAGAT |
| L38951 | 3948 | A | 0.78 | G | 0.22 | 0.35 | CAATTGTTACAAATARCGCAGACTTCAAAAA |
| L38951 | 3996 | T | 0.89 | G | 0.11 | 0.2 | AAACAAACCAAAATTKAAATGATCAGAATTG |
| L38951 | 4081 | A | 0.9 | G | 0.1 | 0.18 | CCAGAAAATTGTGCCRAAGAGTTTAGAAAAA |
| L38951 | 4097 | T | 0.83 | C | 0.17 | 0.28 | AAGAGTTTAGAAAAAYAAATATACAATAAAA |
| M22490 | 749 | G | 0.9 | C | 0.1 | 0.18 | CCAACACCGTGAGGASCTTCCACCACGAAGA |
| M22632 | 208 | C | 0.94 | T | 0.06 | 0.1 | GGGAGTTGGTGCCTAYCGGGATGATAATGGA |
| M22632 | 220 | T | 0.33 | C | 0.67 | 0.44 | CTACCGGGATGATAAYGGAAAGCCTTACGTT |
| M22632 | 235 | T | 0.35 | G | 0.65 | 0.45 | TGGAAAGCCTTACGTKCTGCCTAGCGTCCGC |
| M22632 | 672 | G | 0.94 | A | 0.06 | 0.1 | CGGGAGTGGACCCGCRTCCGGAACAGTGGAA |

EP 1 024 200 A2

| Sequence | | | | | | | Accession |
|---|---|---|---|---|---|---|---|
| GGGCATTAATGTTGYCTCTGCCAATCATAT | 0.44 | 0.67 | T | 0.33 | C | 823 | M22632 |
| TGCAAGAAGTGAAAGKCATGGCTGACCGCAT | 0.4 | 0.27 | T | 0.73 | G | 1044 | M22632 |
| TCAGCCACAGTGTGTRACACTCAGCATTTGA | 0.15 | 0.08 | G | 0.92 | A | 1426 | M22632 |
| CCTTATCAACAGGCCRCACTGCAGAAATGAT | 0.09 | 0.05 | A | 0.95 | G | 1758 | M22632 |
| TTTTTCTGCTGATGCYGTACCCTCACCCTTT | 0.45 | 0.66 | T | 0.34 | C | 2008 | M22632 |
| ATGTGGTGTGCAGCCWCTCATCTCACACTGT | 0.15 | 0.08 | A | 0.92 | C | 2160 | M22632 |
| CGGCGCAGGGTGGTASCAGGAAAGAGGATAT | 0.32 | 0.2 | G | 0.8 | C | 2199 | M22632 |
| GCTGGGGCTACAGCAYTTACGCAGCCGCACG | 0.09 | 0.05 | T | 0.95 | C | 494 | M57567 |
| TGATGCCCGGAAGCTSCTGCCGTGCATCCCG | 0.2 | 0.11 | G | 0.89 | C | 631 | M57567 |
| GAATCTGAAGATGAGYTACTTGTAGATGAAG | 0.28 | 0.17 | T | 0.83 | C | 689 | M63483 |
| CCAACAAAGATACAASTGAAAAACGCAGATGG | 0.22 | 0.13 | C | 0.88 | G | 993 | M63483 |
| AGATACAAGTGAAAAYGCAGATGGTCAAAGT | 0.09 | 0.05 | T | 0.95 | C | 1000 | M63483 |
| TGATACAGCTAAGACYATGTTCCGGATTCCC | 0.09 | 0.05 | T | 0.95 | C | 141 | M87503 |
| AGCGAGGTCACCTTRCATTGCACTCAGCAT | 0.1 | 0.05 | A | 0.95 | G | 315 | M95627 |
| CTGCTTACTGACTACYGGGGCCACACGGCTG | 0.09 | 0.05 | T | 0.95 | C | 1242 | M95627 |
| GCCCTCCCACCCTTGYCCAGACCTGGTGGGC | 0.1 | 0.05 | T | 0.95 | C | 1554 | M95627 |
| CCAGGCGAAACCCAGYCTGGAGCTTGCAGGC | 0.09 | 0.05 | T | 0.95 | C | 105 | U12595 |
| CTGCCAGAAATGGAGRTTCACTTGCAGACCA | 0.09 | 0.05 | G | 0.95 | A | 383 | U12595 |
| AGAACCGGGACAAAARTCATCATCCACCTGA | 0.09 | 0.05 | C | 0.95 | A | 713 | U12595 |
| GACCCCAAGGATGTCSGTGAGTGGCAACATG | 0.5 | 0.5 | T | 0.5 | G | 875 | U12595 |
| CCGCAGCATCTTCTAYGTGCCCGACATGAAA | 0.17 | 0.09 | T | 0.91 | C | 988 | U12595 |
| GGTGGACAGTGAGGASATTCCCCTGAACCTC | 0.24 | 0.14 | G | 0.86 | C | 1141 | U12595 |
| TGCTGGACTTGTTGAYGACCCTAGGGCCATG | 0.1 | 0.05 | T | 0.95 | C | 2007 | U12595 |
| GGGCCATGGTGGGCCRCTTGAATGAGCTGCT | 0.13 | 0.07 | A | 0.93 | G | 2030 | U12595 |
| CTAGGGCTGGGTATYCCCACATCACTCATT | 0.09 | 0.05 | T | 0.95 | C | 65 | U15085 |
| GCCCATGGGAAAGCRCCTGTCTGTTGGATG | 0.1 | 0.06 | G | 0.94 | A | 314 | U15085 |
| TGGAAAGCACCTGKGTTGGATGATGCTGG | 0.09 | 0.05 | G | 0.95 | T | 321 | U15085 |
| TCTCCTTCAACAAGGWTCTGCTGACCTGCTG | 0.1 | 0.06 | T | 0.94 | A | 378 | U15085 |
| TTGGGGTGCTGAATARCTTGGGGAATGTCCT | 0.1 | 0.06 | A | 0.94 | G | 444 | U15085 |
| TGCCTCACAGCAGTGMGCACAAGACTGCCCA | 0.17 | 0.09 | C | 0.91 | C | 717 | U15085 |
| GTGTGGTAGAGCACAYTGGGGCTACCATGCC | 0.42 | 0.3 | C | 0.7 | T | 822 | U15085 |
| TCTTCTCTCTTGGTGYGATCAGCTGGGCGAG | 0.09 | 0.05 | C | 0.95 | T | 942 | U15085 |
| ATTCAAACTTAGCAAYTTGGGGGACCTCATT | 0.09 | 0.05 | C | 0.95 | T | 1079 | U15085 |
| TGTAAAACTTAGCAAYTTGGGGGACCTCATT | 0.25 | 0.15 | T | 0.85 | C | 1189 | U15085 |
| TCAGTGTGCCATAGARGACAGCAACTGGTGA | 0.1 | 0.05 | A | 0.95 | G | 1314 | U15085 |

| | | | | | | | |
|---|---|---|---|---|---|---|---|
| U28386 | 219 | C | 0.94 | T | 0.06 | 0.1 | ACAGAAATGAGGCGTYGCAGAATAGAGGTCA |
| U28386 | 625 | C | 0.95 | G | 0.05 | 0.1 | ATGGAGGTGCCATCCSAGCATTCATTTCTCT |
| U28386 | 787 | C | 0.95 | A | 0.05 | 0.09 | CAGTTCCTGATATGTMATCTTTAGCATGTGG |
| U28386 | 932 | G | 0.6 | A | 0.4 | 0.48 | TGATGATCCAGAAGTRTTAGCAGATACCTGC |
| U28386 | 1283 | T | 0.95 | C | 0.05 | 0.09 | CCCATTCCTTGTCAGYGTTCTCTCTAAGGCA |
| U28386 | 1493 | A | 0.94 | G | 0.06 | 0.1 | GGCTGCTGAGAAACTRGGTGAAACTGAGAAA |
| U28386 | 1549 | T | 0.95 | A | 0.05 | 0.09 | GAGGCTTAGACAAAAWTGAAGCTCTACAAAA |
| U30255 | 81 | G | 0.94 | C | 0.06 | 0.1 | AACATGAATGACCACSGCTTTGTGGTCTGTG |
| U30255 | 125 | C | 0.22 | T | 0.78 | 0.35 | TGTCTCCAAAGTTGAYGATTTCTTGGCCAAT |
| U30255 | 737 | C | 0.71 | T | 0.29 | 0.41 | TCTCAAGTTCCAAGAYACCGATGGCAAACAC |
| U30255 | 741 | G | 0.94 | A | 0.06 | 0.1 | AAGTTCCAAGACACCRATGGCAAACACCTGC |
| U30255 | 1442 | G | 0.92 | A | 0.08 | 0.15 | CACCGTGTCATCCTCRTCATACAATGCCTGA |
| U45328 | 260 | A | 0.86 | G | 0.14 | 0.24 | GGAGTGCGCCATTCCRGGAAAGAAAGGGACT |
| U45328 | 623 | G | 0.8 | A | 0.2 | 0.32 | CTTGTGGCATCGTCARAAGGAAGGGATTGGT |
| U45328 | 756 | C | 0.94 | G | 0.06 | 0.12 | GCGGGTGTGCGGTCTSGATTCGCTGAATTGC |
| U45328 | 761 | C | 0.93 | G | 0.08 | 0.14 | TGTGCGGTCTCGATTSGCTGAATTGCCCGTT |
| U51127 | 351 | G | 0.89 | A | 0.11 | 0.2 | GGATGAAGCCGATCCRGCCAAGTGGAAGGCC |
| U51127 | 1698 | A | 0.94 | T | 0.06 | 0.1 | ACAGCCCCGATGAGCWCCTGGCTGGCTGCAG |
| U51127 | 1745 | T | 0.83 | C | 0.17 | 0.28 | TTCCTGGAAGTGGATYTGGGCCAAGAAGGAG |
| U88964 | 35 | A | 0.56 | G | 0.44 | 0.49 | GAGGGTCCCCAAGGARCATGGCTGGGAGCCG |
| U88964 | 450 | A | 0.19 | G | 0.81 | 0.31 | GAGTGAGCGCCTCCTRCACAAGAGCATCCAG |
| X15729 | 481 | C | 0.94 | G | 0.06 | 0.1 | TTTCCCTGCAAATGTSATGGATGTTATTGCA |
| X15729 | 513 | A | 0.94 | C | 0.06 | 0.1 | GACAGAATTTCACTGMACCCACTGCTATTCA |
| X15729 | 546 | T | 0.89 | G | 0.11 | 0.2 | CTCAGGGATGGCCAGKTGCTCTAAGTGGATT |
| X15729 | 624 | T | 0.94 | C | 0.06 | 0.1 | ATTTGCTTCCTGCCAYTGTCCACATCAATCA |
| X15729 | 1127 | C | 0.94 | G | 0.06 | 0.1 | ATTGTGGATGTGTGTSATGACGTAGAAAAGG |
| X15729 | 1604 | T | 0.73 | G | 0.27 | 0.4 | GTCAAGACAGAGGTKCAGGTCGTTCCAGGG |
| X56253 | 240 | T | 0.72 | A | 0.28 | 0.4 | GCAGTGAGAGAATCCWGGCAGACAGAAGAAA |
| X75861 | 402 | A | 0.1 | G | 0.9 | 0.18 | TGCTTTCATGGGCACRGCAATGATCTTTACC |
| X75861 | 609 | A | 0.95 | G | 0.05 | 0.09 | CCTTGTTGATACTCARCTCATTATTGAAAAG |
| X75861 | 982 | A | 0.95 | G | 0.05 | 0.1 | CTCCCTTTTTTGTCARCCCCATCTGTAGCCT |
| X75861 | 1146 | T | 0.89 | C | 0.11 | 0.2 | CCCTTCCTTCCTCATYGTTGTTTGGTATGCG |
| X75861 | 1209 | T | 0.77 | G | 0.23 | 0.35 | CCATTGGAAAGAAGTKCAGTGGTCCCATTGT |
| D13900 | 29 | C | 0.95 | T | 0.05 | 0.09 | GAGAGCCATGGCCGCYCTGCGTGTCCTGCTG |
| D13900 | 52 | C | 0.63 | T | 0.38 | 0.47 | TCCTGCTGTCCTGCGYCCGCGGCCCGCTGAG |

EP 1 024 200 A2

| | | | | | | | |
|---|---|---|---|---|---|---|---|
| D13900 | 244 | T | 0.89 | C | 0.11 | 0.2 | ACCAGGCCCTGAAGAYCTTCGAGGAGGACCC |
| D13900 | 260 | G | 0.83 | A | 0.17 | 0.28 | CTTCGAGGAGGACCCRGCCGTTGGGGGCATT |
| D13900 | 602 | C | 0.94 | T | 0.06 | 0.1 | GGAGATGGTCCTCACYGGTGACGCGATCTCA |
| D13900 | 1008 | C | 0.9 | T | 0.1 | 0.17 | CCGTGGTGTGCAGTYCTCTCCAATTGCTGC |
| D13900 | 1175 | T | 0.73 | C | 0.27 | 0.4 | GGAAAGCGGGGCCTGYGGGTCCTTGTGTCCC |
| D14812 | 1300 | T | 0.43 | C | 0.57 | 0.49 | TGTTTCAGTTTGTTTYCCGTTCTGTTTTAAA |
| D14812 | 1465 | A | 0.91 | C | 0.09 | 0.17 | GTTCCTATCCCTTTGMCACTACACAATTTTC |
| D14812 | 1656 | G | 0.94 | C | 0.06 | 0.1 | AGGCATTCTATCTTTSCTCAAATTGTTGAAG |
| D28473 | 39 | T | 0.84 | A | 0.16 | 0.26 | GCTGGCCCGCGGGGCWGTCCAGGCACGCGAG |
| D28473 | 498 | T | 0.95 | G | 0.05 | 0.09 | AAGATTTGGATGGGAKTGCCATGGCTTACCT |
| D28473 | 1012 | T | 0.95 | G | 0.05 | 0.09 | GGACGATTACTCATTKTAATGGAAGCCAGAT |
| D28473 | 1719 | A | 0.94 | G | 0.06 | 0.1 | AGAACTGTCAGGAGCRAAGATCTCAGATCTC |
| D28473 | 2291 | C | 0.91 | T | 0.09 | 0.17 | TCTACAATGAGAACAYGGTTAGAGAAAGCCC |
| D28473 | 2904 | T | 0.95 | C | 0.05 | 0.09 | AGATAAAAACAAGTAYGGCATTCGGCTAAGG |
| D28473 | 3201 | T | 0.93 | G | 0.08 | 0.14 | AGATGAAGGAATGGCKCGGGAAGTCATCAAT |
| D28473 | 3296 | G | 0.94 | T | 0.06 | 0.1 | AAGCAAAGTCTGAAGKAACATATCTGAATAG |
| D28473 | 3376 | G | 0.95 | A | 0.05 | 0.09 | TTGAAACCATATCCARTTTCTCCATCGGATA |
| D28473 | 3784 | A | 0.84 | G | 0.16 | 0.27 | CAGCTCCTGAATGCARAGCCACAAGAGTGTT |
| D28473 | 4152 | T | 0.85 | C | 0.15 | 0.26 | GGTGTGTGTAAAATAYGCTGCTTGGATTGAA |
| D28473 | 4202 | A | 0.82 | T | 0.18 | 0.3 | GTCAAGTAACTTGAGWCCTCACAGTAATCTT |
| D32050 | 933 | G | 0.91 | A | 0.09 | 0.16 | TTCAGAAGGGCACAGRTGCCCGACCATACAC |
| D32050 | 1012 | T | 0.36 | C | 0.64 | 0.46 | GGTGCTGGCTGACCAYGCTCGGACCATCACT |
| D32050 | 1342 | T | 0.95 | C | 0.05 | 0.1 | TCCCGGAGACACTGCYTGGCTCCTCTATGAC |
| D32050 | 2483 | C | 0.95 | G | 0.05 | 0.1 | CCAAACAAGGATGTGSAGAGGGAGATCGCTG |
| D32050 | 2824 | C | 0.89 | T | 0.11 | 0.2 | GTGCCTGTGTCAAGTYCCCCAGAATGCAGCC |
| D32050 | 3267 | A | 0.94 | C | 0.06 | 0.1 | TCGCATCTATAGATAMCGGCTCTCCAGACCT |
| D78151 | 56 | G | 0.85 | A | 0.15 | 0.26 | GGGAGGCCGGGACAARGCGCCGGTGCAGCCC |
| D78151 | 638 | T | 0.72 | G | 0.28 | 0.4 | CCACAATGCAGAGCAKGAGGCTTGCGACCTG |
| D78151 | 971 | G | 0.91 | T | 0.09 | 0.17 | TGAAGATGTCGAGGAKTATGAGGACCTGACA |
| D78151 | 1165 | A | 0.83 | G | 0.17 | 0.28 | CCTCCTCTTTTGTGARTGGCTTTGTGAATGC |
| D78151 | 1411 | A | 0.83 | T | 0.17 | 0.28 | TCCGGAATGAGTGTGWCCCTGCTCTGGCACT |
| D78151 | 1441 | T | 0.94 | A | 0.06 | 0.1 | TGCTCTCAGACTATGWTCTCCACAACAGCAA |
| D78151 | 1811 | A | 0.95 | G | 0.05 | 0.09 | CAGTTTTGCCAACACRCTGGTGGATGTGTGT |
| D78151 | 2085 | A | 0.83 | G | 0.17 | 0.28 | AGATATGGGGAGCCTRCACTCCGGAGGGCTG |
| D78151 | 2265 | C | 0.83 | T | 0.17 | 0.28 | CGTCTGGCTGCAATGYTGCGCCAGTTAGCTC |

| | | | | | | | |
|---|---|---|---|---|---|---|---|
| D78151 | 2650 | T | 0.83 | C | 0.17 | 0.28 | ATACAACCCCAGTGTYGTTGGCCCACGGGGA |
| D78151 | 2784 | C | 0.67 | T | 0.33 | 0.44 | AGGGGCTCTGAACTGYAGCTGATGTTATCAG |
| D78151 | 2804 | C | 0.95 | A | 0.05 | 0.09 | GATGTTATCAGCAGGMCATGCATCCTGCTGC |
| J04611 | 169 | A | 0.83 | G | 0.17 | 0.28 | TGGTTGATGCCTCCARGGCTATGTTTGAATC |
| J04611 | 849 | A | 0.94 | G | 0.06 | 0.1 | GTGATCTCTGTGGGCRTTTATAATCTGGTCC |
| J04611 | 984 | A | 0.94 | C | 0.06 | 0.1 | CCTAGCGATACCAAGMGGTCTCAGATCTATG |
| J04611 | 1108 | A | 0.83 | G | 0.17 | 0.28 | TACTGCTGAAGAAACRCCATTACCTGAGGCC |
| J04611 | 1593 | T | 0.83 | C | 0.17 | 0.28 | AAAAGACTGGGCTCCYTGGTGGATGAGTTTA |
| J04611 | 1620 | T | 0.83 | C | 0.17 | 0.28 | TTTAAGGAGCTTGTTYACCCACCAGATTACA |
| J04611 | 1811 | T | 0.22 | G | 0.78 | 0.35 | CGGGCTGAAGAGTGGKCTGAAGAAGCAGGAG |
| L15189 | 506 | T | 0.88 | C | 0.13 | 0.22 | GAGGCTCATGGGAAAYTGTATTCTCCGAGTC |
| L15189 | 540 | T | 0.81 | C | 0.19 | 0.3 | TTGGAGCATTTGTGTYGATGAAGATGAAAGA |
| L15189 | 925 | T | 0.81 | C | 0.19 | 0.3 | AGGGGTTGATTTGACYAAAGACAACATGGCA |
| L15189 | 976 | A | 0.58 | G | 0.42 | 0.49 | TGCTGAAAAGGCTAARTGTGAACTCTCCTCA |
| L15189 | 1684 | A | 0.89 | G | 0.11 | 0.19 | CCAGTCTTCTGGTGGRTTAAGCAAAGATGAT |
| L15189 | 1880 | A | 0.89 | G | 0.11 | 0.19 | GAAGAGATTTCCAAARTGAGGGAGCTCCTGG |
| L15189 | 1961 | T | 0.64 | C | 0.36 | 0.46 | CTTCAGCAGGCATCAYTGAAGCTGTTCGAAA |
| M29877 | 31 | C | 0.86 | G | 0.14 | 0.24 | GGATGAGGTCGCGGCSGGCGGGTCCCGCGCT |
| M29877 | 329 | G | 0.88 | A | 0.13 | 0.22 | CGACTTCGGACCGCARTTCACTGCGCGCTTC |
| M29877 | 404 | G | 0.95 | C | 0.05 | 0.09 | CAAGTATGTAGTTTTSACGACAAAGCATCAC |
| M29877 | 439 | C | 0.95 | T | 0.05 | 0.09 | GCTTCACAAACTGGCYGAGTCCTGTGTCTTG |
| M29877 | 859 | A | 0.84 | G | 0.16 | 0.27 | ATAAATTCAAGCCACRGAGCTTGCCAGATCA |
| M97935 | 1750 | A | 0.93 | C | 0.07 | 0.13 | AAGCTTCTTGGTCCTMACGCCAGCCCCGATG |
| M97935 | 3357 | A | 0.95 | G | 0.05 | 0.1 | AGTCTATTAGCCACARAATTGGGAAAGGAGT |
| M97935 | 3814 | A | 0.92 | G | 0.08 | 0.15 | TGTATTGCATTAAATRTAATATCGACACAGT |
| U09587 | 740 | T | 0.94 | C | 0.06 | 0.1 | CCAGCTTGATAACTAYGGACAGCAAGAACTT |
| U09587 | 1055 | T | 0.95 | C | 0.05 | 0.09 | AGAAATTGAGCACTTYGTAGATCCCAGTGAG |
| U09587 | 1593 | C | 0.95 | G | 0.05 | 0.09 | GAAATGGAGATGCTGSTGAATGAGAAAGGGG |
| U09587 | 1605 | G | 0.94 | A | 0.06 | 0.1 | CTGCTGAATGAGAAARGGGAATTCACAATTG |
| U09587 | 1826 | T | 0.95 | C | 0.05 | 0.1 | TTTCCCTGCTGTAGTYGCTCCATTCAAATGT |
| U09587 | 1846 | T | 0.95 | C | 0.05 | 0.09 | CATTCAAATGTTCCGYCCTCCCACTGAGCCA |
| U09587 | 2138 | A | 0.93 | G | 0.07 | 0.13 | CAATGGCAACATCACRTGGGCTGATGTGGAG |
| U41371 | 84 | T | 0.33 | G | 0.67 | 0.44 | GAGCTTCAGGCCAAGKTGGCAGAGATCGGAG |
| U41371 | 1155 | T | 0.89 | G | 0.11 | 0.2 | AGGATCTTTGAGGCTKTTAAGCTCACTGATG |
| U54778 | 341 | G | 0.95 | A | 0.05 | 0.09 | TGAGACTGAGCTAAARTTAATCTGTTGTGAC |

| | | | | | | | |
|---|---|---|---|---|---|---|---|
| U54778 | 1104 | G | 0.88 | A | 0.13 | 0.22 | TTTTTCAGCATTACTRTGTCGTCTCCGTGCC |
| U54778 | 1178 | T | 0.91 | C | 0.09 | 0.16 | AAGCCATTAGACTTAYAGGTGATGCAAGCAT |
| U54778 | 1227 | A | 0.83 | G | 0.17 | 0.28 | ACACTATAGAGGCATRAGTGGTATCAGTTTT |
| U54778 | 1304 | T | 0.83 | C | 0.17 | 0.28 | TAATTGGGTGTTAGCYTGAGATGGTTAAAGG |
| U54778 | 1662 | G | 0.72 | T | 0.28 | 0.4 | ACAGATACAGAATCTKTATTGTTCTTACTGA |
| U57650 | 997 | A | 0.94 | G | 0.06 | 0.1 | GAGCCTCTCCGAGACRTTGTTCCAGCGACTG |
| U57650 | 1327 | A | 0.22 | G | 0.78 | 0.35 | GACAAGCCTGTTGTCRTCCATTGAAGACAAG |
| U57650 | 1549 | C | 0.38 | G | 0.63 | 0.47 | GAAAATCCTGCAGCTSATTAAGTCACAGAAA |
| U57650 | 3478 | G | 0.94 | A | 0.06 | 0.1 | TCCCAGGACACAGGARTCAAGGCCCAGTGAC |
| U57650 | 4013 | C | 0.77 | T | 0.23 | 0.36 | AACACCGAGCTCCCGYATCACGGCAAGCACC |
| U57650 | 4083 | C | 0.94 | T | 0.06 | 0.12 | CCATGCAGTGAAGCCYTCAGTGAGCTGCCAC |
| U57650 | 4324 | C | 0.88 | T | 0.12 | 0.21 | TCTGGGTCCCCAGCTYGCTCTTGGTACTTGG |
| U57650 | 4353 | C | 0.62 | T | 0.38 | 0.47 | GGGACCCCAGTGCCTYGTTGAGGGCGCCATT |
| U57650 | 4463 | A | 0.56 | G | 0.44 | 0.49 | GATCGAACACTCATGRTGTGCCAAGTGCTGT |
| U57650 | 4736 | C | 0.95 | T | 0.05 | 0.1 | GCTTTAGCTAAAGTCYCGCGGGTTCCGGCAT |
| U80040 | 211 | A | 0.5 | C | 0.5 | 0.5 | GAACCGGCCGCTGACMCTCTCGGAGAAGATT |
| U80040 | 689 | C | 0.68 | T | 0.32 | 0.44 | GGGATCCCCTGGGAGYTGAAGTGCCCCAAGG |
| U80040 | 880 | T | 0.85 | C | 0.15 | 0.26 | AATCTGCAACATGGGYGCAGAAATTGGGGCC |
| U80040 | 1875 | T | 0.83 | C | 0.17 | 0.28 | CCAACAACCTGCTCAYTGGTGCCATCAACAT |
| U80040 | 2686 | T | 0.91 | G | 0.09 | 0.17 | GATTCTAGAGAACCTKTTGTTCTTGCAAGGA |
| U83843 | 171 | G | 0.83 | C | 0.17 | 0.28 | ATTCTGAAACTTCTTSATGTTGTCCATCCTG |
| U83843 | 758 | A | 0.83 | G | 0.17 | 0.28 | TGAGGATTATCAGGCRATTGTTGATGCTGAG |
| U83843 | 770 | T | 0.89 | C | 0.11 | 0.2 | GGCAATTGTTGATGCYGAGTGGAACATTCTC |
| U83843 | 929 | A | 0.95 | G | 0.05 | 0.09 | GGATCTGAAGAGGACRATGATGGCCTGTGGA |
| U83843 | 1127 | C | 0.94 | T | 0.06 | 0.1 | GGAGACAGAGCGGTCYCTGCATGATGCCATC |
| U91932 | 71 | C | 0.09 | G | 0.91 | 0.16 | AGTGCCCACCCGGTCSGCCCGGCACAGCCAT |
| X54942 | 425 | T | 0.91 | C | 0.09 | 0.17 | TGAGAAATGTACAAAYCTTTCATCCATACCT |
| X54942 | 427 | T | 0.85 | C | 0.15 | 0.26 | AGAAATGTACAAATCYTTCATCCATACCTGT |
| X59417 | 54 | C | 0.79 | G | 0.21 | 0.33 | TTTAAAGTAGTGCTTSTACCAACATGTCCCG |
| X59417 | 516 | A | 0.94 | G | 0.06 | 0.1 | AAGGCCCTCAGGTATRTAAGTGTGATCCTGC |
| X59417 | 637 | A | 0.94 | G | 0.06 | 0.1 | TGATTGGACATTTGARCAGACAGTGGAAACT |
| X59417 | 739 | A | 0.94 | G | 0.06 | 0.1 | AGTTGAAAATCCTAARTTCAGGATTCTTACA |
| X64364 | 125 | C | 0.94 | T | 0.06 | 0.1 | AGCCTCCGGGGCTGCYGGCACAGTCTTCACT |
| X64364 | 290 | C | 0.82 | G | 0.18 | 0.29 | GTTCAAGGTGGACTCSGACGACCAGTGGGGA |
| X64364 | 383 | C | 0.25 | T | 0.75 | 0.38 | TCCCAGAGTGAAGGCYGTGAAGTCGTCAGAA |

| | | | | | | | |
|---|---|---|---|---|---|---|---|
| X64364 | 897 | G | 0.63 | A | 0.37 | 0.47 | GACGCTCCCTGCTCCRCGTCTGCGCCGCCGC |
| X64364 | 1390 | G | 0.92 | A | 0.08 | 0.15 | ACCGAGGGCGACCCCRTCACAGCCTCAAGTC |
| X64364 | 1540 | T | 0.69 | C | 0.31 | 0.43 | GGACGGCCGGCTCTCYATAGCACCAGGGCTC |

I. ANALYSIS of POLYMORPHISMS

A. PREPARATION of SAMPLES

Polymorphisms are detected in a target nucleic acid from the subject(s) being analyzed. Any suitable biological sample can be used for assay of genomic DNA. Convenient suitable tissue samples include whole blood, semen, saliva, tears, urine, fecal material, sweat, buccal, skin and hair. Pure red blood cells are not suitable. As those skilled in the art will appreciate, for assays of cDNA or mRNA, the tissue sample must be obtained from an organ in which the target nucleic acid is expressed, e.g., the liver for a target nucleic acid of a cytochrom P450.

Many known methods such as, for example those described below require amplification of the DNA from target samples. This can be accomplished by e.g., PCR. See, e.g., PCR Technology: Principles and Applications for DNA Amplification (ed. H.A. Erlich, Freeman Press, NY, NY, 1992); PCR Protocols: A Guide to Methods and Applications (eds. Innis, et al., Academic Press, San Diego, CA, 1990); Mattila et al., Nucleic Acids Res. 19, 4967 (1991); Eckert et al., PCR Methods and Applications 1, 17 (1991); PCR (eds. McPherson et al. IRL Press, Oxford); and U.S. Patent 4,683,202.

Other suitable amplification methods include the ligase chain reaction (LCR) (e.g., Wu and Wallace, Genomics 4,560 (1989) and Landegren et al., Science 241, 1077 (1988)), transcription amplification (Kwoh et al., Proc. Natl. Acad. Sci. USA 86, 1173 (1989)), and self-sustained sequence replication (Guatelli et al., Proc. Nat. Acad. Sci. USA, 87, 1874 (1990)) and nucleic acid based sequence amplification (NABSA). The latter two amplification methods include isothermal reactions based on isothermal transcription, which produce both single-stranded RNA (ssRNA) and double-stranded DNA (dsDNA) as the amplification products in a ratio of about 30 or 100 to 1, respectively.

B. DETECTION of NOVEL POLYMORPHISMS in TARGET DNA

There are two distinct types of analyses depending upon whether a given polymorphism has already been characterized. The first type of analysis is sometimes referred to as de novo characterization and compares target sequences in different subjects to identify points of variation, i.e., polymorphic sites. By analyzing a group of subjects representing the greatest ethnic diversity among human beings and the greatest breed and species variety of plants and animals, patterns characteristic of the most common alleles/haplotypes of a given locus can be identified, and the frequencies of such patterns in the population can be determined. Additional allelic frequencies can be determined for subpopulations characterized by criteria such as, for example, geography, race, or gender. This de novo characterization of the polymorphisms of this invention is described in the EXAMPLES section of this description. The second type of analysis determines which form(s) of a characterized polymorphism are present in the subjects under test. Many suitable procedures exist and these are discussed immediately below.

1. ALLELE-SPECIFIC PROBES

The design and use of allele-specific probes for analyzing polymorphisms is known in the art, e.g., Saiki et al., Nature 324, 163-166 (1986); Dattagupta, EP 235,726 and Saiki, WO 89/11548. Allele-specific probes can be designed that hybridize to a segment of target DNA from one subject but not to the corresponding segment from another subject due to the presence of different polymorphic forms in their respective segments. Hybridization conditions should be suitably stringent so that there is a significant difference in hybridization intensity between alleles, preferably an essentially binary response, whereby the chosen probe hybridizes to only one of the alleles. Some probes are designed to hybridize to a segment of target DNA such that the polymorphic site aligns with a central position (e.g., in a 15 mer at the 7 position; in a 16 mer, at either the 8 or 9 position) of the probe. This particular design of probe achieves useful discrimination in hybridization between different allelic forms.

Allele-specific probes are often used in pairs, with one member of a pair showing a perfect match to a reference form of a target sequence and the other member showing a perfect match to a variant form. Several pairs of probes can then be immobilized on the same support for simultaneous analyses of multiple polymorphisms within the same target sequence.

2. TILING ARRAYS

Polymorphisms can also be identified by hybridization to nucleic acid arrays, (See, e.g., WO 95/11995). One form of such arrays is described in the EXAMPLES section of this description in connection with de novo identification of polymorphisms. The same array or a different array can be used for analysis of characterized polymorphisms. The aforementioned WO 95/11995 also discloses subarrays that are optimized for the detection of variant forms of a pre-characterized polymorphism. Such subarrays contain probes designed to be complementary to a second reference sequence, which is an allelic variant of the first reference sequence. The second group of probes is designed by the same principles as described in the EXAMPLES section of this description except that the probes exhibit complementarity to the second reference sequence. The inclusion of a second group (or further groups) can be particularly useful for analyzing short subsequences of the primary reference sequence in which multiple mutations are predicted to occur within a short distance commensurate with the length of the probes (i.e., two or more mutations within 9 to 21 bases).

### 3. ALLELE-SPECIFIC PRIMERS

An allele-specific primer hybridizes to a site on a target DNA overlapping a polymorphism and only primes amplification of an allelic form to which the primer exhibits perfect complementarity. (See, e.g., Gibbs, Nucleic Acid Res. 17, 2427-2448 (1989)). This primer is used in conjunction with a second primer which hybridizes at a distal site. Amplification proceeds from the two primers leading to a detecTABLE product which signifies that the particular allelic form is present. A control is generally performed with a second pair of primers, one of which shows a single base mismatch at the polymorphic site and the other of which exhibits perfect complementarity the distal site. This single-base mismatch prevents amplification and, as such, no detectable product is formed. Most preferably, the mismatch is included in the 3'-most position of the oligonucleotide aligned with the polymorphism, i.e., the 3'-most position is the position most destabilizing to elongation from the primer. (See, e.g., WO 93/22456.)

### 4. DIRECT SEQUENCING

The direct analysis of the sequence of polymorphisms of this present invention can be accomplished by using either the dideoxy- chain termination method or the Maxam -Gilbert method (e.g., Sambrook et al., Molecular Cloning, A Laboratory Manual (2nd Ed., CSHP, New York 1989) and Zyskind et al., Recombinant DNA Laboratory Manual, (Acad. Press, 1988)).

### 5. DENATURING GRADIENT GEL ELECTROPHORESIS

Amplification products generated using PCR can be analyzed by the use of denaturing gradient gel electrophoresis. Different alleles can be identified based on the different sequence-dependent melting properties and electrophoretic migration of DNA in solution. (See, e.g., Erlich, ed., PCR Technology, Principles and Applications for DNA Amplification, (W.H. Freeman and Co, New York, 1992), Chapter 7.)

### 6. SINGLE STRAND CONFORMATION POLYMORPHISM

ANALYSIS

Alleles of target sequences can be differentiated using single-strand conformation polymorphism analysis, which identifies base differences by alteration in electrophoretic migration of single stranded PCR products. (See, e.g., Orita et al., Proc. Nat. Acad. Sci. 86, 2766-2770 (1989)). Amplified PCR products can be generated as described above, and heated or otherwise denatured, to form single stranded amplification products. Single-stranded nucleic acids may refold or form secondary structures which are partially dependent on the base sequence. The different electrophoretic mobilities of single-stranded amplification products can be related to base-sequence difference between alleles of the target sequences.

### 7. SINGLE BASE EXTENSION METHODS

Single base extension methods are described by e.g., US 5,846,710, US 6,004,744, US 5,888,819 and US 5,856,092. In brief; the methods work by hybridizing a primer that is complementary to a target strand such that the 3' end of the primer aligns with the base immediately 3' to the site of variation in the target strand but the primer does not include a base aligning with the site of variation. In other words, if the primer is formed from the complementary strand to the target strand, the primer includes sequence up to the base immediately 5' to the site of variation on the complementary strand. The hybridization is performed in the presence of one or more labelled nucleotides complementary to base(s) that may occupy the site of potential variation. For example, for a bilallelic polymorphisism two differentially labelled nucleotides can be used. In some methods, particularly, methods employing multiple differentially labelled nucleotides, the nucleotides are dideoynucleotides. Hybridization is performed uner conditions permitting primer extension if a nucleotide complementary to a base occupying the site of variation in the target sequence is present. Extension incorporates a labelled nucleotide thereby generating a labelled extended primer. If multiple differentially labelled nucleotides are used and the target is heterozygous then multiple differentially labelled extended primers can be obtained.

Extended primers are detected providing an indication of which base(s) occupy the site of variation in the target polynucleotide.

III. METHODS of USE

The presence of polymorphic form(s) in a subject at one or more polymorphic sites is useful for, e.g., forensics, paternity testing, correlation of polymorphisms with phenotypic traits, and genetic mapping of phenotypic traits.

A. FORENSICS

Determination of which polymorphic forms occupy a set of polymorphic sites in an individual identifies a set of polymorphic forms that distinguishes the individual. (See, e.g., National Research Council, *The Evaluation of Forensic DNA Evidence* (Eds. Pollard et al., National Academy Press, DC, 1996)). Increasing the number of sites analyzed the probability that the set of polymorphic forms in one individual is the same as that in an unrelated individual. Preferably, where multiple sites are analyzed, the sites are unlinked. Thus, polymorphisms of this invention are often used in conjunction with polymorphisms in distal genes. Preferred polymorphisms for use in forensics are diallelic because the population frequencies of two polymorphic forms can usually be determined with greater accuracy than those of multiple polymorphic forms at multi-allelic loci.

As discussed above, the capacity to identify a distinguishing or unique set of forensic markers in an individual is useful for forensic analysis. For example, one can determine whether a blood sample from a suspect matches a blood or other tissue sample from a crime scene by determining whether the set of polymorphic forms occupying selected polymorphic sites is the same in the samples from suspect and the sample taken from the crime scene. Where the set of polymorphic markers taken from the crime scene does not the sample from the suspect, it can be concluded (barring experimental error) that the suspect is not the source of the sample taken from the crime scene. Where the set of markers does match, one can conclude that the DNA from the suspect is consistent with that found at the crime scene. Where frequencies of the polymorphic forms at the loci tested have been determined (e.g., by analysis of a suitable population of individuals), a statistical analysis can be performed to determine the probability that such a match of suspect and crime scene would occur merely by chance.

p(ID) is the probability that two random individuals have the same polymorphic or allelic form at a given polymorphic site. In diallelic loci, four genotypes are possible: AA, AB, BA, and BB. Where alleles A and B occur in a haploid genome of the organism with frequencies x and y, the probability of each genotype in a diploid organism, as disclosed in WO 95/12607:

Homozygote: $p(AA) = x^2$ .
Homozygote: $p(BB) = y^2 = (1-x)^2$ .
Single Heterozygote: $p(AB) = p(BA) = xy = x(1-x)$ .
Both Heterozygotes: $p(AB+BA) = 2xy = 2x(1-x)$ .

The probability of identity at one locus (i.e, the probability that two individuals, picked at random from a population will have identical polymorphic forms at a given locus) is given by the equation:

$$p(ID) = (x^2)^2 + (2xy)^2 + (y^2)^2 .$$

These calculations can be extended for any number of polymorphic forms at a given locus. For example, the probability of identity p(ID) for a 3-allele system where the alleles have the frequencies in the population of x, y and z, respectively, is equal to the sum of the squares of the genotype frequencies:

$$p(ID) = x^4 + (2xy)^2 + (2yz)^2 + (2xz)^2 + z^4 + y^4 .$$

In a locus of n alleles, the appropriate binomial expansion is used to calculate p(ID) and p(exc).
The cumulative probability of identity (cum p(ID)) for each of multiple unlinked loci is determined by multiplying the probabilities provided by each locus.:

$$cum\ p(ID) = p(ID1)p(ID2)p(ID3).... p(IDn).$$

The cumulative probability of non-identity for w loci (i.e., the probability that two random individuals will be different at 1 or more loci) is given by the equation:

cum p(nonID) = 1-cum p(ID).

If several polymorphic loci are tested, the cumulative probability of non-identity for random individuals becomes very high (e.g., one in a billion). Such probabilities can be taken into account together with other evidence in determining the guilt or innocence of the suspect.

B. PATERNITY TESTING

The object of paternity testing is usually to determine whether a particular male is the father of a given child. In most cases, the mother of the child is known and thus, the mother's contribution to the child's genotype can be traced. Hence, paternity testing investigates whether the part of the child's genotype not attributable to the mother is consistent with that of the alleged father. Paternity testing can be performed by analyzing the sets of polymorphisms in both the alleged father and in his alleged child.

Where the set of polymorphisms in the child not attributable to the mother does not match the set of the alleged father, it can be concluded (barring experimental error) that the alleged father is not the child's father. Where the set of polymorphisms in the child not attributable to the mother does match the set of polymorphisms of the alleged father, a statistical calculation can be performed to determine the probability of a coincidental match.

The probability of parentage exclusion (representing the probability that a random male will have a polymorphic form at a given polymorphic site that makes him incompatible as the father) is given by the equation disclosed WO 95/12607:

$$p(exc) = xy(l-xy)$$

where x and y are the population frequencies of alleles A and B of a diallelic polymorphic site.

At a triallelic site $p(exc) = xy(l-xy) + yz(l-yz) + xz(l-xz)+ 3xyz(l-xyz)$, where x, y and z are the respective population frequencies of alleles A, B, and C.

The probability of non-exclusion is:

$$p(non-exc) = l-p(exc).$$

The cumulative probability of non-exclusion (representing the value obtained when n loci are used) is thus:

$$cum\ p(non-exc) = p(non-exc1)p(non-exc2)p(non-exc3).... p(non-excn).$$

The cumulative probability of exclusion for z loci (representing the probability that a random male will be excluded)

$$cum\ p(exc) = 1 - cum\ p(non-exc).$$

Where several polymorphic loci are included in the analysis, the cumulative probability of exclusion of a random male is very high. This probability can be taken into account in assessing the liability of an alleged father whose polymorphic marker set matches the child's polymorphic marker set attributable to the child's father.

C. CORRELATION of POLYMORPHISMS with PHENOTYPIC TRAITS

The polymorphisms of this invention may contribute to the phenotype of an organism in different ways. As discussed above, some polymorphisms occur within a protein coding sequence and contribute to phenotype by affecting protein structure. The effect of such a change to the structure of a protein may be neutral, beneficial, or detrimental, or both beneficial and detrimental. For instance, a heterozygous sickle cell mutation confers resistance to malaria, but a homozygous sickle cell mutation is usually lethal. Other polymorphisms occur in noncoding regions and exert phenotypic effects indirectly via influence on, e.g., replication, transcription, and translation. Moreover, a single polymorphism may affect more than one phenotypic trait. Likewise, a single phenotypic trait may be affected by polymorphisms in different genes. Further, some polymorphisms predispose an individual to a distinct mutation that is causally related to a certain phenotype.

Phenotypic traits include diseases that have known (but hitherto unmapped) genetic components (e.g., agammaglobulimenia, diabetes insipidus, Lesch-Nyhan syndrome, muscular dystrophy, Wiskott-Aldrich syndrome, Fabry's disease, familial hypercholesterolemia, polycystic kidney disease, hereditary spherocytosis, von Willebrand's disease, tuberous sclerosis, hereditary hemorrhagic telangiectasia, familial colonic polyposis, Ehlers-Danlos syndrome, osteogenesis imperfecta, and acute intermittent porphyria and the like). Phenotypic traits also include symptoms of, or susceptibility to, multifactorial diseases of which a component is, or may be, genetic, such as autoimmune diseases, inflammation, cancer, diseases of the nervous system, and infection by pathogenic microorganisms. Some examples of autoimmune diseases include rheumatoid arthritis, multiple sclerosis, diabetes (insulin-dependent and non-independent), systemic lupus erythematosus, and Graves disease. Some examples of cancers include cancers of the bladder, brain, breast, colon, esophagus, kidney, leukemia, liver, lung, oral cavity, ovary, pancreas, prostate, skin, stomach and uterus. Phenotypic traits also include characteristics such as longevity, appearance (e.g., baldness, obesity), strength, speed, endurance, fertility, and susceptibility or receptivity to particular drugs or therapeutic treatments.

Correlation of polymorphisms with phenotypic traits is performed for a population of subjects who have been tested for the presence or absence of a phenotypic trait of interest and for polymorphic markers sets. To perform such analysis, the presence or absence of a set of polymorphisms (i.e. a polymorphic set) is determined for a set of the subjects, some of whom exhibit a particular trait, and some of whom do not. The alleles of each polymorphism of the set are then reviewed to determine whether the presence or absence of a particular allele is associated with the phenotypic trait of interest. Correlation can be performed by using standard statistical methods such as a ê-squared test and by noting any statistically significant correlations between the polymorphic form(s) and the phenotypic characteristics. For example, it might be found that the presence of allele A1 at polymorphism A correlates with heart disease. As a further example, it might be found that the combined presence of allele A1 at polymorphism A and allele B1 at polymorphism B correlates with increased milk production of a farm animal.

These correlations are useful in several ways. For example, where a strong correlation exists between a set of one or more polymorphic forms and a disease for which treatment is available, detection of the polymorphic form set in a subject, i.e., a human being or an animal may justify immediate administration of treatment, or at least the institution of regular monitoring of the subject. Detection of a polymorphic form correlated with a serious disease can assist in various types of decisions, for example, reproductive decision making. For instance, a female partner might elect to undergo in vitro fertilization to avoid the possibility of transmitting such a polymorphism from her male partner to her offspring.

Where a weaker yet statistically significant correlation exists between a polymorphic set and a human disease, immediate therapeutic intervention or monitoring may not be justified. Nevertheless, the patient can be motivated to begin simple life-style changes (e.g., diet, exercise) that can be accomplished at little cost to the patient but confer potential benefits in reducing the risk of conditions to which the patient may have increased susceptibility by virtue of variant alleles. Identification of a polymorphic set in a patient correlated with enhanced receptiveness to one of several treatment regimes for a disease indicates that this treatment regime should be followed.

For animals and plants, correlations between characteristics and phenotype are useful for e.g., breeding for desired characteristics. For example, Beitz et al., in US 5,292,639, disclose use of bovine mitochondrial polymorphisms in a breeding program to improve milk production in cows. To evaluate the effect of mitochondrial DNA (mtDNA) displacement loop (D-loop) sequence polymorphism on milk production, each cow was assigned a value of 1, where variant, or 0, where wildtype, with respect to a prototypical mtDNA sequence at each of the 17 locations considered. Each production trait was analyzed individually with the following animal model:

$$Y_{ijkpn} = \mu + YS_i + P_j + X_k + \beta_1 + \dots \beta_{17} + PE_n + a_n + e_p$$

wherein: $Y_{ijknp}$ is the milk, fat, fat percentage, solids-not-fat (SNF), SNF percentage, energy concentration, or lactation energy record; $\mu$ is an overall mean; $YS_i$ is the effect common to all cows calving in year-season; $X_k$ is the effect common to cows in either the high or average selection line; $\beta_1$ to $\beta_{17}$ are the binomial regressions of production record on mtDNA D-loop sequence polymorphisms; $PE_n$ is permanent environmental effect common to all records of cow n; $a_n$ is the effect of animal n and is composed of the additive genetic contribution of sire and dam breeding values and a Mendelian sampling effect; and $e_p$ is a random residual. It was found that eleven of the seventeen polymorphisms tested influenced at least one production trait. Bovines having the best polymorphic forms for milk production at these eleven loci are selected to breed the next generation of the herd.

D. GENETIC MAPPING of PHENOTYPIC TRAITS

The previous section described the identification of correlations between phenotypic traits and polymorphisms that directly or indirectly contribute to those traits. The present section describes the identification of a physical linkage between a genetic locus associated with a trait of interest and polymorphic markers that are not associated with that trait, but rather are in physical proximity with the genetic locus responsible for the trait and co-segregate with it. Such analysis is useful for, e.g., mapping a genetic locus associated with a phenotypic trait to a chromosomal position, and thereby cloning the gene(s) responsible for the trait. (See, e.g., Lander et al., *Proc. Natl. Acad. Sci.* (USA) 83, 7353--7357 (1986); Lander et al., *Proc. Natl. Acad. Sci.* (USA) 84, 2363-2367 (1987); Donis-Keller et al., *Cell* 51, 319-337 (1987); Lander et al., *Genetics* 121, 185-199 (1989)). Genes localized by linkage can be cloned by a process known as directional cloning. (See, e.g., Wainwright, *Med. J. Australia* 159, 170-174 (1993); and Collins, *Nature Genetics* 1, 3-6 (1992)).

Linkage studies are generally performed on members of a family. Available members of the family are characterized for the presence or absence of a phenotypic trait and for a set of polymorphic markers. The distribution of polymorphic markers in an informative meiosis is then analyzed to determine which polymorphic markers co-segregate with a phenotypic trait. (See, e.g., Kerem et al., *Science* 245, 1073-1080 (1989); Monaco et al., *Nature* 316, 842 (1985); Yamoka et al., *Neurology* 40, 222-226 (1990); and Rossiter et al*., FASEB Journal* 5, 21-27 (1991)).

Linkage is analyzed by calculation of log of the odds (LOD) values. A LOD value is the relative likelihood of obtaining observed segregation data for a marker and a genetic locus when the two are located at a recombination fraction è, versus the situation in which the two are not linked, and thus segregating independently (See, e.g., Thompson & Thompson, *Genetics in Medicine* (5th ed, W.B. Saunders Company, Philadelphia, 1991); and Strachan, "Mapping the human genome" in *The Human Genome* (BIOS Scientific Publishers Ltd, Oxford), Chapter 4). A series of likelihood ratios are calculated at various $\theta$, ranging from $\theta$ = 0.0 (coincident loci) to $\theta$ = 0.50 (unlinked). Thus, the likelihood at a given value of $\theta$ is the probability of data where loci are linked at $\theta$ to the probability of data where loci are unlinked. The computed likelihoods are usually expressed as the $\log_{10}$ of this ratio (i.e., a LOD value). For example, a LOD value of 3 indicates 1000:1 odds against an apparent observed linkage being a coincidence. The use of logarithms allows data collected from different families to be combined by simple addition. Computer programs are available for the calculation of LOD scores for differing values of $\theta$ (e.g., LIPED, MLINK (See e.g., Lathrop, *Proc. Nat. Acad. Sci.* (USA) 81, 3443-3446 (1984)). For any particular LOD score, a recombination fraction may be determined from mathematical tables. (See, e.g., Smith et al., *Mathematical tables for research workers in human genetics* (Churchill, London, 1961); and Smith, *Ann. Hum. Genet.* 32, 127-150 (1968)). The value of $\theta$ at which the LOD score is the highest is considered to be the best estimate of the recombination fraction. Positive LOD score values suggest that the two loci are linked, whereas negative LOD values suggest that linkage is less likely (at that value of $\theta$) than the possibility that the two loci are unlinked. By convention, a combined LOD value of +3 or greater (equivalent to greater than 1000:1 odds in favor of linkage) is considered definitive evidence that the two loci are linked. Similarly, by convention, a negative LOD value of -2 or less is taken as definitive evidence against linkage of the two loci being compared. Negative linkage data are useful in excluding a chromosome or a segment thereof from consideration. The search then focuses on the remaining non-excluded chromosomal locations.

IV. <u>MODIFIED POLYPEPTIDES and GENE SEQUENCES</u>

This invention further provides variant forms of nucleic acids and corresponding proteins. The nucleic acids comprise one of the sequences described in TABLE 1, column 8, in which the polymorphic position is occupied by one of the alternative bases for that position. Some nucleic acid encode full-length variant forms of proteins. Similarly, variant proteins have the prototypical amino acid sequences encoded by the nucleic acid sequence shown in TABLE 1, column 8, (read so as to be in-frame with the full-length coding sequence of which it is a component) except at an amino acid encoded by a codon including one of the polymorphic positions shown in TABLE 1. That position is occupied by the amino acid coded by the corresponding codon in any of the alternative forms shown in TABLE 1.

Variant genes can be expressed in an expression vector in which a variant gene is operably linked to a native or other promoter. Usually, the promoter is a eukaryotic promoter for expression in a mammalian cell. The transcription regulation sequences typically include a heterologous promoter and optionally an enhancer that is recognized by the host. The selection of a promoter, for example trp, lac, phage, glycolytic enzyme and tRNA promoters, depends, in part, on the host selected. Commercially available expression vectors can be used in this invention. Vectors can include host-recognized replication systems, amplifiable genes, selecTABLE markers, host sequences useful for insertion into the host genome, and the like.

The means of introducing the expression construct into a host cell varies depending upon, for example, the particular construction and the target host. Suitable means include fusion, conjugation, transfection, transduction, electroporation or injection, as described, for example, in Sambrook, *supra*. A wide variety of prokaryotic and eukaryotic host cells can be employed for expression of the variant gene. Suitable host cells include, for example, bacteria such as *E. coli*, yeast, filamentous fungi, insect cells, mammalian cells, typically immortalized, e.g., mouse, CHO, human and monkey cell lines, as well as derivatives thereof. Preferred host cells are able to process the variant gene product to produce the mature polypeptide, where processing includes glycosylation, ubiquitination, disulfide bond formation, general post-translational modification, and the like.

The protein may be isolated by conventional means of protein biochemistry and purification to obtain a substantially pure product, i.e., 80, 95 or 99% free of cell component contaminants. (See, e.g., Jacoby, *Methods in Enzymology* Volume 104, Academic Press, New York (1984); Scopes, *Protein Purification, Principles and Practice,* 2nd Edition, Springer-Verlag, New York (1987); and Deutscher (ed), *Guide to Protein Purification, Methods in Enzymology,* Vol. 182 (1990)). Where the protein is secreted, it can be isolated from the supernatant in which the host cell is grown. Where the protein is not secreted, the protein can be isolated from a lysate of the host cells.

This invention further provides transgenic nonhuman animals capable of expressing an exogenous variant gene and/or having one or both alleles of an endogenous variant gene inactivated. Expression of an exogenous variant gene is usually achieved by operably linking the gene to a promoter and optionally an enhancer, and microinjecting the construct into a zygote. (See, e.g., Hogan et al., "*Manipulating the Mouse Embryo, A Laboratory Manual,*" Cold Spring Harbor Laboratory.) Inactivation of endogenous variant genes can be achieved by forming a transgene in which a cloned variant gene is inactivated by insertion of a positive selection marker. (See, e.g., Capecchi, *Science* 244, 1288-1292 (1989)). The transgene is then introduced into an embryonic stem cell, where it undergoes homologous recombination with an endogenous variant gene. Mice and other rodents are preferred animals. Such animals provide useful drug screening systems.

In addition to substantially full-length polypeptides expressed by variant genes, this present invention also includes biologically active fragments of the polypeptides, or analogs thereof, including organic molecules which simulate the interactions of the peptides. Biologically active fragments include any portion of the full-length polypeptide that confers a biological function on the variant gene product, including ligand and antibody binding. Ligand binding includes binding by nucleic acids, proteins or polypeptides, small biologically active molecules, or large cellular structures.

Polyclonal and/or monoclonal antibodies that specifically bind to variant gene products but not to corresponding prototypical gene products are also provided by this invention. Antibodies can be made by injecting mice or other animals with the variant gene product or synthetic peptide fragments thereof Monoclonal antibodies are screened as are described, for example, in Harlow & Lane, *Antibodies, A Laboratory Manual,* Cold Spring Harbor Press, New York (1988); and Goding, *Monoclonal antibodies, Principles and Practice* (2d ed.) Academic Press, New York (1986). Monoclonal antibodies are tested for specific immunoreactivity with a variant gene product and lack of immunoreactivity to the corresponding prototypical gene product. These antibodies are useful in diagnostic assays for detection of the variant form, or as an active ingredient in a pharmaceutical composition.

V. KITS

This invention further provides kits comprising at least one allele-specific oligonucleotide as described above. Preferably, the kits contain one or more pairs of allele-specific oligonucleotides hybridizing to different forms of a polymorphism. In some kits, the allele-specific oligonucleotides are provided immobilized to a substrate. For example, the same substrate can comprise allele-specific oligonucleotide probes for detecting at least 10, 100 or all of the polymorphisms shown in TABLE 1. Optional additional components of the kit include, for example, restriction enzymes, reverse-transcriptase or polymerase, the substrate nucleoside triphosphates, means used to label (for example, an avidin-enzyme conjugate and enzyme substrate and chromogen where the label is biotin), and the appropriate buffers for reverse transcription, PCR, or hybridization reactions. Generally, the kit also contains instructions for carrying out the methods.

VI. Computer Systems For Storing Polymorphism Data

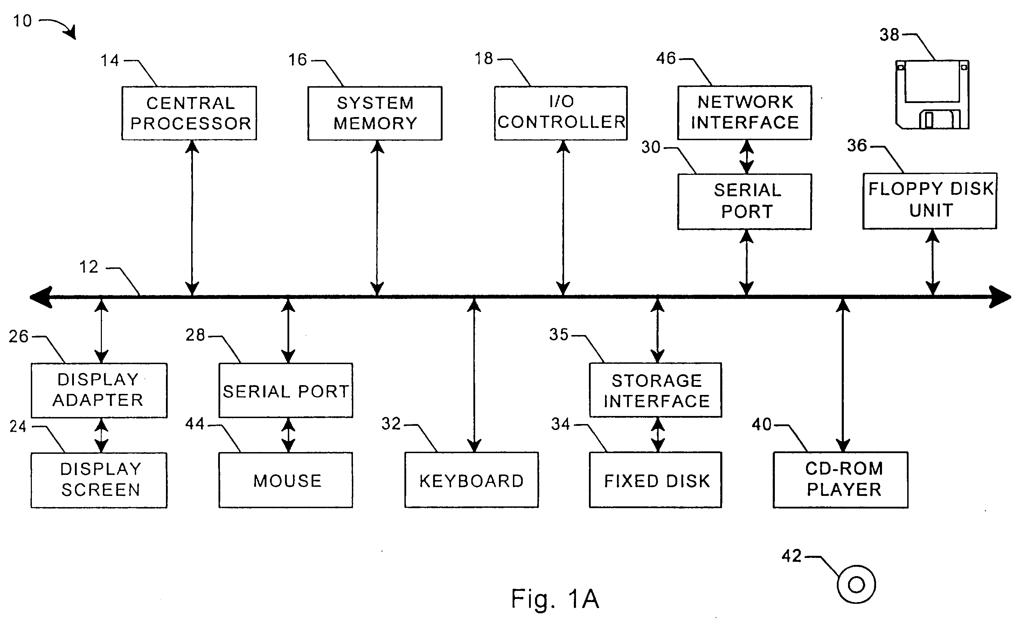

Fig. 1A depicts a block diagram of a computer system 10 suitable for implementing the present invention. Computer system 10 includes a bus 12 which interconnects major subsystems such as a central processor 14, a system memory 16 (typically RAM), an input/output (I/O) controller 18, an external device such as a display screen 24 via a display adapter 26, serial ports 28 and 30, a keyboard 32, a fixed disk drive 34 via a storage interface 35 and a floppy disk drive 36 operative to receive a floppy disk 38, and a CD-ROM (or DVD-ROM) device 40 operative to receive a CD-ROM 42. Many other devices can be connected such as a user pointing device, e.g., a mouse 44 connected via serial port 28 and a network interface 46 connected via serial port 30.

Many other devices or subsystems (not shown) may be connected in a similar manner. Also, it is not necessary for all of the devices shown in Fig. 1A to be present to practice the present invention, as discussed below. The devices and subsystems may be interconnected in different ways from that shown in Fig. 1A. The operation of a computer system such as that shown in Fig. 1A is well known. Databases storing polymorphism information according to the present invention can be stored, e.g., in system memory 16 or on storage media such as fixed disk 34, floppy disk 38, or CD-ROM 42. An application program to access such databases can be operably disposed in system memory 16 or sorted on storage media such as fixed disk 34, floppy disk 38, or CD-ROM 42.

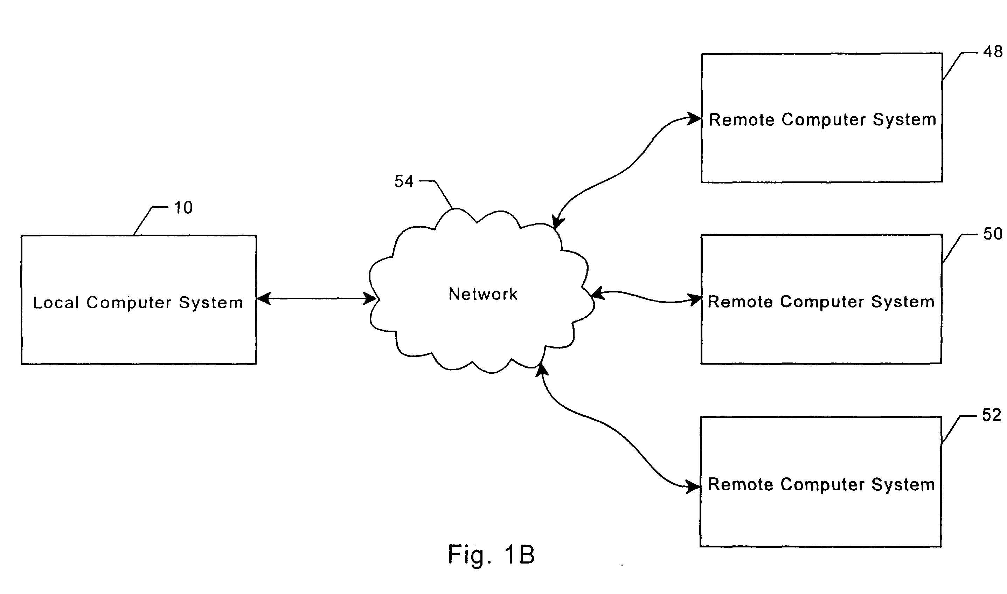

Fig. 1B depicts the interconnection of computer system 10 to remote computers 48, 50, and 52. Fig. 1B depicts a network 54 interconnecting remote servers 48, 50, and 52. Network interface 46 provides the connection from client computer system 10 to network 54. Network 54 can be, e.g., the Internet. Protocols for exchanging data via the Internet and other networks are well known. Information identifying the polymorphisms described herein can be transmitted across network 54 embedded in signals capable of traversing the physical media employed by network 54.

Information identifying polymorphisms shown in Table 1 is represented in records, which optionally, are subdivided into fields. Each record stores information relating to a different polymorphisms in Table 1. Collectively, the records can store information relating to all of the polymorphisms in Table 1, or any subset thereof, such as 5, 10, 50, or 100 polymorphisms from Table 1. In some databases, the information identifies a base occupying a polymorphic position and the location of the polymorphic position. The base can be represented as a single letter code (i.e., A, C, G or T/U) present in a polymorphic form other than that in the reference allele. Alternatively, the base occupying a polymorphic site can be represented in IUPAC ambiguity code as shown in Table 1. The location of a polymorphic site can be identified as its position within one of the sequences shown in Table 1. For example, in the first sequence shown in Table 1, the polymorphic site occupies the Aor C base. The position can also be identified by reference to, for example, a chromosome, and distance from known markers within the chromosome. In other databases, information identifying a polymorphism contains sequences of 10-100 bases shown in Table 1 or the complements thereof, including a polymorphic site. Preferably, such information records at least 10, 15, 20, or 30 contiguous bases of sequences including a polymorphic site.

EXAMPLES

The polymorphisms shown in TABLE 1 were identified by resequencing of target sequences from eight unrelated individuals of diverse ethnic and geographic backgrounds by hybridization to probes immobilized to microfabricated arrays. The strategy and principles for design and use of such arrays are generally described in WO 95/11995. The strategy provides arrays of probes for analysis of target sequences showing a high degree of sequence identity to the reference sequences of the fragments shown in TABLE 1, column 1. The reference sequences were sequence-tagged sites (STSs) developed in the course of the Human Genome Project (see, e.g., *Science* 270, 1945-1954 (1995); *Nature* 380, 152-154 (1996)). Most STS's ranged from 100 base pair (bp) to 300 bp in size.

A typical probe array used in this analysis has two groups of four sets of probes that respectively tile both strands of a reference sequence. A first probe set comprises a plurality of probes exhibiting perfect complementarily with one of the reference sequences. Each probe in the first probe set has an interrogation position that corresponds to a nucleotide in the reference sequence. That is, the interrogation position is aligned with the corresponding nucleotide in the reference sequence, where the probe and reference sequence are aligned to maximize complementarity between the two. For each probe in the first set, there are three corresponding probes from three additional probe sets. Thus, there are four probes corresponding to each nucleotide in the reference sequence. The probes from these three additional probe sets are identical to the corresponding probe from the first probe set except at the interrogation position, which occurs in the same position in each of the four corresponding probes from the four probe sets, and is occupied by a different nucleotide in the four probe sets. In the present analysis, probes were 25 nucleotides long. Arrays tiled for multiple different reference sequences were included on the same substrate.

Multiple target sequences from an individual were amplified from human genomic DNA using primers for the fragments. The amplified target sequences were fluorescently labeled during or after PCR. The labeled target sequences were hybridized with a substrate bearing immobilized arrays of probes. The amount of label bound to the probes was measured. Analysis of the pattern of label revealed the nature and position of differences between the target and reference sequence. For example, comparison of the intensities of four corresponding probes reveals the identity of a corresponding nucleotide in the target sequences aligned with the interrogation position of the probes. The corresponding nucleotide is the complement of the nucleotide occupying the interrogation position of the probe showing the highest intensity. (See, e.g., WO 95/11995). The existence of a polymorphism is also manifested by differences in normalized hybridization intensities of probes flanking the polymorphism where the probes hybridized to corresponding targets from different individuals. For example, relative loss of hybridization intensity in a "footprint" of probes flanking a polymorphism signals a difference between the target and the reference sequence (i.e., a polymorphism) (See, e.g., EP 717,113). Additionally, hybridization intensities for corresponding targets from different individuals can be classified into groups or clusters suggested by the data, not defined a *priori*, such that isolates in a give cluster tend to be similar and isolates in different clusters tend to be dissimilar. See US Patent Application No. 08/797,812, filed February 7, 1997. Hybridizations to samples from different individuals were performed separately. TABLE 1 summarizes the data obtained for target sequences in comparison with a reference sequence for the eight individuals tested.

From the foregoing, it is clear that the invention includes a number of general uses that can be expressed concisely as follows. This invention provides for the use of any of the nucleic acid segments described above in the diagnosis or monitoring of diseases, such as cancer, inflammation, heart disease, diseases of the CNS, and susceptibility to infection by microorganisms. This invention further provides for the use of any of the nucleic acid segments in the manufacture of a medicament for the treatment (includes, inter alia, preventative (e.g., prophylactic), palliative and curative treatment) of such diseases. This invention further provides for the use of any of the DNA segments as a pharmaceutical in any suitable dosage form.

The present invention is described in full, clear, concise and exact terms to enable those skilled in the art to make and use this invention. In addition, EXAMPLES and illustrations are provided albeit solely for the purposes of even more clarity and understanding and, as such, do not limit this invention which is defined by the appendant claims. It will also be understood that certain changes and modifications that may be practiced are within the scope of the appendant claims.

**Claims**

1. A nucleic acid segment of between 10 and 100 contiguous bases selected from a fragment shown in TABLE 1, wherein said segment comprises a polymorphic site or an immediately adjacent base, or the complement of said segment.

2. The segment of claim 1 that is DNA.

3. The segment of claim 1 that is RNA.

4. The segment of claim 1 that is less than 50 bases.

5. The segment of claim 1 that is less than 20 bases.

6. The segment of claim 1, wherein the fragment is D42043 and the polymorphic site is at position 1470.

7. The segment of claim 1, wherein the polymorphic site is diallelic.

8. The segment of claim 1, wherein the polymorphic form occupying the polymorphic site is the reference base for the fragment listed in TABLE 1, column 4.

9. The segment of claim 1, wherein the polymorphic form occupying the polymorphic site is an alternative form for the fragment listed in TABLE 1, column 5.

10. An allele-specific oligonucleotide that hybridizes to a segment of a fragment shown in TABLE 1, column 6 or its complement.

11. The allele-specific oligonucleotide of claim 10 that is probe.

**12.** The allele-specific oligonucleotide of claim 10, wherein a central position of the probe aligns with the polymorphic site of the fragment.

**13.** The allele-specific oligonucleotide of claim 10 that is a primer.

**14.** The allele-specific oligonucleotide of claim 13, wherein the 3' end of the primer aligns with the polymorphic site or an immediately adjacent base of the fragment.

**15.** An isolated nucleic acid comprising a sequence of TABLE 1, column 6 or the complement thereof, wherein the polymorphic site within the sequence or complement is occupied by a base other than the reference base show in TABLE 1, column 4.

**16.** A method of analyzing a nucleic acid, comprising:

   obtaining the nucleic acid from an individual; and

   determining a base occupying any one of the polymorphic sites shown in TABLE 1.

**17.** The method of claim 16, wherein the determining comprises determining a set of bases occupying a set of the polymorphic sites shown in TABLE 1.

**18.** The method of claim 16, wherein the nucleic acid is obtained from a plurality of individuals, and a base occupying one of the polymorphic positions is determined in each of the individuals, and the method further comprising testing each individual for the presence of a disease phenotype, and correlating the presence of the disease phenotype with the base.

**19.** The nucleic acid of claim 15 that is DNA.

**20.** The nucleic acid of claim 15 that is RNA.

**21.** The nucleic acid of claim 15 that is less than 50 bases.

**22.** The nucleic acid of claim 15 that is less than 20 bases.

**23.** The nucleic acid of claim 15, wherein the fragment is AF005058 and the polymorphic site is at position 3583.

**24.** The nucleic acid of claim 15, wherein the polymorphic site is diallelic.

**25.** The nucleic acid of claim 15, wherein the base occupying the polymorphic site is the correlating base for the fragment listed in TABLE 1, column 4.

**26.** The nucleic acid of claim 15, wherein the base occupying the polymorphic site is the correlating base for the fragment listed in TABLE 1, column 5.

**27.** The segment of claim 1, wherein said fragment is selected from TABLE 1, SEQ ID Nos: 1-10.

**28.** The segment of claim 1, wherein said fragment is selected from TABLE 1, SEQ ID Nos: 11-20.

**29.** The segment of claim 1, wherein said fragment is selected from TABLE 1, SEQ ID Nos:21-30.

**30.** The segment of claim 1, wherein said fragment is selected from TABLE 1, SEQ ID NOS:31-40.

**31.** The segment of claim 1, wherein said fragment is selected from TABLE 1, SEQ ID NOS:41-50.

**32.** The segment of claim 1, wherein said fragment is selected from TABLE 1, SEQ ID NOS:51-60.

**33.** The segment of claim 1, wherein said fragment is selected from TABLE 1, SEQ ID NOS:61-70.

**34.** The segment of claim 1, wherein said fragment is selected from TABLE 1, SEQ ID NOS:71-80.

**35.** The segment of claim 1, wherein said fragment is selected from TABLE 1, SEQ ID NOS:81-90.

**36.** The segment of claim 1, wherein said fragment is selected from TABLE 1, SEQ ID NOS:91-100.

**37.** The segment of claim 1, wherein said fragment is selected from TABLE 1, SEQ ID NOS:101-110.

**38.** The segment of claim 1, wherein said fragment is selected from TABLE 1, SEQ ID NOS:111-120.

**39.** The segment of claim 1, wherein said fragment is selected from TABLE 1, SEQ ID NOS:121-130.

**40.** The segment of claim 1, wherein said fragment is selected from TABLE 1, SEQ ID NOS:131-140.

**41.** The segment of claim 1, wherein said fragment is selected from TABLE 1, SEQ ID NOS:141-150.

**42.** The segment of claim 1, wherein said fragment is selected from TABLE 1, SEQ ID NOS:151-158.

**43.** The segment of claim 15, wherein said fragment is selected from TABLE 1, SEQ ID NOS: 1-10.

**44.** The segment of claim 15, wherein said fragment is selected from TABLE 1, SEQ ID NOS: 11-20.

**45.** The nucleic acid of claim 15, wherein said fragment is selected from TABLE 1, SEQ ID NOS: 21-30.

**46.** The nucleic acid of claim 15, wherein said fragment is selected from TABLE 1, SEQ ID NOS:31-40.

**47.** The nucleic acid of claim 15, wherein said fragment is selected from TABLE 1, SEQ ID NOS:41-50.

**48.** The nucleic acid of claim 15, wherein said fragment is selected from TABLE 1, SEQ ID NOS:51-60.

**49.** The nucleic acid of claim 15, wherein said fragment is selected from TABLE 1, SEQ ID NOS:61-70.

**50.** The nucleic acid of claim 15, wherein said fragment is selected from TABLE 1, SEQ ID NOS:71-80.

**51.** The nucleic acid of claim 15, wherein said fragment is selected from TABLE 1, SEQ ID NOS:81-90.

**52.** The nucleic acid of claim 15, wherein said fragment is selected from TABLE 1, SEQ ID NOS:91-100.

**53.** The nucleic acid of claim 15, wherein said fragment is selected from TABLE 1, SEQ ID NOS:101-110.

**54.** The nucleic acid of claim 15, wherein said fragment is selected from TABLE 1, SEQ ID NOS:111-120.

**55.** The nucleic acid of claim 15, wherein said fragment is selected from TABLE 1, SEQ ID NOS:121-130.

**56.** The nucleic acid of claim 15, wherein said fragment is selected from TABLE 1, SEQ ID NOS:131-140.

**57.** The nucleic acid of claim 15, wherein said fragment is selected from TABLE 1, SEQ ID NOS:141-150.

**58.** The nucleic acid of claim 15, wherein said fragment is selected from TABLE 1, SEQ ID NOS:151-158.

10

CENTRAL PROCESSOR 14

SYSTEM MEMORY 16

I/O CONTROLLER 18

NETWORK INTERFACE 46

SERIAL PORT 30

FLOPPY DISK UNIT 36

38

12

DISPLAY ADAPTER 26

DISPLAY SCREEN 24

SERIAL PORT 28

MOUSE 44

KEYBOARD 32

STORAGE INTERFACE 35

FIXED DISK 34

CD-ROM PLAYER 40

42

Fig. 1A

Remote Computer System — 48

Remote Computer System — 50

Remote Computer System — 52

54 — Network

10 — Local Computer System

Fig. 1B